# EUROPEAN PATENT SPECIFICATION

(11) **EP 2 241 630 B1**
(45) Date of publication and mention of the grant of the patent: **01.06.2016**
(21) Application number: 08857657.4
(22) Date of filing: 04.12.2008
(51) Int. Cl.: C12P 7/46, C12N 9/88, C08G 69/10

(54) **A METHOD FOR PRODUCING AN ORGANIC ACID**
VERFAHREN ZUR HERSTELLUNG VON ORGANISCHER SÄURE
PROCÉDÉ DE PRODUCTION D'UN ACIDE ORGANIQUE

(30) Priority: 06.12.2007 JP 2007315764
(43) Date of publication of application: 20.10.2010
(73) Proprietor: Ajinomoto Co., Inc., Tokyo 104-8315 (JP)
(72) Inventor: TAJIMA, Yoshinori, Kawasaki-shi Kanagawa 210-8681 (JP); FUKUI, Keita, Kawasaki-shi Kanagawa 210-8681 (JP); HASHIGUCHI, Kenichi, Kawasaki-shi Kanagawa 210-8681 (JP)
(74) Representative: Strehl Schübel-Hopf & Partner
(86) International application number: PCT/JP2008/072055
(87) International publication number: WO 2009/072562

(56) References cited:
- WO-A1-2005/113745
- WO-A1-2005/116227
- WO-A2-2006/034156
- KIM P ET AL: "Effect of Overexpression of Actinobacillus succinogenes Phosphoenolpyruvate Carboxykinase on Succinate Production in Escherichia coli", APPLIED AND ENVIRONMENTAL MICROBIOLOGY, AMERICAN SOCIETY FOR MICROBIOLOGY, US, vol. 70, no. 2, 1 February 2004 (2004-02-01), pages 1238-1241, XP002999937, ISSN: 0099-2240, DOI: 10.1128/AEM.70.2.1238-1241.2004
- WU HUI ET AL.: 'Improved Succinic Acid Production in the Anaerobic Culture of an Escherichia coli pflB ldhA Double Mutant as a Result of Enhanced Anaplerotic Activities in the Preceding Aerobic Culture.' APPL. ENVIRON. MICROBIOL. vol. 73, no. 24, October 2007, pages 7837 - 7843, XP008136337
- SANCHEZ M. AILEN ET AL.: 'Efficient Succinic Acid Production from Glucose through Overexpression of Pyruvate Carboxylase in an Escherichia coli Alcohol Dehydrogenase and Lactate Dehydrogenase Mutant.' BIOTECHNOL. PROG. vol. 21, 2005, pages 358 - 365, XP008136355
- ITO TAKESHI ET AL.: 'High-Yield Production of Hydrogen by Enterobacter aerogenes Mutants with Decreased a-Acetolactate Synthase Activity.' J. BIOSCI. BIOENG. vol. 97, no. 4, 2004, pages 227 - 232, XP008136338
- D. Urdaneta ET AL: "Short-chain organic acids produced on glucose, lactose, and citrate media by Enterococcus faecalis, Lactobacillus casei, and Enterobacter aerogenes strains", Bioresource Technology, vol. 54, no. 2, 1 January 1995 (1995-01-01), pages 99-103, XP055141709, ISSN: 0960-8524, DOI: 10.1016/0960-8524(95)00103-4
- Converti A. ET AL: "Use of carbon and energy balances in the study of the anaerobic metabolism of Enterobacter aerogenes at variable starting glucose concentrations", Applied Microbiology and Biotechnology, vol. 59, no. 2-3, 1 July 2002 (2002-07-01) , pages 303-309, XP055141710, ISSN: 0175-7598, DOI: 10.1007/s00253-002-1009-5
- C. Riondet ET AL: "Extracellular Oxidoreduction Potential Modifies Carbon and Electron Flow in Escherichia coli", Journal of Bacteriology, vol. 182, no. 3, 1 February 2000 (2000-02-01), pages 620-626, XP055141712, ISSN: 0021-9193, DOI: 10.1128/JB.182.3.620-626.2000

## Description

### Technical Field

The present invention relates to a method for producing succinic acid or malic acid using a bacterium.

### Background Art

For the production of non-amino organic acids, including succinic acid, by fermentation, anaerobic bacteria including those belonging to the genus *Anaerobiospirillum* or *Actinobacillus* are usually used (Patent documents 1 and 2, Non-patent document 1). Although use of such anaerobic bacteria provides high yields of products, many nutrients are required for their proliferation, and therefore it is necessary to add large amounts of organic nitrogen sources such as corn steep liquor (CSL) into the culture medium. The addition of sources of large amounts of organic nitrogen results in not only an increase in cost for the culture medium, but also an increase in the purification cost for isolating the product, and therefore it is not economical.

In addition, methods are known in which aerobic bacteria such as coryneform bacteria are cultured once under aerobic conditions to proliferate the bacterial cells, then harvested, washed, and allowed as resting cells to produce a non-amino organic acid without supplying oxygen (Patent documents 3 and 4). These methods are economical, since organic nitrogen may be added in a smaller amount for proliferating the bacterial cells, and the bacteria can sufficiently grow in a simple culture medium. However, there is still a room for improvement in terms of production amounts, concentration, and production rate per cell of the target organic acids as well as simplification of the production process, and the like.

Furthermore, as for *Escherichia coli,* which is a facultative anaerobic gram negative bacterium, methods for producing a non-amino organic acid by culturing it once under aerobic conditions to allow growth of cells, and then culturing it as resting cells without supplying oxygen to anaerobically produce the non-amino organic acid (Non-patent document 2), like the methods using coryneform bacteria, or aerobically culturing it to aerobically produce the non-amino organic acid (Patent document 5) are known. However, since *Escherichia coli* is a gram negative bacterium, it is vulnerable to osmotic pressure, and there remains room for improvement in productivity per cell etc.

As for the breeding of such bacteria as described above, concerning the anaplerotic pathway, there have been also reported production of non-amino organic acids by fermentation utilizing a strain of *Escherichia coli*, coryneform bacterium or the like, in which phosphoenolpyruvate carboxylase (PEPC) activity or pyruvate carboxylase (PYC) activity is enhanced, and the like (for example, Patent documents 4 and 6, Non-patent document 3).

As for phosphoenolpyruvate carboxykinase (PEPCK), it has been considered that this enzyme generates phosphoenolpyruvic acid from oxalacetic acid by decarboxylation, and it mainly advances the metabolism to induce glyconeogenesis (Non-patent documents 3 and 4). It is also known that there is another type of PEPCK as an enzyme that shows equilibrium for advancing the reverse reaction of the aforementioned reaction, i.e., the reaction that generates oxalacetic acid from phosphoenolpyruvic acid by carbon dioxide fixation. Presence of PEPCK of this type has been confirmed in some bacteria which has an ability to produce succinic acid in the presence of high concentration carbon dioxide, i.e., *Mannheimia succiniciproducens*, *Actinobacillus succinogenes*, *Anaerobiospirillum succini-ciproducens*, and *Selenomonas ruminantium* (Non-patent documents 5, 6, 7 and 9). As for methods for producing a non-amino organic acid utilizing a strain which has enhanced activity of PEPCK derived from the aforementioned bacteria, for example, it is known that enhancement of the activity of PEPCK derived from *Actinobacillus succinogenes* in *Escherichia coli* is effective for accumulation of succinic acid (Non-patent document 7). However, this effect was confirmed only in a PEPC-deficient strain, and it has also been reported that it is ineffective for accumulation of succinic acid in a non-deficient strain (Non-patent document 7).

Furthermore, although strains of *Enterobacter* bacteria efficiently producing ethanol and hydrogen have been reported, any strain efficiently producing a non-amino organic acid has not been known (Non-patent document 8 and Patent document 7).
Patent document 1: U.S. Patent No. 5,142,834
Patent document 2: U.S. Patent No. 5,504,004
Patent document 3: Japanese Patent Laid-open (KOKAI) No. 11-113588
Patent document 4: Japanese Patent Laid-open No. 11-196888
Patent document 5: U.S. Patent Published Application No. 20050170482
Patent document 6: Japanese Patent Laid-open No. 11-196887
Patent document 7: Japanese Patent Laid-open No. 2006-180782
Non-patent document 1: International Journal of Systematic Bacteriology (1999), 49, 207-216
Non-patent document 2: Journal of Industrial Microbiology and Biotechnology (2002), 28 (6), 325-332
Non-patent document 3: Applied and Environmental Microbiology (1996), 62, 1808-1810
Non-patent document 4: Applied and Environmental Microbiology (1993), 59, 4261-4265
Non-patent document 5: Applied and Environmental Microbiology (2006), 72, 1939-1948
Non-patent document 6: Applied and Environmental Microbiology (1997), 63, 2273-2280
Non-patent document 7: Applied and Environmental Microbiology (2004), 70, 1238-1241
Non-patent document 8: Journal of Bioscience and Bioengineering (2005), 100, 260-265
Non-patent document 9: Microbiology (2001), 147, 681-690

Kim, P. et al.: "Effect of Overexpression of Actinobacillus succinogenes Phosphoenolpyruvate Carboxykinase on Succinate Production in Escherichia coli", APPLIED AND ENVIRONMENTAL MICROBIOLOGY, AMERICAN SOCIETY FOR MICROBIOLOGY, US, vol. 70, no. 2, 1 February 2004, pages 1238-1241 discloses succinate production using a mutant strain of E. coli showing phosphoenolpyruvate carboxylase overexpression.
WO 2006/034156 A2 discloses genes which increase the production of succinic acid.
WO 2005/116227 A1 discloses genetically modified E. coli bacteria used in a method for producing succinic acid from raw hydrolysates.

### Disclosure of the Invention

### Object to be Achieved by the Invention

An object of the present invention is to provide a method for producing an organic acid using a bacterium, which shows higher production efficiency.

### Means for Achieving the Object

The inventors of the present invention conducted various researches in order to achieve the aforementioned object, as a result, found that production or yield of organic acid was increased by using a bacterium which belongs to genus *Enterobacter* and has been modified so that the phosphoenolpyruvate carboxykinase activity is enhanced or a product obtained by processing the bacterium, and thus accomplished the present invention.

That is, the present invention provides the followings.
[1] A method for producing succinic acid or malic acid comprising:
   allowing a bacterium belonging to the genus Enterobacter which has an ability to produce succinic acid or malic acid and has been modified so that the phosphoenolpyruvate carboxykinase activity is enhanced as compared to a parent strain or a wild-type strain or immobilized cells of said bacterium obtained by immobilizing the cells of said bacterium with acrylamide or carragheenan to act on an organic raw material in a reaction mixture containing carbonate ions, bicarbonate ions, or carbon dioxide gas to produce succinic acid or malic acid, and collecting succinic acid or malic acid,
   wherein said bacterium has been modified so that expression of the pckA gene coding for phosphoenolpyruvate carboxykinase is enhanced by increasing the copy number of the gene and/or by modifying an expression control sequence of the gene, and wherein the pckA gene is a DNA defined in (a) or (b):
   (a) a DNA comprising a nucleotide sequence of SEQ ID NO: 6, 8, 10, 12, 14, 16 or 64,
   (b) a DNA which hybridizes with a nucleotide sequence complementary to a nucleotide sequence of SEQ ID NO: 6, 8, 10, 12, 14, 16 or 64 under stringent conditions, and codes for a protein having the phosphoenolpyruvate carboxykinase activity.
[2] The method according to [1] above, wherein the pckA gene codes for a protein having an amino acid sequence of SEQ ID NO: 7, 9, 11, 13, 15, 17, 65 or 24, or a protein having an amino acid sequence of SEQ ID NO: 7, 9, 11, 13, 15, 17, 65 or 24 including substitutions, deletions, insertions or additions of one or several amino acid residues.
[3] The method according to [1] or [2] above, wherein the bacterium has been further modified so that one or more of enzymatic activities selected from alcohol dehydrogenase activity, lactate dehydrogenase activity, phosphate acetyltransferase activity, α-acetolactate decarboxylase activity, and pyruvate formate lyase activity are decreased.
[4] The method according to [3] above, wherein the bacterium has been modified so that the alcohol dehydrogenase activity is decreased.
[5] The method according to [3] above, wherein the bacterium has been modified so that the alcohol dehydrogenase activity and the lactate dehydrogenase activity are decreased.
[6] The method according to [3] above, wherein the bacterium has been modified so that the alcohol dehydrogenase activity, the lactate dehydrogenase activity, and the phosphate acetyltransferase activity are decreased.
[7] The method according to [3] above, wherein the bacterium has been modified so that the alcohol dehydrogenase activity, the lactate dehydrogenase activity, the phosphate acetyltransferase activity, and the α-acetolactate decarboxylase activity are decreased.
[8] The method according to [3] above, wherein the bacterium has been modified so that the alcohol dehydrogenase activity, the lactate dehydrogenase activity, the phosphate acetyltransferase activity, the α-acetolactate decarboxylase activity, and the pyruvate formate lyase activity are decreased.
[9] The method according to any one of [1] to [8] above, wherein the bacterium has been further modified so that pyruvate carboxylase activity is enhanced.
[10] The method according to any one of [1] to [9] above, wherein succinic acid or malic acid is succinic acid.
[11] The method according to [10] above, wherein the bacterium has been further modified so that the glucose PTS is decreased.
[12] A method for producing a succinic acid-containing polymer comprising the steps of:
   producing succinic acid by the method according to [10] or [11] above, and
   polymerizing the obtained succinic acid.

### Brief Description of the Drawings

Fig. 1 shows the structure of helper plasmid RSF-Red-TER.
Fig. 2 shows the construction scheme of helper plasmid RSF-Red-TER.
Fig. 3 shows alignment and consensus sequences of amino acid sequences of various types of PEPCK.
Fig. 4 shows alignment and consensus sequences of amino acid sequences of various types of PEPCK (continuation).
Fig. 5 shows alignment and consensus sequences of amino acid sequences of various types of PEPCK (continuation).
Fig. 6 shows alignment and consensus sequences of amino acid sequences of various types of PEPCK (continuation).

### Description of Preferred Embodiments

Hereinafter, the present invention will be explained in detail.

### <1> Bacterium of the present invention

The bacterium used in the method of the present invention is a bacterium which has an ability to produce an organic acid and has been modified so that the phosphoenolpyruvate carboxykinase (henceforth abbreviated as "PEPCK") activity is enhanced. The term "ability to produce an organic acid" used herein means an ability of the bacterium of the present invention to produce and accumulate an organic acid in a medium to such a degree that the organic acid can be collected from the medium when the bacterium of the present invention is cultured in the medium. In the present invention, the organic acid is succinic acid or malic acid. Preferably, the bacterium of the present invention is a bacterium that can accumulate a target organic acid in a medium in an amount of 0.5 g/L or more, more preferably 1.0 g/L or more. Such a bacterium can be obtained by modifying a bacterium which has an ability to produce an organic acid as a parent strain so that the PEPCK activity in the bacterium is enhanced. When the parent strain does not have the ability to produice an organic acid, such a bacterium can be obtained by imparting the ability to produice an organic acid to the parent strain and modifying the strain so that the PEPCK activity is enhanced. Furthermore, such a bacterium can also be obtained by imparting an ability to produce an organic acid to a strain which has been modified so that the PEPCK activity is enhanced. The bacterium having an ability to produce an organic acid may be a bacterium inherently having the ability to produce an organic acid, or may be a bacterium obtained by modifying a bacterium such as those mentioned below using mutation techniques or recombinant DNA techniques so that the bacterium has the ability to produce an organic acid.

The organic acid is succinic acid or malic acid.

The parent strain of the bacterium that can be used for the present invention is a bacterium belonging to the genus *Enterobacter. Enterobacter* bacteria are classified as γ-proteobacteria. (J. Gen. Appl. Microbiol., 1997, 43, 355-361; Int. J. Syst. Bacteriol., 1997, 43, 1061-1067). In recent years, some bacteria belonging to the genus *Enterobacter* were reclassified as *Pantoea agglomerans*, *Pantoea dispersa*, or the like, on the basis of DNA-DNA hybridization experiments etc. (Int. J. Syst. Bacteriol., 1989, 39:337-345).

Examples of the *Enterobacter* bacteria include *Enterobacter agglomerans*, *Enterobacter aerogenes,* and the like. Specifically, the strains exemplified in European Patent Application Laid-open No. 952221 can be used. Typical strains of the genus *Enterobacter* include *Enterobacter agglomerans* ATCC 12287 strain, *Enterobacter aerogenes* ATCC 13048 strain, *Enterobacter aerogenes* NBRC 12010 strain (Biotechnol Bioeng., 2007, Mar. 27;98(2):340-348), *Enterobacter aerogenes* AJ110637 strain (FERM ABP-10955), and the like.

These strains are available from, for example, the American Type Culture Collection (Address: 10801 University Boulevard, Manassas, VA 20110, United States of America). That is, registration numbers are given to each of the strains, and the strains can be ordered by using these numbers. The registration numbers of the strains are listed in the catalogue of the American Type Culture Collection. The *Enterobacter aerogenes* AJ110637 strain was deposited at International Patent Organism Depository, Agency of Industrial Science and Technology (Central 6, 1-1, Higashi 1-Chome, Tsukuba-shi, Ibaraki-ken, 305-8566, Japan) on August 22, 2007, and assigned an accession number of FERM P-21348. Then, the deposit was converted to an international deposit based on the Budapest Treaty on March 13, 2008, and assigned a receipt number of FERM BP-10955.

### <1-1> Impartation of an ability to produce an organic acid

Hereinafter, methods for imparting an ability to produce an organic acid to bacteria or methods for enhancing the ability to produce an organic acid of bacteria are described.

To impart an ability to produce an organic acid, methods conventionally employed in the breeding of bacteria for producing substances by fermentation (see "Amino Acid Fermentation", Japan Scientific Societies Press, 1st Edition, published May 30, 1986, pp. 77-100) can be applied. Such methods include the acquisition of an auxotrophic mutant, an analogue-resistant strain, or a metabolic regulation mutant, or construction of a recombinant strain having enhanced expression of an enzyme involved in the biosynthesis of an organic acid. In the breeding of bacteria which produce an organic acid, one or two or more properties, such as an auxotrophic mutation, analogue resistance, or metabolic regulation mutation, may be imparted. The expression of one or two or more enzymes involved in biosynthesis of organic acid can be enhanced. Furthermore, impartation of properties such as auxotrophy, analogue resistance, or metabolic regulation may be combined with the enhancement of biosynthetic enzymes.

An auxotrophic mutant strain, a strain resistant to an analogue of an organic acid, or a metabolic regulation mutant strain having an ability to produce an organic acid can be obtained by subjecting a parent or wild-type strain to a conventional mutagenesis, such as exposure to X-rays or UV irradiation, or treatment with a mutagen such as N-methyl-N'-nitro-N-nitrosoguanidine, and then selecting those which exhibit an auxotrophy, analogue resistance, or metabolic regulation mutation and which also have an ability to produce an organic acid.

Methods for imparting an ability to produce an organic acid to bacteria, and organic acid-producing bacteria will be specifically exemplified below.

### Succinic acid-producing bacteria

As succinic acid-producing bacteria, strains deficient in abilities to form acetic acid, lactic acid, ethanol, 2,3-butanediol and formic acid can be used.

Strains deficient in abilities to form acetic acid, lactic acid, ethanol, 2,3-butanediol and formic acid can be obtained by obtaining a strain that cannot assimilate acetic acid and lactic acid in a minimal medium, or by decreasing the activities of the lactic acid biosynthesis genes and acetic acid biosynthesis enzymes mentioned below (International Patent Publication WO2005/052135).

Moreover, such strains as described above can also be obtained by imparting monofluoroacetic acid resistance (U.S. Patent No. 5, 521, 075).

Other examples of the method for obtaining a strain in which succinic acid-producing ability is improved include imparting a glucose-assimilating ability under anaerobic conditions to a strain that is deficient in both formic acid-producing ability and lactic acid-producing ability (International Patent Publication WO97/16528).

The ability to produce succinic acid can also be imparted by amplification or deletion of a gene which is involved in the succinic acid biosynthesis system.

The An ability to produce succinic acid can also be imparted by modifying a bacterium to decrease the enzymatic activity of lactate dehydrogenase (LDH, hereafter abbreviations of enzyme names are mentioned in parentheses), which is a lactic acid biosynthesis system enzyme (International Patent Publications WO2005/052135, WO2005/116227 U.S. Patent No. 5,770,435, U.S. Patent Published Application No. 20070054387, International Patent Publication WO99/53035, Alam, K.Y. and Clark, D.P., 1989, J. Bacteriol., 171:6213-6217). Some bacteria may have L-lactate dehydrogenase and D-lactate dehydrogenase, and such bacteria may be modified so that activity of either one of the enzymes, preferably activities of both the enzymes, are decreased.

The ability to produce succinic acid can also be imparted by modifying a bacterium to decrease the enzymatic activity of the formic acid biosynthesis system enzyme, pyruvate-formate lyase (PFL) (U.S. Patent Published Application No. 20070054387, International Patent Publications WO2005/116227, WO2005/52135, Donnelly, M.I., Millard, C.S., Clark, D.P., Chen, M.J., Rathke, J.W., 1998, Appl. Biochem. Biotechnol., 70-72, 187-198.).

The ability to produce succinic acid can also be imparted by modifying a bacterium to decrease the enzymatic activities of phosphate acetyltransferase (PTA), acetate kinase (ACK), pyruvate oxidase (POXB), acetyl-CoA synthetase (ACS) and acetyl-CoA hydrolase (ACH), which are acetic acid biosynthesis system enzymes (U.S. Patent Published Application No. 20070054387, International Patent Publications WO2005/052135, WO99/53035, WO2006/031424, WO2005/113745, and WO2005/113744).

The ability to produce succinic acid can also be enhanced by modifying a bacterium to decrease the enzymatic activity of alcohol dehydrogenase (ADH), which is an ethanol biosynthesis system enzyme (refer to International Patent Publication WO2006/031424).

A strain having an enhanced ability to produce succinic acid can also be obtained by obtaining a strain having decreased activity of α-acetolactate decarboxylase, which is a 2,3-butanediol biosynthesis system enzyme (J. Biosci. Bioeng., 2004, 97(4):227-32).

The ability to produce succinic acid can also be enhanced by decreasing activities of pyruvate kinase, glucose PTS *(ptsG),* ArcA protein, IclR protein *(iclR),* glutamate dehydrogenase (*gdh*) and/or glutamine synthetase (*glnA*), and glutamate synthase *(gltBD)* (International Patent Publication WO2006/107127, No. 2007007933, Japanese Patent Laid-open No. 2005-168401). Mentioned here in the parentheses following the enzyme names are gene names.

The ability to produce succinic acid can also be imparted by enhancing a biosynthesis system enzyme involved in succinic acid production.

The ability to produce succinic acid can also be enhanced by enhancing enzymatic activities of pyruvate carboxylase, malic enzyme, phosphoenolpyruvate carboxylase, fumarase, fumarate reductase, and malate dehydrogenase (Japanese Patent Laid-open No. 11-196888, International Patent Publication WO99/53035, 2001. Biotechnol. Bioeng., 74:89-95, Millard, C.S., Chao, Y.P., Liao, J.C., Donnelly, M.I., 1996, Appl. Environ. Microbiol., 62:1808-1810, International Patent Publication WO2005/021770, Japanese Patent Laid-open No. 2006-320208, Pil Kim, Maris Laivenieks, Claire Vieille, and J. Gregory Zeikus, 2004, Appl. Environ. Microbiol., 70:1238-1241). Enhancement of enzymatic activities of these target enzymes can be performed by referring to the methods for enhancing expression of the *pckA* gene described later.

Specific examples of succinic acid-producing bacteria include *Enterobacter aerogenes* AJ110637 strain (FERM ABP-10955) and *Enterobacter aerogenes* VP-1 strain (J. Biosci. Bioeng., 2004, 97(4):227-32)

### <1-2> Enhancement of phosphoenolpyruvate carboxykinase

The bacterium of the present invention can be obtained by modifying a bacterium having an ability to produce an organic acid such as those described above so that the phosphoenolpyruvate carboxykinase (PEPCK) activity is enhanced. However, the modification to enhance the PEPCK activity is performed first, and then the ability to produce an organic acid may be imparted.

The phosphoenolpyruvate carboxykinase (PEPCK) referred to in the present invention is an enzyme that reversibly catalyzes the reaction of producing oxalacetic acid (OAA) from phosphoenolpyruvic acid (PEP) by carbon dioxide fixation. In the present invention, the PEPCK activity means the activity of catalyzing this reaction to produce OAA from PEP. As PEPCK used for the present invention, those showing reaction equilibrium for advancing the reaction to produce OAA from PEP are preferred. The enzyme activity can be determined by, for example, a method of measuring ATP production amount at 37°C according to the method of Pil Kim et al. using Sigma Diagnostics ATP Kit (Pil, Kim., Maris, Laivenieks., Claire, Vieille., Gregory, Zeikus., Applied And Environmental Microbiology, Feb. 2004, pp.1238-1241).

The increase of the PEPCK activity as compared to that of, for example, a wild-type or unmodified strain can be confirmed by measuring the enzyme activity according to the aforementioned method, or comparing amount of mRNA of a gene coding for PEPCK with that of the wild-type or unmodified strain. Examples of the method for confirming the expression level include Northern hybridization and reverse transcriptase PCR (RT-PCR, Sambrook, J., and Russell, D.W., Molecular Cloning A Laboratory Manual/Third Edition, New York: Cold Spring Harbor Laboratory Press (2001)). The enzyme activity may be increased to any level so long as the level is increased as compared to that of a wild-type or unmodified strain, and for example, it is desirably increased not less than 1.5 times, more preferably not less than 2 times, further preferably not less than 3 times, as compared to that of, for example, a wild-type or an unmodified strain. Moreover, the increase in the enzyme activity can also be confirmed on the basis of an increase in the amount of the PEPCK protein as compared to that in an unmodified or a wild-type strain, and it can be detected by, for example, Western blotting using an antibody (Sambrook, J., and Russell, D.W., Molecular Cloning A Laboratory Manual/Third Edition, New York: Cold Spring Harbor Laboratory Press (2001)).

Examples of the gene coding for PEPCK which can be used for the present invention include the *pckA* gene derived from *Actinobacillus succinogenes* (GenBank Accession No. YP_001343536.1, SEQ ID NO: 6), and homologues of this *pckA* gene. A *pckA* gene homologue is a gene which is derived from another microorganism, shows high homology to the aforementioned *pckA* gene of *Actinobacillus succinogenes,* and codes for a protein having the PEPCK activity. Examples include, for example, the *pckA* gene of *Haemophilus influenzae* (GenBank Accession No. YP_248516.1, SEQ ID NO: 8), the *pckA* gene of *Pasteurella multocida* (GenBank Accession No. NP_246481.1, SEQ ID NO: 10), the *pckA* gene of *Mannheimia succiniciproducens* (GenBank Accession No. Yp_089485.1, SEQ ID NO: 12), the *pckA* gene of *Yersinia pseudotuberculosis* (GenBank Accession No. YP_072243, SEQ ID NO: 14), the *pckA* gene of *Vibrio cholerae* (GenBank Accession No. ZP_01981004.1, SEQ ID NO: 16), the *pckA* gene of *Selenomonas ruminantium* (GenBank Accession No. AB016600, SEQ ID NO: 64), and so forth.

Examples of *pckA* gene homologues include genes coding for a protein showing a homology of 90% or more, preferably 95% or more, more preferably 98% or more, particularly preferably 99% or more, to the amino acid sequence of SEQ ID NO: 7, 9, 11, 13, 15, 17 or 65, and coding for phosphoenolpyruvate carboxykinase. Homology of amino acid sequences and nucleotide sequences can be determined by using, for example, the algorithm BLAST of Karlin and Altschul (Pro. Natl. Acad. Sci. USA, 90, 5873 (1993)) or FASTA (Methods Enzymol., 183, 63 (1990)). Programs called BLASTN and BLASTX have been developed on the basis of this algorithm BLAST (refer to http://www.ncbi.nlm.nih.govbi.nlm.nih.gov).

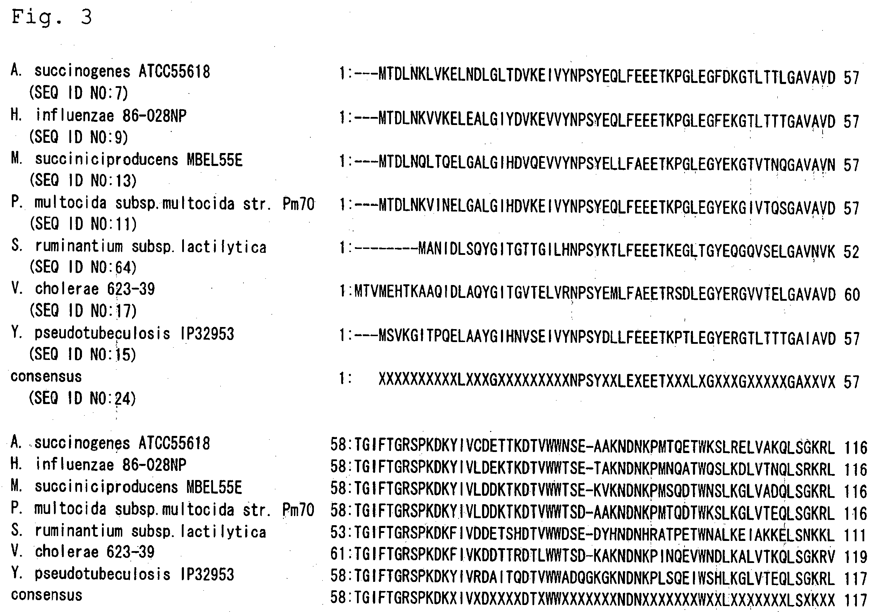

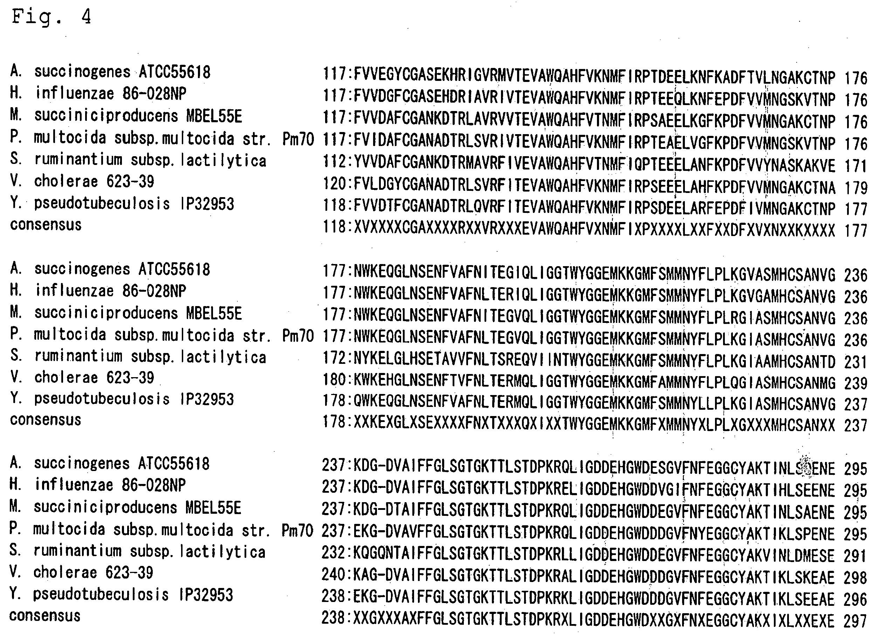

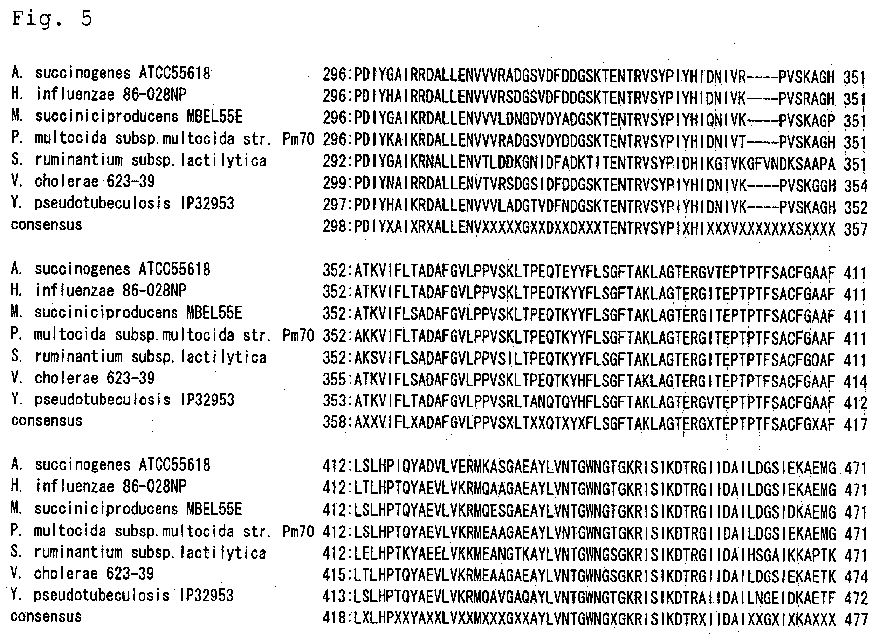

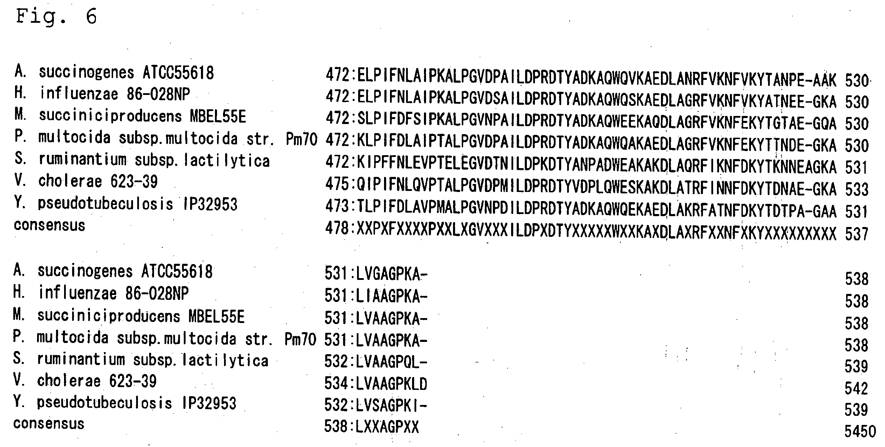

Alignment of the amino acid sequences of SEQ ID NOS: 7, 9, 11, 13, 15, 17 and 65 is shown in Figs. 3 to 6. A consensus sequence of these sequences is shown as SEQ ID NO: 24. The aforementioned *pckA* gene homologues include a gene coding for the amino acid sequence of SEQ ID NO: 24, and a genes coding for a protein showing a homology of 90% or more, preferably 95% or more, more preferably 98% or more, particularly preferably 99% or more, to the amino acid sequence of SEQ ID NO: 24, and coding for phosphoenolpyruvate carboxykinase.

Since several sequences of the *pckA* gene have already been elucidated as described above, the gene can be obtained by PCR using primers prepared on the basis of those nucleotide sequences. For example, the coding region of the *pckA* gene of *Actinobacillus succinogenes* and a flanking region thereof including a control region of the *pckA* gene can be obtained by PCR (polymerase chain reaction, see White, T.J. et al., Trends Genet., 5, 185 (1989)) using the primers shown in SEQ ID NOS: 4 and 5 and chromosomal DNA of *Actinobacillus succinogenes* as the template. Specific examples of *Actinobacillus succinogenes* include the 130Z strain (ATCC 55618). This strain can be obtained from American Type Culture Collection (Address: 10801 University Boulevard, Manassas, VA 20110, United States of America). Homologues of *pckA* of other microorganisms can also be obtained in a similar manner.

Since the nucleotide sequence of the *pckA* gene differs depending on the species or strains of bacteria belonging to the family *Enterobacteriaceae,* the *pckA* gene to be used for obtaining a bacterium used in the method of the present invention is not limited to a gene coding for the amino acid sequence of SEQ ID NO: 7, 9, 11, 13, 15, 17, 65 or 24, and it may be a mutant or artificially modified gene that codes for a protein having a sequence of SEQ ID NO: 7, 9, 11, 13, 15, 17, 65 or 24 including substitutions, deletions, insertions, additions, etc. of one or several amino acid residues at one or more positions so long as it remains to be a gene that improves organic acid-producing ability of a bacterium when expression of the gene is enhanced in the bacterium. Although number meant by the term "several" used herein may differ depending on positions in the three-dimensional structure of the protein or types of amino acid residues, it is preferably 1 to 20, more preferably 1 to 10, particularly preferably 1 to 5. The substitutions, deletions, insertions, additions, inversions or the like of amino acid residues described above also include those caused by naturally occurring mutation based on individual differences, differences in species of microorganisms that contain the *pckA* gene (mutant or variant), or the like.

The aforementioned substitution is preferably a conservative substitution that is a neutral substitution not providing functional change. The conservative mutation is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of substitution considered to be conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gin, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val.

Furthermore, as the *pckA* gene, there may be used a sequence encoding a protein showing a homology not less than 80%, preferably not less than 90%, more preferably not less than 95%, particularly preferably not less than 97%, to the total amino acid sequence of SEQ ID NO: 7, 9, 11, 13, 15, 17, 65 or 24 and coding for a protein which improves organic acid-producing ability of a bacterium when expression thereof is enhanced in the bacterium.

Furthermore, the degree of degeneracy of a gene varies depending on the hosts into which the *pckA* gene is introduced, and therefore codons may be replaced with those which are easily used by a host into which *pckA* is introduced. Moreover, the *pckA* gene may be a gene coding for a protein in which N- or C-terminal sequence is elongated or deleted, so long as the gene has a function of improving organic acid-producing ability of a bacterium when expression thereof is enhanced in the bacterium. The length of the amino acid sequence to be elongated or deleted may be 50 or less, preferably 20 or less, more preferably 10 or less, particularly preferably 5 or less, in terms of number of amino acid residues. More specifically, the *pckA* gene may be a gene coding for a protein having the amino acid sequence of SEQ ID NO: 7, 9, 11, 13, 15, 17, 65 or 24 with elongation or deletion of 5 to 50 amino acid residues on the N-terminal side or 5 to 50 amino acid residues on the C-terminal side.

Such genes homologous to the *pckA* gene as described above can be obtained by modifying a gene coding for the amino acid sequence of SEQ ID NO: 7, 9, 11, 13, 15, 17, 65 or 24 so that the protein encoded by the gene includes substitutions, deletions, insertions, or additions of amino acid residues at a specific site(s) by, for example, site-specific mutagenesis. Furthermore, such genes can also be obtained by conventionally known mutation treatment described below. As for the mutation treatment, by a method of treating the *pckA* gene with hydroxylamine or the like *in vitro,* or a method of treating a microorganism, for example, *Actinobacillus succinogenes*, containing the gene with ultraviolet ray irradiation or a mutagen used in a usual mutation treatment such as N-methyl-N'-nitro-N-nitrosoguanidine (NTG) or ethyl methanesulfonate (EMS), or by error-prone PCR (Cadwell, R.C., PCR Meth. Appl., 2, 28 (1992)), DNA shuffling (Stemmer, W.P., Nature, 370, 389 (1994)), or StEP-PCR (Zhao, H., Nature Biotechnol., 16, 258 (1998)), a mutation can be artificially introduced into the *pckA* gene by gene recombination to obtain a gene coding for highly active PEPCK.

Whether such *pckA* homologue genes code for a protein which improves organic acid-producing ability when expression thereof is enhanced can be confirmed by, for example, introducing these genes into a ΔadhE strain of the *Enterobacter aerogenes* AJ110637 strain (FERM BP-10955) or the like, and examining whether the organic acid-producing ability of the bacterium is improved or not. In this case, for example, by adding a reducing substance such as glucose and an organic acid such as malic acid to the medium, and comparing the amount of succinic acid or fumaric acid which is converted from the organic acid such as malic acid utilizing the reducing power obtained upon assimilation of glucose, the effect can be more clearly verified.

Examples of the *pckA* gene also include a DNA that hybridizes with a nucleotide sequence complementary to the sequence of SEQ ID NO: 6, 8, 10, 12, 14, 16 or 64, or a probe that can be prepared from the sequences under stringent conditions and codes for a protein which has the PEPCK activity. The "stringent conditions" referred to herein is a condition under which a so-called specific hybrid is formed, and non-specific hybrid is not formed. Examples include, for example, conditions under which DNAs showing high homology to each other, for example, DNAs showing a homology of, for example, not less than 80%, preferably not less than 90%, more preferably not less than 95%, particularly preferably not less than 97%, hybridize with each other, and DNAs having homology lower than the above level do not hybridize with each other, and conditions of washing in ordinary Southern hybridization, i.e., conditions of washing once, preferably twice or three times, at salt concentrations and temperatures of 1 x SSC, 0.1% SDS at 60°C, preferably 0.1 x SSC, 0.1% SDS at 60°C, more preferably 0.1 x SSC, 0.1% SDS at 68°C.

A partial sequence of a nucleotide sequence complementary to the sequence of SEQ ID NO: 6, 8, 10, 12, 14, 16 or 64 may also be used as the probe. Such a probe may be prepared by PCR using oligonucleotides prepared on the basis of any one of these nucleotide sequences as primers and a DNA fragment containing any one of the sequences as the template. When a DNA fragment having a length of about 300 bp is used as the probe, the washing conditions after hybridization under the aforementioned conditions can be exemplified by 2 x SSC, 0.1% SDS at 50°C.

The aforementioned descriptions concerning gene homologue and conservative mutations are similarly applied to the other enzyme genes described in this specification.

By modifying a bacterium so that expression of such a *pckA* gene as described above is enhanced, the PEPCK activity can be enhanced.

The expression "modified so that expression of the *pckA* gene is enhanced" means that the number of PEPCK molecules per cell is increased, or that the activity per PEPCK molecule is increased, etc., as compared to a parent strain or a wild-type strain. Examples of the wild-type strain to be used for comparison include the *Enterobacter aerogenes* ATCC 13048 strain and so forth as *Enterobacter aerogenes.*

Expression of the *pckA* gene may be enhanced by increasing the copy number of the *pckA* gene. For example, the copy number of the gene may be increased by ligating a fragment containing the *pckA* gene to a vector that functions in a target bacterium, preferably a multi copy vector, to prepare a recombinant DNA, and transforming such a bacterium having an ability to produce an organic acid as described above with the DNA. Alternatively, after such a recombinant DNA as described above is introduced into a wild type strain of a bacterium to obtain a transformant, an ability to produce an organic acid may be imparted to the transformant. The copy number of the gene may also be increased by transferring single copy or multiple copies of the *pckA* gene to a chromosome. Transfer of the *pckA* gene to the chromosome can be confirmed by Southern hybridization using a part of the *pckA* gene as a probe.

Expression of the *pckA* gene can also be enhanced by modifying an expression control sequence of the pckA gene. For example, the enhancement can be achieved by replacing a promoter sequence of the *pckA* gene with a stronger promoter, or by making a promoter sequence closer to a consensus sequence (WO00/18935).

Methods for constructing a bacterium which has an ability to produce an organic acid and has been modified so that the expression level of the *pckA* gene is increased are explained below. These methods can be performed as described in a manual such as Molecular Cloning (Cold Spring Harbor Laboratory Press, Cold Spring Harbor (USA), 2001).

Expression of the *pckA* gene can be enhanced by increasing the copy number of the *pckA* gene, and the copy number can be increased by amplifying the *pckA* gene using a plasmid as described below. First, the *pckA* gene is cloned from the chromosome of *Actinobacillus succinogenes* or the like. Chromosomal DNA can be prepared from a bacterium serving as a DNA donor by, for example, the method of Saito and Miura (see H. Saito and K. Miura, Biochem. Biophys. Acta, 72, 619 (1963); Text for Bioengineering Experiments, Edited by the Society for Bioscience and Bioengineering, Japan, p 97-98, Baifukan Co., Ltd., 1992) or the like. Oligonucleotides for use in PCR can be synthesized on the basis of the aforementioned known information, for example, the synthetic oligonucleotides shown in SEQ ID NOS: 4 and 5 can be used to amplify the *pckA* gene.

A gene fragment including the *pckA* gene amplified by PCR is preferably amplified by inserting the fragment into a vector having a replication origin that enables autonomous replication in a bacterium in which the gene is amplified any transforming the bacterium with the vector. Examples of vectors for introduction into *Enterobacteriaceae* bacteria include pUC19, pUC18, pHSG299, pHSG399, pHSG398, RSF1010, pBR322, pACYC184, pMW219, and the like.

To prepare a recombinant DNA by ligation of the *pckA* gene to a vector that functions in a bacterium in which the gene is to be amplified, the vector is digested with a restriction enzyme suitable for the ends of the *pckA* gene. Such a restriction enzyme site may be introduced in advance into the synthetic oligonucleotide which is used to amplify the *pckA* gene. Ligation is usually performed by using a ligase such as T4 DNA ligase.

In order to introduce a recombinant plasmid prepared as described above into a bacterium, any known transformation method reported to date may be employed. For example, there are a method of treating recipient cells with calcium chloride so as to increase permeability for the DNA, which has been reported for *Escherichia coli* K-12 (Mandel, M. and Higa, A., J. Mol. Biol., 53, 159 (1970)), and a method of using competent cells prepared from growing cells and introducing DNA into them, which has been reported for *Bacillus subtilis* (Duncan, C.H., Wilson, G.A. and Young, F.E., Gene, 1, 153 (1977)). Also employable is a method of making DNA recipient cells into protoplasts or spheroplasts which easily take up a recombinant DNA, and introducing a recombinant DNA into them, which are known for *Bacillus subtilis,* actinomycetes, and yeasts (Chang, S. and Choen, S.N., Mol. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Acad. Sci. USA, 75, 1929 (1978)). In addition, transformation of bacteria can also be performed by the electric pulse method (Japanese Patent Laid-open No. 2-207791) or by the conjugal transfer method (Biotechnology (NY). 1991 January; 9(1):84-7).

The copy number of the *pckA* gene can also be increased by integrating multiple copies of the *pckA* gene into the chromosomal DNA of a bacterium. In order to integrate multiple copies of the *pckA* gene into a chromosomal DNA of a bacterium, homologous recombination is performed by targeting a sequence which is present in multiple copies on the chromosomal DNA. As the sequences present on the chromosomal DNA in a multiple copy number, a repetitive DNA or inverted repeat present at the end of a transposable element can be used. Alternatively, as disclosed in Japanese Patent Laid-open No. 2-109985, multiple copies of the *pckA* gene can be introduced into a chromosomal DNA by incorporating them into a transposon and transferring it (Japanese Patent Laid-open Nos. 2-109985, 7-107976, Mol. Gen. Genet., 245, 397-405 (1994); Plasmid, 2000 November; 44(3): 285-91).

Expression level of the *pckA* gene can also be enhanced by replacing expression control sequence of the *pckA* gene on the chromosomal DNA or a plasmid such as promoter with a stronger promoter, by modifying a factor involved in expression control of the *pckA* gene such as operator or repressor, or by ligating a strong terminator (Hamilton et al., Journal of Bacteriology 171:4617-4622; WO98/004715). For example, the lac promoter, trp promoter, trc promoter, tac promoter, PR promoter derived from λ-phage, lacUV promoter, and the like are known as strong promoters. Methods for evaluating the strength of promoters and examples of strong promoters are described in the paper of Goldstein et al. (Prokaryotic promoters in biotechnology, Biotechnol. Annu. Rev., 1955, 1, 105-128), and the like. Furthermore, as disclosed in WO00/18935, a promoter can be made stronger by introducing nucleotide substitutions for several nucleotides in the promoter region of a target gene so as to make the sequence closer to a consensus sequence. For example, the -35 region may be replaced with TTGACA or TTGCCA sequence, and the -10 region may be replaced with TATAAT or TATAAC sequence. In addition, it is known that the translation efficiency of mRNA is significantly affected by the substitution of several nucleotides in the spacer sequence between the ribosome-binding site (RBS) and the translation initiation codon, in particular, the sequence immediately upstream of the initiation codon, and such a sequence may be modified.

Expression level of a gene can also be enhanced by extending the survival time of mRNA or by preventing degradation of an enzyme protein in the cells. An expression control sequence such as a promoter which is upstream of the *pckA* gene can also be identified by using a promoter search vector or gene analysis software such as GENETYX. Expression of the *pckA* gene is enhanced by such promoter substitution or modification.

Modifying an expression control sequence may be combined with increasing the copy number of the *pckA* gene.

In the method of the present invention, use of a bacterial strain which is modified so that activity of one or more enzymes among lactate dehydrogenase (LDH), α-acetolactate decarboxylase (α-ALDC), alcohol dehydrogenase (ADH), pyruvate formate lyase (PFL) and phosphate acetyltransferase (PTA) are decreased in addition to that the expression of the *pckA* gene is increased is more effective. The expression "modified so that lactate dehydrogenase activity is decreased" used herein means that the lactate dehydrogenase activity is decreased as compared to that of a strain in which lactate dehydrogenase is unmodified. The lactate dehydrogenase activity per cell is preferably decreased to 10% or lower of that of a strain in which the lactate dehydrogenase is unmodified. The lactate dehydrogenase activity may be completely deleted. The decrease of the lactate dehydrogenase activity can be confirmed by measuring the lactate dehydrogenase activity by a known method (Kanarek, L. and Hill, R.L., 1964, J. Biol. Chem., 239:4202). Specific examples of a method for producing a mutant strain of bacteria belonging to the family *Enterobacteriaceae* in which the lactate dehydrogenase activity is decreased include the method described in Alam, K.Y., and Clark, D.P., 1989, J. Bacteriol., 171:6213-6217, and the like.

In order to decrease or delete the activity of LDH, a mutation that decreases or deletes the intracellular activity of LDH can be introduced into the LDH gene on the chromosome by a usual mutagenesis method. For example, it can be attained by deleting the gene coding for LDH on the chromosome, or modifying an expression control sequence such as a promoter and the Shine-Dalgarno (SD) sequence by gene recombination. Furthermore, it can also be attained by introducing a mutation for amino acid substitution (missense mutation), or a stop codon (nonsense mutation), or a frame shift mutation that adds or deletes one or two nucleotides into the LDH coding on the chromosome, or by deleting the gene partially or entirely (Journal of Biological Chemistry 272:8611-8617 (1997)). Furthermore, the LDH activity can also be decreased or deleted by gene disruption, for example, by constructing a gene coding for a mutant LDH in which at least a part of the coding region is deleted, and substituting the constructed gene for the normal LDH gene on the chromosome by homologous recombination or the like, or by introducing a transposon or IS factor into the gene.

In order to introduce a mutation for reducing or deleting the LDH activity by genetic recombination, for example, the following methods are used. The LDH gene on the chromosome can be replaced with a mutant gene by preparing a mutant LDH gene in which a partial sequence of the LDH gene is modified so that it does not produce an enzyme that can normally function, and transforming a bacterium with a DNA containing the mutant gene to cause homologous recombination between the mutant gene and the gene on the chromosome. Such site-specific mutagenesis based on gene substitution utilizing homologous recombination has been already established, and there are a method called Red driven integration developed by Datsenko and Wanner (Proc. Natl. Acad. Sci. USA, 2000, vol. 97, No. 12, pp. 6640-6645), a method of using a linear DNA such as a method utilizing the Red driven integration in combination with an excision system derived from λ phage (Cho, E.H., Gumport, R.I., Gardner, J.F., J. Bacteriol., 184: 5200-5203 (2002)) (refer to WO2005/010175), a method of using a plasmid containing a temperature sensitive replication origin (U.S. Patent No. 6,303,383, Japanese Patent Laid-open No. 05-007491, WO2005/010175), and the like. Such site-specific mutagenesis based on gene substitution using homologous recombination as described above can also be performed by using a plasmid not having ability to replicate in a host.

As the LDH gene of *Enterobacter aerogenes,* the nucleotide sequence of the D-LDH gene (*ldhA*) of the *Enterobacter aerogenes* AJ110637 strain (FERM BP-10955) is shown in SEQ ID NO: 20, and the amino acid sequence encoded by this gene is shown in SEQ ID NO: 48.

The expression "modified so that alcohol dehydrogenase activity is decreased" used herein means that the alcohol dehydrogenase activity is decreased as compared to that of a strain in which the alcohol dehydrogenase is unmodified. The alcohol dehydrogenase activity per cell is preferably decreased to 10% or lower of that of a strain in which the alcohol dehydrogenase is unmodified. The alcohol dehydrogenase activity may be completely deleted. The decrease of the alcohol dehydrogenase activity can be confirmed by measuring the alcohol dehydrogenase activity by a known method (Lutstorf, U.M., Schurch, P.M. & von Wartburg, J. P. , Eur. J. Biochem., 17, 497-508 (1970)). Specific examples of the method for producing a mutant strain of bacterium belonging to the family *Enterobacteriaceae* in which the alcohol dehydrogenase activity is decreased include the method described in Sanehez A.M. et al., 2005, Biotechnol. Prog., 21:358-365, and the like. The bacterium of the present invention in which the alcohol dehydrogenase activity is decreased and expression of the *pckA* gene is enhanced can be obtained by, for example, preparing a bacterium in which the gene coding for alcohol dehydrogenase (ADH) is disrupted, and transforming this bacterium with a recombinant vector containing the *pckA* gene. However, either the modification for decreasing the ADH activity or the modification for enhancing expression of the *pckA* gene may be performed first. The alcohol dehydrogenase activity can be decreased by a method similar to the method for decreasing the lactate dehydrogenase activity described above.

As the ADH gene of *Enterobacter aerogenes,* the nucleotide sequence of the ADH gene *(adhE)* of the *Enterobacter aerogenes* AJ110637 strain (FERM ABP-10955) is shown in SEQ ID NO: 21, and the amino acid sequence encoded by this gene is shown in SEQ ID NO: 49.

The bacterium of the present invention in which the alcohol dehydrogenase activity is decreased and expression of the *pckA* gene is enhanced can be obtained by, for example, preparing a bacterium in which an ADH gene is disrupted, and transforming this bacterium with a recombinant vector containing the *pckA* gene, as described in the examples mentioned later. A bacterium in which the ADH activity and the LDH activity are decreased and expression of the *pckA* gene is enhanced can be obtained by, for example, preparing a bacterium in which an LDH gene is disrupted from a bacterium in which an ADH gene is disrupted, and transforming this bacterium with a recombinant vector containing the *pckA* gene, as described in the examples mentioned later.

The expression "modified so that phosphate acetyltransferase activity is decreased" used herein means that the phosphate acetyltransferase activity is decreased as compared to that of a phosphate acetyltransferase-unmodified strain. The phosphate acetyltransferase activity per cell is preferably decreased to 10% or lower of that of a strain in which the phosphate acetyltransferase is unmodified. The phosphate acetyltransferase activity may be completely deleted. The decrease of the phosphate acetyltransferase activity can be confirmed by measuring the phosphate acetyltransferase activity by a known method (Klotzsch, H.R., Meth. Enzymol., 12, 381-386 (1969)). The bacterium of the present invention in which the phosphate acetyltransferase activity is decreased and expression of the *pckA* gene is enhanced can be obtained by, for example, preparing a bacterium in which the PTA gene is disrupted, and transforming this bacterium with a recombinant vector containing the *pckA* gene. However, either the modification for decreasing the PTA activity or the modification for enhancing expression of the *pckA* gene may be performed first. The phosphate acetyltransferase activity can be decreased by a method similar to the method for decreasing the lactate dehydrogenase activity described above.

As the PTA gene of *Enterobacter aerogenes,* the nucleotide sequence of the PTA gene (*pta*) of the *Enterobacter aerogenes* AJ110637 strain (FERM ABP-10955) is shown in SEQ ID NO: 50, and the amino acid sequence encoded by this gene is shown in SEQ ID NO: 51. Although the first amino acid residue is indicated as Val using the universal code in these sequences, gtg may be used as the start codon in bacteria, and it is very likely to be actually Met.

The bacterium of the present invention in which the ADH activity, the LDH activity and the PTA activity are decreased, and expression of the *pckA* gene is enhanced can be obtained by, for example, preparing a bacterium in which the PTA gene is disrupted from a bacterium in which the ADH gene and the LDH gene are disrupted, and transforming this bacterium with a recombinant vector containing the *pckA* gene, as described in the examples mentioned later.

The expression "modified so that α-acetolactate decarboxylase activity is decreased" used herein means that the α-acetolactate decarboxylase activity is decreased as compared to that of an α-acetolactate decarboxylase-unmodified strain. The α-acetolactate decarboxylase activity per cell is preferably decreased to 10% or lower of that of a strain in which an α-acetolactate decarboxylase is unmodified. The α-acetolactate decarboxylase activity may be completely deleted. The decrease of the α-acetolactate decarboxylase activity can be confirmed by measuring the α-acetolactate decarboxylase activity by a known method (Juni, E.J., Biol. Chem., 195, 715-726 (1952)). The bacterium of the present invention in which the α-acetolactate decarboxylase is decreased and expression of the *pckA* gene is enhanced can be obtained by, for example, preparing a bacterium in which the α-ALDC gene (*α-aldc*) is disrupted, and transforming this bacterium with a recombinant vector containing the *pckA* gene. However, either the modification for decreasing the α-ALDC activity or the modification for enhancing expression of the *pckA* gene may be performed first. The α-ALDC activity can be decreased by a method similar to the method for decreasing the lactate dehydrogenase activity described above.

As the α-ALDC gene of *Enterobacter aerogenes,* the nucleotide sequence of the α-ALDC gene (*α-aldc*) of the *Enterobacter aerogenes* AJ110637 strain (FERM ABP-10955) is shown in SEQ ID NO: 52, and the amino acid sequence encoded by this gene is shown in SEQ ID NO: 53.

The bacterium of the present invention in which the ADH activity, the LDH activity, the PTA activity and the α-ALDC activity are decreased, and expression of the *pckA* gene is enhanced can be obtained by, for example, preparing a bacterium in which the α-ALDC gene is disrupted from a bacterium in which the ADH gene, the LDH gene and the PTA gene are disrupted, and transforming this bacterium with a recombinant vector containing the *pckA* gene, as described in the examples mentioned later.

The expression "modified so that pyruvate formate lyase activity is decreased" used herein means that the pyruvate formate lyase activity is decreased as compared to that of a pyruvate formate lyase-unmodified strain. The pyruvate formate lyase activity per cell is preferably decreased to 10% or lower of that of a strain in which the pyruvate formate lyase is unmodified. The pyruvate formate lyase activity may be completely deleted. The decrease of the pyruvate formate lyase activity can be confirmed by measuring the pyruvate formate lyase activity by a known method (Knappe, J. and Blaschkowski, H.P., 1975, Meth. Enzymol., 41:508-518). The bacterium of the present invention in which pyruvate formate lyase activity is decreased and expression of the *pckA* gene is enhanced can be obtained by, for example, preparing a bacterium in which PFL gene is disrupted, and transforming this microorganism with a recombinant vector containing the *pckA* gene. However, either the modification for decreasing the PFL activity or the modification for enhancing expression of *pckA* may be performed first. The pyruvate formate lyase activity can be decreased by a method similar to the method for decreasing the lactate dehydrogenase activity described above.

As the PFL gene of *Enterobacter aerogenes,* the nucleotide sequence of the PFL gene (*pflB*) of the *Enterobacter aerogenes* AJ110637 strain (FERM ABP-10955) is shown in SEQ ID NO: 54, and the amino acid sequence encoded by this gene is shown in SEQ ID NO: 55.

The bacterium of the present invention in which the ADH activity, the LDH activity, the PTA activity, the α-ALDC activity and the PFL activity are decreased, and expression of the *pckA* gene is enhanced can be obtained by, for example, preparing a bacterium in which the PFL gene is disrupted from a bacterium in which the ADH gene, the LDH gene, the PTA gene and the α-ALDC gene are disrupted, and transforming this bacterium with a recombinant vector containing the *pckA* gene, as described in the examples mentioned later.

For the reaction of the method of the present invention, a bacterium modified so that the pyruvate carboxylase (PC) activity is enhanced, in addition to that expression of the *pckA* gene is enhanced, may also be used. Enhancement of the pyruvate carboxylase activity may be combined with decrease of the activities of one or more enzymes among the lactate dehydrogenase (LDH), the α-acetolactate decarboxylase (α-ALDC), the alcohol dehydrogenase (ADH), the pyruvate formate lyase (PFL), and the phosphate acetyltransferase (PTA). The expression "modified so that pyruvate carboxylase activity is enhanced" means that the pyruvate carboxylase activity is increased as compared to that of an unmodified strain such as a wild-type strain or parent strain. The pyruvate carboxylase activity can be measured by, for example, a method of measuring decrease of NADH as described later (Moss, J. & Lane, M.D., Adv. Enzymol., 35, 321-442 (1971)).

As the PC gene used in the method of the present invention, a gene having an already determined nucleotide sequence or a gene obtained by isolating a DNA fragment encoding a protein having the PC activity from a chromosome of a microorganism, animal, plant, or the like and determining the nucleotide sequence may be used. After the nucleotide sequence is determined, a gene synthesized on the basis of that sequence may also be used.

As the PC gene, for example, a PC gene derived from a coryneform bacterium such as *Corynebacterium glutamicum* or *Brevibacterium flavum* (Peters-Wendisch, P.G. et al., 1998, Microbiology, vol. 144:915-927) (SEQ ID NO: 22) may be used. Furthermore, so long as the function of the encoded PC, i.e., the characteristic thereof concerning carbon dioxide fixation, is not substantially degraded, the PC gene may have the nucleotide sequence of SEQ ID NO: 22 including substitution of other nucleotides for a part of the nucleotides or deletion of them, insertion of other nucleotides, or dislocation of a part of the nucleotide sequence, and any type of such derivatives may be used for the present invention. A DNA that hybridizes with a DNA having the nucleotide sequence of SEQ ID NO: 22 under stringent conditions, or a DNA showing a homology of 90% or more, preferably 95% or more, more preferably 99% or more, to the nucleotide sequence of SEQ ID NO: 22 and coding for a protein having the PC activity can also be preferably used. Examples of the stringent conditions referred to here include conditions of washing in ordinary Southern hybridization, i.e., conditions for hybridization at salt concentrations and temperature of 1 x SSC, 0.1% SDS at 60°C, preferably 0.1 x SSC, 0.1% SDS at 60°C.

PC genes obtained from any of bacteria other than *Corynebacterium glutamicum*, other microorganisms, animals and plants can also be used. In particular, sequences of PC genes derived from microorganisms, animals and plants described below are known (references are indicated below), and they can be obtained by hybridization or amplification of the ORF regions by PCR in the same manner as described above.
Human [Biochem. Biophys. Res. Comm., 202, 1009-1014, (1994)]
Mouse [Proc. Natl. Acad. Sci. USA., 90, 1766-1779, (1993)] Rat [GENE, 165, 331-332, (1995)]
Yeast: *Saccharomyces cerevisiae* [Mol. Gen. Genet., 229, 307-315, (1991)],
*Schizosaccharomyces pombe* [DDBJ Accession No.; D78170] *Bacillus stearothermophilus* [GENE, 191, 47-50, (1997)] *Rhizobium etli* [J. Bacteriol., 178, 5960-5970, (1996)]

The PC gene can be enhanced in the same manner as those used for enhancing expression of the *pckA* gene described above.

The activity of phosphoenolpyruvate carboxylase (PEPC) of the bacterium of the present invention may not be decreased or eliminated, and the gene thereof may be a wild-type gene.

### <2> Method for producing organic acid of the present invention

In the present invention, an organic acid is produced by using such a bacterium having an ability to produce an organic acid and modified so that expression of the *pckA* gene is enhanced as described above. Specifically, an organic acid can be produced by allowing the bacterium of the present invention or a product obtained by processing the bacterium to act on an organic raw material in a reaction mixture containing carbonate ions, bicarbonate ions, or carbon dioxide gas to produce the organic acid, and collecting the organic acid.

In the first embodiment, by culturing the bacterium in a medium containing carbonate ions, bicarbonate ions, or carbon dioxide gas, and an organic raw material, proliferation of the bacterium and production of the organic acid are simultaneously attained. In this embodiment, the medium corresponds to the reaction mixture. Proliferation of the bacterium and production of the organic acid can be simultaneously attained, or there can be a period during the culture when proliferation of the bacterium mainly occurs, and a period in which production of the organic acid mainly occurs.

In the second embodiment, by allowing cells proliferated by culture in a medium to coexist with a reaction mixture containing carbonate ions, bicarbonate ions, or carbon dioxide gas, and an organic raw material, and thereby allowing the bacterium to act on the organic raw material in the reaction mixture, an organic acid is produced. In this embodiment, a product obtained by processing the cells of the bacterium can also be used. Examples of the product obtained by processing cells include, for example, immobilized cells obtained by immobilizing cells with acrylamide or carragheenan.

Although the bacteria used for the culture may be those obtained on a solid medium such as agar medium by slant culture, those cultured beforehand in a liquid medium (seed culture) are preferred.

As the medium used for the culture, a medium usually used for culture of bacteria can be used. For example, a generally-used medium obtained by adding natural nutrients such as meat extract, yeast extract and peptone, to a composition including inorganic salts such as ammonium sulfate, potassium phosphate and magnesium sulfate can be used.

In the aforementioned first embodiment, the carbon source that is added to the medium also serves as the organic raw material for the production of the organic acid.

In the aforementioned second embodiment, after the culture, the cells are collected by centrifugation, membrane separation, or the like, and used for the organic acid production reaction.

The organic raw material used in the method of the present invention is not particularly limited so long as a carbon source, which the bacterium of the present invention can assimilate to produce succinic acid, is used. However, fermentable carbohydrates including carbohydrates such as galactose, lactose, glucose, fructose, glycerol, sucrose, saccharose, starch and cellulose, polyalcohols such as glycerin, mannitol, xylitol and ribitol, and the like are usually used. Among these, glucose, fructose and glycerol are preferred, and glucose is particularly preferred. When the organic acid is succinic acid, fumaric acid or the like may be added in order to efficiently produce succinic acid as described in Japanese Patent Laid-open No. 5-68576, and malic acid may be added instead of fumaric acid.

Furthermore, a saccharified starch solution, molasses, or the like containing the aforementioned fermentable carbohydrates may also be used. Those fermentable carbohydrates may be used independently or in combination. Although the concentration of the aforementioned organic raw material is not particularly limited, it is more advantageous when the concentration is as high as possible within such a range that the production of the organic acid is not inhibited. In the aforementioned first embodiment, concentration of the organic raw material in the medium is generally in the range of 5 to 30% (w/v), preferably 10 to 20% (w/v). Furthermore, in the aforementioned second embodiment, the concentration of the organic raw material in the reaction mixture is generally in the range of 5 to 30% (w/v), preferably 10 to 20% (w/v). Furthermore, the organic raw material may be supplemented along with decrease of the organic raw material with progress of the reaction.

The aforementioned reaction mixture containing carbonate ions, bicarbonate ions, or carbon dioxide gas and the organic raw material is not particularly limited, and it may be, for example, a medium for culturing bacteria, or it may be a buffer such as phosphate buffer. The reaction mixture is preferably an aqueous solution containing a nitrogen source, inorganic salts and the like. The nitrogen source is not particularly limited so long as it is a nitrogen source which the bacterium of the present invention can assimilate to produce an organic acid is chosen, and specific examples include various organic or inorganic nitrogen compounds such as ammonium salts, nitrates, urea, soybean hydrolysate, casein degradation products, peptone, yeast extract, meat extract, and corn steep liquor. Examples of the inorganic salts include various phosphates, sulfates, and metallic salts such as those of magnesium, potassium, manganese, iron, and zinc. If necessary, growth-promoting factors including vitamins such as biotin, pantothenic acid, inositol, and nicotinic acid, nucleotides, amino acids and the like are added. In order to suppress foaming at the time of the reaction, it is desirable to add an appropriate amount of a commercially available antifoam to the medium.

pH of the reaction mixture can be adjusted by adding sodium carbonate, sodium bicarbonate, potassium carbonate, potassium bicarbonate, magnesium carbonate, sodium hydroxide, calcium hydroxide, magnesium hydroxide, or the like. Since pH for the reaction is usually 5 to 10, preferably 6 to 9.5, pH of the reaction mixture is adjusted to be within the aforementioned range with an alkaline substance, carbonate, urea, or the like even during the reaction, if needed.

As the reaction mixture used in the present invention, water, buffer, medium or the like is used, but a medium is most preferred. The medium can be made to contain, for example, the aforementioned organic raw material, and carbonate ions, bicarbonate ions, or carbon dioxide gas, and the reaction can be performed under anaerobic conditions. The carbonate or bicarbonate ions are supplied from magnesium carbonate, sodium carbonate, sodium bicarbonate, potassium carbonate, or potassium bicarbonate, which can also be used as a neutralizing agent. However, if necessary, carbonate or bicarbonate ions may also be supplied from carbonic acid or bicarbonic acid or salts thereof or carbon dioxide gas. Specific examples of the salts of carbonic acid or bicarbonic acid include, for example, magnesium carbonate, ammonium carbonate, sodium carbonate, potassium carbonate, ammonium bicarbonate, sodium bicarbonate, potassium bicarbonate, and the like. Carbonate ions or bicarbonate ions may be added at a concentration of 0.001 to 5 M, preferably 0.1 to 3 M, more preferably 1 to 2 M. When carbon dioxide gas is contained, carbon dioxide gas is contained in an amount of 50 mg to 25 g, preferably 100 mg to 15 g, more preferably 150 mg to 10 g, per liter of the solution.

The optimal growth temperature of the bacterium used for the reaction is generally in the range of 25 to 40°C. The reaction temperature is generally in the range of 25 to 40°C, preferably in the range of 30 to 37°C. Amount of bacterial cells in the reaction mixture is, although it is not particularly limited, 1 to 700 g/L, preferably 10 to 500 g/L, more preferably 20 to 400 g/L. The reaction time is preferably 1 to 168 hours, more preferably 3 to 72 hours. The reaction may be performed batchwise or on a column.

Culture of the bacterium is preferably performed under aerobic conditions. On the other hand, the organic acid production reaction may be performed under aerobic conditions, microaerobic conditions or anaerobic conditions. For the reaction under microaerobic conditions or anaerobic conditions, a method of performing the reaction in a sealed reaction vessel without aeration, a method of performing the reaction with supplying an inert gas such as nitrogen gas to the reaction mixture, a method of performing the reaction with supplying an inert gas containing carbon dioxide gas to the reaction mixture, and the like can be used.

The organic acid that accumulates in the reaction mixture (culture medium) may be separated and purified from the reaction mixture in a conventional manner. Specifically, solids such as bacterial cells can be removed by centrifugation, filtration, or the like, and then the resulting solution can be desalted with an ion exchange resin or the like, and the organic acid can be separated and purified from the solution by crystallization or column chromatography.

Furthermore, in the present invention, when the target organic acid is succinic acid, after succinic acid is produced by the method of the present invention described above, a polymerization reaction can be carried out by using the obtained succinic acid as a raw material to produce a polymer containing succinic acid. In recent years, with the increase of environmentally friendly industrial products, polymers prepared from raw materials of plant origin have been attracting attention. Succinic acid produced in the present invention can be converted into polymers such as polyesters and polyamides and used (Japanese Patent Laid-open No. 4-189822). Specific examples of succinic acid-containing polymers include succinic acid polyesters obtainable by polymerizing a diol such as butanediol and ethylene glycol and succinic acid, succinic acid polyamides obtainable by polymerizing a diamine such as hexamethylenediamine and succinic acid, and the like. In addition, succinic acid and succinic acid-containing polymers obtained by the production method of the present invention, and compositions containing these can be used for food additives, pharmaceutical agents, cosmetics, and the like.

### Examples

Hereinafter, the present invention will be explained more specifically with reference to examples.

### Example 1

### <1-1> Acquisition of threonine operon promoter fragment of Escherichia coli MG1655 strain

The entire genomic nucleotide sequence of *Escherichia coli (Escherichia coli* K-12 strain) has already been elucidated (Genbank Accession No.U00096, Science, 277, 1453-1474 (1997)). On the basis of this sequence, PCR amplification of the promoter region of the threonine operon *(thrLABC)* was performed. PCR was performed by using the synthetic oligonucleotide having an *Sac*I site shown in SEQ ID NO: 1 as a 5' primer, the synthetic oligonucleotide shown in SEQ ID NO: 2 as a 3' primer, and the genomic DNA of *Escherichia coli* MG1655 strain (ATCC 47076, ATCC 700926) as the template to obtain a threonine operon promoter fragment (A) (SEQ ID NO: 3).

### <1-2> Acquisition of phosphoenolpyruvate carboxykinase gene fragment of Actinobacillus succinogenes 130Z strain (ATCC 55618)

The entire genomic nucleotide sequence of the *Actinobacillus succinogenes* 130Z strain has also already been published (GenBank Accession No. CP000746). Primers were designed on the basis of the nucleotide sequence of the gene coding for PEPCK (gene name: *pckA*), and used to perform PCR amplification. PCR was performed by using the synthetic oligonucleotide shown in SEQ ID NO: 4 as a 5' primer, the synthetic oligonucleotide having an *Sac*I site shown in SEQ ID NO: 5 as a 3' primer, and the genomic DNA of the *Actinobacillus succinogenes* 130Z strain as the template to obtain a *pckA* gene fragment (B) (SEQ ID NO: 6).

### <1-3> Construction of plasmid for pckA gene amplification

PCR was performed by using the fragments (A) and (B) as templates, and the primers of SEQ ID NOS: 1 and 5 having the *Sal*I site to obtain a gene fragment (C) consisting of the fragments (A) and (B) ligated to each other. This gene fragment (C) was treated with the restriction enzyme *Sac*I, and purified, and the product was ligated with the plasmid vector pSTV28 (Takara Bio) digested with the restriction enzyme *Sac*I to construct a plasmid pSTV28::Pthr::pckA for *pckA* amplification.

### <1-4> Preparation of pckA-amplified strain of Enterobacter aerogenes AJ110637 strain (FERM BP-10955)

The *Enterobacter aerogenes* AJ110637 strain (FERM BP-10955, refer to Reference Example 1) was transformed with pSTV28::Pthr::pckA obtained above and pSTV28 by the electric pulse method, applied to an LB agar medium containing 40 µg/ml of chloramphenicol, and cultured at 37°C for about 18 hours. The colonies that appeared were purified, and plasmids were extracted in a conventional manner to confirm that the target plasmids were introduced. The obtained strains were designated *Enterobacter aerogenes* AJ110637 pSTV28::Pthr::pckA and *Enterobacter aerogenes* AJ110637 pSTV28, respectively.

### Example 2: Effect of pckA amplification in succinic acid-producing strain of Enterobacter bacterium

The *Enterobecter aerogenes* AJ110637
pSTV28::Pthr::pckA and the *Enterobacter aerogenes* AJ110637 pSTV28 were each uniformly applied to an LB plate containing 40 µg/ml of chloramphenicol, and cultured at 37°C for 16 hours. Then, each plate was put into Anaeropack (for compromised culture of anaerobes, Mitsubishi Gas Chemical, product number A-04), and incubated at 37° C for 16 hours under anaerobic conditions. The obtained cells on the plate were washed with 0.8% brine, and then prepared so that a cell suspension has an

OD = 1.0 (600 nm) after 51-times dilution. This cell suspension in a volume of 100 µl and a production medium in a volume of 1.3 ml in which dissolving gases in the medium were replaced with carbon dioxide gas beforehand were put into a 1.5-ml volume microtube, and the cells were cultured at 31.5°C for 10 hours by using a microtube shaker. The composition of the production medium is shown below.

### Composition of organic acid production medium for Enterobacter bacteria

**[Plot A]**

| | |
|---|---|
| Glucose | 40 g/L |
| | (final concentration) |
| Magnesium sulfate heptahydrate | 1 g/L |
| [Plot B] | |
| Ammonium sulfate | 1 g/L |
| Potassium dihydrogenphosphate | 1 g/L |
| Manganese sulfate pentahydrate | 10 mg/L |
| Iron sulfate heptahydrate | 10 mg/L |
| Yeast Extract | 2 g/L |
| Biotin | 1 mg/L |
| (adjusted to pH 5.5 with KOH) | |
| [Plot C] | |
| Calcium carbonate | 50 g/L |
| (Japanese Pharmacopoeia) | |

The ingredients of the plots A and B were sterilized at 115°C for 10 minutes by autoclaving, the ingredient of the plot C was sterilized at 180°C for 3 hours with dry heat, and then left to cool, and the ingredients were mixed.

After the culture, amount of the organic acid accumulated in the medium was analyzed by liquid chromatography. Two of Shim-pack SCR-102H (Shimadzu) connected in series were used as the column, and a sample was eluted at 50°C with 5 mM p-toluenesulfonic acid. The eluate was neutralized with 20 mM Bis-Tris aqueous solution containing 5 mM p-toluenesulfonic acid and 100 µM EDTA, and the organic acid was quantified by measuring electric conductivity with CDD-10AD (Shimadzu). The amount of consumed saccharide, change of OD, accumulated organic acid and yield based on the consumed saccharide determined after 24 hours are shown in Table 1.

**Table 1**

| | pSTV28 | pSTV28::Pthr::pckA |
|---|---|---|
| Amount of consumed saccharide (g/L) | 16.6 (± 0.78) | 13.6 (± 0.13) |
| ΔOD (600 nm) | 1.54 (± 0.08) | 2.27 (± 0.07) |
| Accumulated malic acid (g/L) | 0.04 (± 0.01) | 0.20 (± 0.02) |
| Yield of malic acid based on consumed saccharide (%) | 0.24 (± 0.05) | 1.45 (± 0.15) |
| Accumulated succinic acid (g/L) | 0.63 (± 0.01) | 2.97 (± 0.13) |
| Yield of succinic acid based on consumed saccharide (%) | 3.80 (± 0.12) | 21.8 (± 0.75) |

The *pckA* gene-amplified strain, *Enterobacter aerogenes* AJ110637 pSTV28::Pthr::pckA provided markedly improved accumulation of malic acid and succinic acid and yields thereof based on consumed saccharide compared with the control, *Enterobacter aerogenes* AJ110637 pSTV28.

### Example 3

### <3-1> Construction of adhE-deficient strain of Enterobacter aerogenes AJ110637

When *Enterobacter aerogenes* AJ110637 is grown in a medium containing a sugar source, it produces a marked amount of ethanol. Therefore, *adhE* coding for alcohol dehydrogenase was deleted to suppress production of ethanol.

A gene fragment for deletion of *adhE* was prepared by PCR using a plasmid pMW-attL-Tc-attR described in WO2005/010175 as the template and oligonucleotides of SEQ ID NOS: 18 and 19 as primers. pMW118-attL-Tc-attR is a plasmid obtained by inserting *attL* and *attR* genes, which are the attachment sites of λ phage, and the *Tc* gene, which is an antibiotic resistance gene, into pMW118 (Takara Bio), and the genes are inserted in the order of *attL-Tc-attR* (see Reference Example 3). By PCR described above, a gene fragment containing a tetracycline resistance gene, *attL* and *attR* sites of λ phage at the both ends of tetracycline gene, and 60 bp upstream sequence and 59 bp downstream sequence of the *adhE* gene added to the outer ends of the A phage sequences was amplified. This fragment was purified by using Wizard PCR Prep DNA Purification System (Promega).

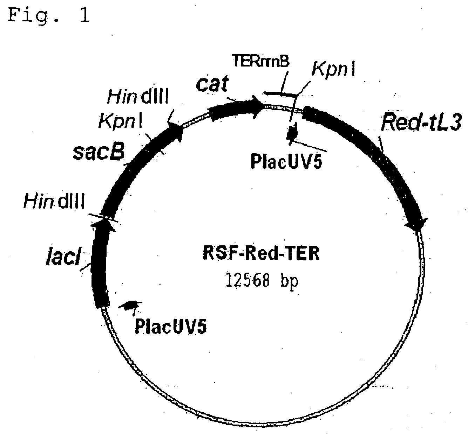

Then, the *Enterobacter aerogenes* AJ110637 strain was transformed with RSF-Red-TER (see Fig. 1, Reference Example 2) to obtain *Enterobacter aerogenes* AJ110637/RSF-Red-TER strain. This strain was cultured overnight in the LB medium containing 40 µg/mL of chloramphenicol, the culture medium was inoculated in a 1/100 volume to 50 mL of the LB medium containing 40 µg/mL of chloramphenicol and 0.4 mM isopropyl-β-D-thiogalactopyranoside, and culture was performed at 31°C for 4 hours. The cells were collected, washed three times with ice-cooled 10% glycerol, and finally suspended in 0.5 mL of 10% glycerol. The suspended cells were used as competent cells, and 500 ng of the PCR fragment prepared in the above section was introduced into the cells by using GENE PULSER II (BioRad) under the conditions of a field strength of 20 kV/cm, capacitor capacity of 25 µF and resistance of 200 Q. Ice-cooled SOC medium (20 g/L of Bacto tryptone, 5 g/L of yeast extract, 0.5 g/L of NaCl, 10 g/L of glucose) was added to the cell suspension, and culture was performed at 31°C for 2 hours with shaking. Then, the culture was applied to a LB plate containing 25 µg/mL of tetracycline. The colonies that appeared were purified with the same plate, and then it was confirmed by PCR that the *adhE* gene was replaced with the tetracycline resistance gene.

Then, in order to eliminate the RSF-Red-TER plasmid from each recombinant strain obtained as described above, the strains were applied to an LB medium containing 10% sucrose and 1 mM IPTG, and cultured overnight at 37°C. A strain from which the chloramphenicol resistance was deleted was selected as AJ110637ΔadhE from the colonies that appeared.

### <3-2> Construction of pckA-amplified strain of Enterobacter aerogenes AJ110637ΔadhE

The *Enterobacter aerogenes* AJ110637ΔadhE strain obtained above was transformed with the pSTV28::Pthr::pckA plasmid for amplification of *pckA* and pSTV28, applied to an LB agar medium containing 40 µg/ml of chloramphenicol and 25 µg/ml of tetracycline, and cultured at 37°C for about 18 hours. The colonies that appeared were purified, and plasmids were extracted in a conventional manner to confirm that the target plasmids were introduced. The obtained strains were designated *Enterobacter aerogenes* AJ110637ΔadhE+pSTV28::Pthr::pckA and *Enterobacter aerogenes* AJ110637ΔadhE+pSTV28, respectively.

### <3-3> Effect of pckA amplification in succinic acid-producing strain of Enterobacter bacterium having characteristic of alcohol dehydrogenase deficiency

Succinic acid-producing abilities of *Enterobacter aerogenes* AJ110637ΔadhE+pSTV28::Pthr::pckA and *Enterobacter aerogenes* AJ110637ΔadhE+pSTV28 were compared by using the same evaluation system as that mentioned above. The results obtained after 45 hours are shown in Table 2.

**Table 2**

| | ΔadhE+pSTV28 | ΔadhE+pSTV28::Pthr::pckA |
|---|---|---|
| Amount of consumed saccharide (g/L) | 3.29 (± 0.43) | 7.45 (± 0.95) |
| ΔOD (600 nm) | 0.30 (± 0.10) | 0.85 (± 0.18) |
| Accumulated succinic acid (g/L) | 0.40 (± 0.10) | 5.19 (± 0.69) |
| Yield of succinic acid based on consumed saccharide (%) | 11.95 (± 1.48) | 69.61 (± 0.39) |

The *pckA* gene-amplified strain, *Enterobacter aerogenes* AJ110637ΔadhE+pSTV28::Pthr::pckA, showed markedly increased accumulation of succinic acid and yield thereof based on consumed saccharide compared with *Enterobacter aerogenes* AJ110637ΔadhE+pSTV28 as the control.

### Example 4

### <4-1> Construction of acetic acid, lactic acid, 2,3-butanediol and formic acid synthesis gene-deficient strain from Enterobacter aerogenes AJ110637ΔadhE

If AJ110637ΔadhE is allowed to grow in a medium containing a sugar source, it produces acetic acid, lactic acid, 2,3-butanediol and formic acid in the medium. Therefore, a strain showing further improved succinic acid-producing ability was constructed by deleting synthesis pathways of these substances.

### <4-2> Deletion of drug resistance gene from AJ110637ΔadhE

In order to remove the tetracycline resistance gene from AJ110637ΔadhE, the RSF-int-xis plasmid was used (refer to Reference Example 4). RSF-int-xis was introduced into the gene-disrupted strain by the electric pulse method, and the transformant was applied to an LB medium containing 40 µg/ml of chloramphenicol, and cultured at 30°C to obtain a strain harboring RSF-int-xis. The obtained plasmid-harboring strain was purified on an LB medium containing 40 µg/ml of chloramphenicol and 1 mM IPTG, and plural single colonies were obtained. The obtained strains were applied to the medium to which 25 µg/ml of tetracycline was added, and cultured overnight at 37°C, and it was confirmed that they could not grow. Thus, they were confirmed to be strains from which the antibiotic resistance gene was removed. Then, in order to eliminate the RSF-int-xis plasmid from the obtained strains, they were applied to an LB medium to which 10% sucrose and 1 mM IPTG were added, and cultured overnight at 37°C. A strain in which the chloramphenicol resistance was deleted was selected from the colonies that appeared, and used for deletion of biosynthetic pathways of acetic acid, lactic acid, 2,3-butanediol and formic acid.

### <4-3> Construction of D-lactate dehydrogenase-deficient strain

Production of lactic acid can be suppressed by deleting lactate dehydrogenase. The *ldhA* gene coding for D-lactate dehydrogenase was disrupted as follows. By performing PCR using the oligonucleotides shown in SEQ ID NOS: 56 and 57 and pMW-attL-Km-attR as a template, AJ110687ΔadhEΔldhA was constructed from AJ110637ΔadhE in which the drug resistance gene was deleted in the same manner as that of Example 3 described above. The obtained strain was designated ES02.

### <4-4> Construction of phosphate acetyltransferase-deficient strain

Production of acetic acid can be suppressed by deleting phosphate acetyltransferase. The *pta* gene coding for phosphate acetyltransferase was disrupted as follows. By performing PCR using the oligonucleotides shown in SEQ ID NOS: 58 and 59 and pMW-attL-Km-attR as a template, AJ110637ΔadhEΔldhAΔpta was constructed from AJ110637ΔadhEΔldhA in which the drug resistance gene was deleted in the same manner as that of Example 3 mentioned above. The obtained strain was designated ES03.

### <4-5> Construction of α-acetolactate decarboxylase-deficient strain

Production of 2,3-butanediol can be suppressed by deleting α-acetolactate decarboxylase. The *α-aldc* gene coding for α-acetolactate decarboxylase was disrupted as follows. By performing PCR using the oligonucleotides shown in SEQ ID NOS: 60 and 61 and pMW-attL-Km-attR as a template, AJ110637ΔadhEΔldhAΔptaΔα-aldc was constructed from AJ110637ΔadhEΔldhAΔpta in which the drug resistance gene was deleted in the same manner as that of Example 3 described above. The obtained strain was designated ES04.

### <4-6> Construction of pyruvate formate lyase-deficient strain

Production of formic acid can be suppressed by deleting pyruvate formate lyase. The *pflB* gene coding for pyruvate formate lyase was disrupted as follows. By performing PCR using the oligonucleotides shown in SEQ ID NOS: 62 and 63 and pMW-attL-Km-attR as a template, AJ110637ΔadhEΔldhAΔptaΔα-aldcΔpflB was constructed from AJ110637ΔadhEΔldhAΔptaΔα-aldc in which the drug resistance gene was deleted in the same manner as that of Example 3 mentioned above. The obtained strain was designated ES05.

### Example 5: Effect of pckA amplification on succinic acid fermentation in various deficient strains

Each of the ES02, ES03, ES04 and ES05 strains obtained above was transformed with pSTV28::Pthr::pckA and pSTV28 by the electric pulse method, applied to an LB agar medium containing 40 µg/ml of chloramphenicol, and cultured at 37°C for about 18 hours. The colonies that appeared were purified, and plasmids were extracted in a conventional manner to confirm that the target plasmids were introduced. The obtained strains were designated ES02/pSTV28, ES02/pSTV28::Pthr::pckA, ES03/pSTV28, ES03/pSTV28::Pthr::pckA, ES04/pSTV28, ES04/pSTV28::Pthr::pckA, ES05/pSTV28, and ES05/pSTV28::Pthr::pckA, respectively.

The aforementioned strains were each inoculated into 4 ml of a seed medium containing 40 mg/L of chloramphenicol, and cultured at 31.5°C for 16 hours in a test tube with shaking. Then, 4 ml of a glucose containing production medium was added to the culture, the test tube was closed with a silicone stopper, and culture was performed at 31.5°C for 24 hours with shaking. In this evaluation system, inside of the system was made to be under an anaerobic condition by consumption of glucose. Therefore, the yield of succinic acid based on consumed saccharide is represented by a value obtained with consumed saccharide amount including saccharide amount consumed for establishing the anaerobic condition. The compositions of the seed medium and the glucose-containing production medium are shown below.

### Composition of organic acid production medium for Enterobacter bacteria, test tube evaluation system

**[Seed medium]**

| | |
|---|---|
| Bacto tryptone | 20 g/L |
| | (final concentration) |
| Yeast Extract | 10 g/L |
| Sodium chloride | 10 g/L |

These were autoclaved at 120°C for 10 minutes.

### [Glucose-containing production medium]

**(Plot A)**

| | |
|---|---|
| Glucose | 100 g/L |
| | (final concentration) |

**(Plot B)**

| | |
|---|---|
| Calcium carbonate (Japanese Pharmacopoeia) | |
| | 100 g/L |

The ingredient of the plot A autoclaved at 120°C for 10 minutes and the ingredient of the plot B sterilized at 180°C for 3 hours with dry heat and then left to cool were mixed, and used.

After the culture, amount of the organic acid accumulated in the medium was analyzed by liquid chromatography. Two of Shim-pack SCR-102H (Shimadzu) connected in series were used as the column, and a sample was eluted at 50°C with 5 mM p-toluenesulfonic acid. The eluate was neutralized with 20 mM Bis-Tris aqueous solution containing 5 mM p-toluenesulfonic acid and 100 µM EDTA, and-the organic acid was quantified by measuring electric conductivity with CDD-10AD (Shimadzu). The amount of consumed saccharide, OD, accumulated organic acid and yield based on the saccharide determined after 24 hours are shown in Table 3.

**Table 3**

| Strain | OD (600 nm) | Amount of consumed saccharide (g/L) | Accumulated succinic acid (g/L) | Yield of succinic acid based on consumed saccharide (%) |
|---|---|---|---|---|
| ES02/pSTV28 | 7.03 (± 0.47) | 4.35 (± 0.25) | 0.03 (± 0.06) | 0.72 (± 1.44) |
| ES02/pSTV28::Pthr::pckA | 7.26 (± 0.54) | 6.50 (± 1.05) | 1.63 (± 0.38) | 25.04 (± 2.61) |
| ES03/pSTV28 | 7.30 (± 0.68) | 8.93 (± 0.30) | 0.36 (± 0.04) | 4.03 (± 0.44) |
| ES03/pSTV28::Pthr::pckA | 9.64 (± 0.72) | 20.54 (± 1.19) | 5.80 (± 0.11) | 28.22 (± 1.14) |
| ES04/pSTV28 | 7.65 (± 0.37) | 11.18 (± 1.15) | 0.46 (± 0.03) | 4.30 (± 0.42) |
| ES04/pSTV28::Pthr::pckA | 8.14 (± 0.40) | 18.76 (± 0.86) | 7.20 (± 0.29) | 38.44 (± 2.46) |
| ES05/pSTV28 | 8.18 (± 0.11) | 11.12 (± 0.71) | 2.02 (± 0.48) | 18.23 (± 4.65) |
| ES05/pSTV28::Pthr::pckA | 8.97 (± 0.27) | 15.47 (± 0.36) | 8.23 (± 0.33) | 53.17 (± 1.41) |

Accumulation of succinic acid and yield based on consumed saccharide were markedly increased by the *pckA* amplification in all the ES02, ES03, ES04 and ES05 strains.

### Reference Example 1: Acquisition of succinic acid-producing bacterium belonging to genus Enterobacter

The *Enterobacter aerogenes* AJ110637 strain was obtained from soil of seashore at Susuki Kaigan, Makinohara-shi, Shizuoka-ken on March, 2006 by cumulative liquid culture using glycerol as the carbon source. The full-length 16S rDNA sequence was then determined, and a homology of 99.9% to that of the *Enterobacter aerogenes* NCTC 10006 strain was obtained. Moreover, also in a physiological test using an API kit, the strain showed results similar to the prototype species of *Enterobacter aerogenes,* and therefore the obtained isolated strain was identified as *Enterobacter aerogenes.*

### Reference Example 2: Construction of helper plasmid RSF-Red-TER

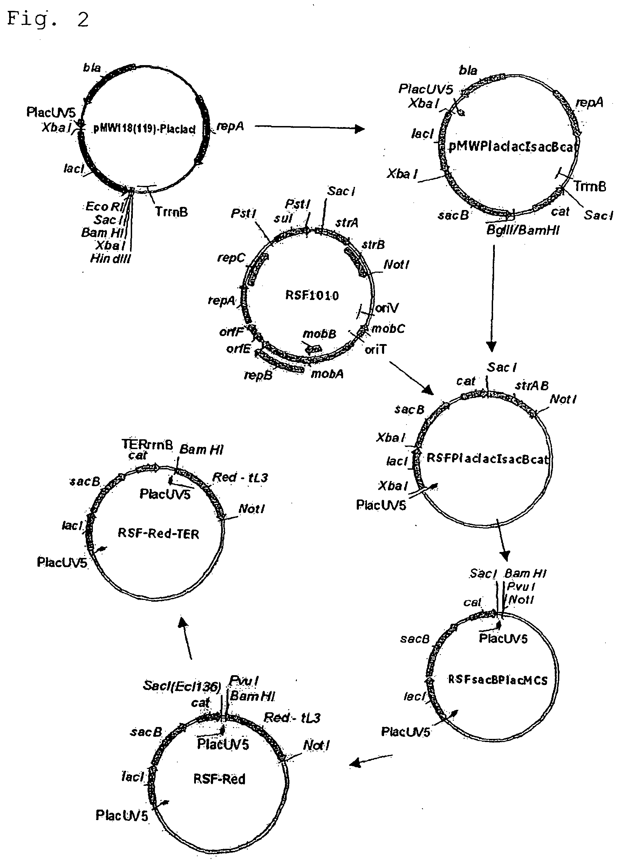

The scheme for constructing the helper plasmid RSF-Red-TER is shown in Fig. 2.

As the first step of the construction, an RSFsacBPlacMCS vector was designed. For this purpose, DNA fragments containing the *cat* gene of the pACYC184 plasmid and the structural region of the *sacB* gene of *Bacillus subtilis* were amplified by PCR using the oligonucleotides of SEQ ID NOS: 25 and 26, and 27 and 28, respectively. These oligonucleotides contained *Bgl*II, *Sac*I, *Xba*I and *Bam*HI restriction enzyme sites, which are required and convenient for further cloning, in the 5' end regions, respectively. The obtained *sacB* fragment of 1.5 kb was cloned into the previously obtained pMW119-P_{lac}lacI vector at the *Xba*I-*Bam*HI site. This vector was constructed in the same manner as that described for the pMW118-P_{lac}lacI vector (Skorokhodova, A.Y. et al, 2004, Biotekhnologiya (Rus), 5:3-21). However, this vector contained a polylinker moiety derived from pMW219 instead of the pMW218 plasmid.

Then, the aforementioned cat fragment of 1.0 kb was treated with *Bgl*II and *Sac*I, and cloned into the RSF-P_{lac}lacIsacB plasmid obtained in the previous step at the *Bam*HI-*Sac*I site. The obtained plasmid pMW-P_{lac}lacIsacBcat contained the PlacUV5-lacI-sacB-cat fragment. In order to subclone this fragment into the RSF1010 vector, pMW-P_{lac}lacIsacBcat was digested with *Bgl*II, blunt-ended with DNA polymerase I Klenow fragment, and successively digested with *Sac*I. A 3.8 kb *Bgl*II-*Sac*I fragment of the pMWP_{lac}lacIsacBcat plasmid was eluted from a 1% agarose gel, and ligated with the RSF1010 vector which had been treated with PstI and *Sac*I. *Escherichia coli* TG1 was transformed with the ligation mixture, and plated on the LB medium containing chloramphenicol (50 mg/L). The plasmids isolated from the grown clones were analyzed with restriction enzymes to obtain an RSFsacB plasmid. In order to construct an RSFsacBP_{lac}MCS vector, a DNA fragment containing the P_{lacUV5} promoter was amplified by PCR using the oligonucleotides of SEQ ID NOS: 29 and 30 as primers and the pMW119-P_{lac}lacI plasmid as the template. The obtained fragment of 146 bp was digested with *Sac*I and *Not*I*,* and ligated with the *Sac*I-*Not*I large fragment of the RSFsacB plasmid. Then, by PCR using the oligonucleotides of SEQ ID NOS: 31 and 32 as primers, and the pKD46 plasmid (Datsenko, K.A., Wanner, B.L., 2000, Proc. Nat1. Acad. Sci. USA, 97, 6640-6645) as the template, a DNA fragment of 2.3 kb containing the λRedαβγ genes and the transcription terminator tL3 was amplified. The obtained fragment was cloned into the RSFsacBP_{lac}MCS vector at the *Pvu*I*-Not*I site. In this way, the RSFRed plasmid was designed.

In order to eliminate read through transcription of the Red genes, a p-dependent transcription terminator of the *rrnB* operon of *Escherichia coli* was inserted at a position between the cat gene and the P_{lacUV5} promoter. For this purpose, a DNA fragment containing the P_{lacUV5} promoter and the TrrnB terminator was amplified by PCR using the oligonucleotides of SEQ ID NOS: 33 and 34 as primers and the chromosome of *Escherichia coli* BW3350 as the template. These obtained fragments were treated with *Kpn*I and ligated. Then, the 0.5 kb fragment containing both P_{lacUV5} and TrrnB was amplified by PCR using the oligonucleotides of SEQ ID NOS: 35 and 36 as primers. The obtained DNA fragment was digested with *EcoR*I*,* blunt-ended by a treatment with DNA polymerase I Klenow fragment, digested with *Bam*HI*,* and ligated with the *Ecl*136II-*Bam*HI large fragment of the RSFsacBPlacMCS vector. The obtained plasmid was designated RSF-Red-TER.

### Reference Example 3: Construction of pMW118-(λattL-Km^{r}-AattR) plasmid

A pMW118-(λattL-Km^{r}-λattR) plasmid was constructed from the pMW118-attL-Tc-attR plasmid by substituting the kanamycin resistance gene of the pUC4K plasmid for the tetracycline resistance marker gene. For this purpose, the *Eco*RI-*Hind*III large fragment of the pMW118-attL-Tc-attR plasmid was ligated with two fragments of the pUC4K plasmid, *Hind*III*-Pst*I (676 bp) and *EcoR*I*-Hind*III (585 bp) fragments. pMW-118-attL-Tc-attR serving as the basic structure was obtained by ligating the following four fragments.

### Construction of pMW118-attL-Tc-attR plasmid

1) The *Bgl*II-*Eco*RI fragment (114 bp) including *attL* (SEQ ID NO: 37) which was obtained by PCR amplification of the region corresponding to *attL* of the *Escherichia coli* W3350 (containing λ prophage) chromosome using the primers P1 and P2 (SEQ ID NOS: 35 and 36) (these primers contained the subsidiary recognition sites for *Bgl*II and *Eco*RI).
2) The *Pst*I*-Hind*III fragment (182 bp) including *attR* (SEQ ID NO: 40) which was obtained by PCR amplification of the region corresponding to *attR* of the *Escherichia coli* W3350 (containing λ prophage) chromosome using the primers P3 and P4 (SEQ ID NOS: 38 and 39) (these primers contained the subsidiary recognition sites for *Pst*I and *Hind*III).
3) The *Bgl*II-HindIII large fragment (3916 bp) of pMW118-ter_rrnB. The plasmid pMW118-ter_rrnB was obtained by ligation of the following three DNA fragments:
   - The large DNA fragment (2359 bp) including the *Aat*II-EcoRI fragment of pMW118 that was obtained by digesting pMW118 with EcoRI, treated with DNA polymerase I Klenow fragment, and then digesting with *Aat*II;
   - The small *Aat*II-*Bgl*II fragment (1194 bp) of pUC19 including the *bla* gene for ampicillin resistance (Ap^{R}), which was obtained by PCR amplification of the corresponding region of the pUC19 plasmid using the primers P5 and P6 (SEQ ID NOS: 41 and 42) (these primers contained the subsidiary recognition sites for PstI, *Aat*II and *Bgl*II) ;
   - The small *Bgl*II-*Pst*I fragment (363 bp) of the transcription terminator ter_rrnB, which was obtained by PCR amplification of the corresponding region of the *Escherichia coli* MG1655 chromosome using the primers P7 and P8 (SEQ ID NOS: 43 and 44) (these primers contained the subsidiary recognition sites for *Pst*I*, Bgl*II and *Pst*I).
4) The small *Eco*RI-*Pst*I fragment (1388 bp) (SEQ ID NO: 45) of pML-Tc-ter_thrL including the tetracycline resistance gene and the ter_thrL transcription terminator; the pML-Tc-ter_thrL plasmid was obtained by the following two steps:
   - the pML-ter_thrL plasmid was obtained by digesting the pML-MCS plasmid (Mashko, S.V. et al., 2001, Biotekhnologiya (in Russian), no. 5, 3-20) with *Xba*I and *Bam*HI, followed by ligation of the large fragment (3342 bp) with the *Xba*I-*Bam*HI fragment (68 bp) carrying ter_thrL terminator obtained by PCR amplification of the corresponding region of the *Escherichia coli* MG1655 chromosome using the primers P9 and P10 (SEQ ID NOS: 46 and 47) (these primers contained the subsidiary recognition sites for PstI, *Xba*I and *Bam*HI);
   - the pML-Tc-ter_thrL plasmid was obtained by digesting the pML-ter_thrL plasmid with *Kpn*I and *Xba*I followed by treatment with Klenow fragment of DNA polymerase I and ligation with the small EcoRI-Van91I fragment (1317 bp) of pBR322 including the tetracycline resistance gene (pBR322 was digested with *EcoR*I and *Van*91I and then treated with DNA polymerase I Klenow fragment).

### Reference Example 4: Construction of plasmid RSF-int-xis for eliminating antibiotic resistance gene

RSF-int-xis was constructed as a plasmid for eliminating the antibiotic resistance gene derived from the plasmid used for disruption of the gene from the gene-disrupted strain. As a material for the construction of RSF-int-xis, pMW-intxis-ts was used. pMW-intxis-ts is a plasmid carrying the gene coding for the integrase (Int) of λ phage, and the gene coding for excisionase (Xis), and having temperature sensitive replication ability (WO2007/037460, Japanese Patent Laid-open No. 2005-058827).

A DNA fragment including the intxis region was amplified by PCR using a primer intxis_f (SEQ ID NO: 66), a primer intxis_R (SEQ ID NO: 67), and pMW-intxis-ts as the template. The obtained DNA fragment was digested with *Not*I and *Pvu*I, and ligated with the large fragment of the RSF-Red-TER plasmid digested with *Not*I and *Pvu*I. The obtained plasmid was designated RSF-int-xis.

### Explanation of Sequence Listing

SEQ ID NO: 1: Primer for amplification of threonine promoter
SEQ ID NO: 2: Primer for amplification of threonine promoter
SEQ ID NO: 3: Threonine promoter gene fragment
SEQ ID NO: 4: Primer for amplification of *pckA* gene of *Actinobacillus succinogenes*
SEQ ID NO: 5: Primer for amplification of *pckA* gene of *Actinobacillus succinogenes*
SEQ ID NO: 6: Gene sequence of *pckA* of *Actinobacillus succinogenes* ATCC 55618 strain
SEQ ID NO: 7: Amino acid sequence of *pckA* of *Actinobacillus succinogenes* ATCC 55618 strain
SEQ ID NO: 8: Gene sequence of *pckA* of *Haemophilus influenzae* 86-028NP strain
SEQ ID NO: 9: Amino acid sequence of *pckA* of *Haemophilus influenzae* 86-028NP strain
SEQ ID NO: 10: Gene sequence of *pckA* of *Pasteurella multocida* subsp. *multocida* str. PM70 strain
SEQ ID NO: 11: Amino acid sequence of *pckA* of *Pasteurella multocida* subsp. *multocida* str. PM70 strain
SEQ ID NO: 12: Gene sequence of *pckA* of *Mannheimia succiniciproducens* MBEL55E strain
SEQ ID NO: 13: Amino acid sequence of *pckA* of *Mannheimia succiniciproducens* MBEL55E strain
SEQ ID NO: 14: Gene sequence of *pckA* of *Yersinia pseudotuberculosis* IP 32953 strain
SEQ ID NO: 15: Amino acid sequence of *pckA* of *Yersinia pseudotuberculosis* IP 32953 strain
SEQ ID NO: 16: Gene sequence of *pckA* of *Vibrio cholerae* 623-39
SEQ ID NO: 17: Amino acid sequence of *pckA* of *Vibrio cholerae* 623-39
SEQ ID NO: 18: Primer for deletion of *adhE*
SEQ ID NO: 19: Primer for deletion of *adhE*
SEQ ID NO: 20: Gene sequence of *ldhA* of *Enterobacter aerogenes* AJ110637
SEQ ID NO: 21: Gene sequence of *adhE* of *Enterobacter aerogenes* AJ110637
SEQ ID NO: 22: Nucleotide sequence of PC gene coding for pyruvate carboxylase of *Brevibacterium fluvum*
SEQ ID NO: 23: Amino acid sequence of pyruvate carboxylase of *Brevibacterium fluvum*
SEQ ID NO: 24: Consensus sequence of PEPCK
SEQ ID NO: 25: Primer for amplification of *cat* gene
SEQ ID NO: 26: Primer for amplification of *cat* gene
SEQ ID NO: 27: Primer for amplification of *sacB* gene
SEQ ID NO: 28: Primer for amplification of *sacB* gene
SEQ ID NO: 29: Primer for amplification of DNA fragment containing P_{lacUV5} promoter
SEQ ID NO: 30: Primer for amplification of DNA fragment containing P_{lacUV5} promoter
SEQ ID NO: 31: Primer for amplification of DNA fragment containing λRedαβγ genes and tL3
SEQ ID NO: 32: Primer for amplification of DNA fragment containing λRedαβγ genes and tL3
SEQ ID NO: 33: Primer for amplification of DNA fragment containing P_{lacUV5} promoter and TrrnB
SEQ ID NO: 34: Primer for amplification of DNA fragment containing P_{lacUV5} promoter and TrrnB
SEQ ID NO: 35: Primer for amplification of *attL*
SEQ ID NO: 36: Primer for amplification of *attL*
SEQ ID NO: 37: Nucleotide sequence of *attL*
SEQ ID NO: 38: Primer for amplification of *attR*
SEQ ID NO: 39: Primer for amplification of *attR*
SEQ ID NO: 40: Nucleotide sequence of *attR*
SEQ ID NO: 41: Primer for amplification of DNA fragment containing *bla* gene
SEQ ID NO: 42: Primer for amplification of DNA fragment containing *bla* gene
SEQ ID NO: 43: Primer for amplification of DNA fragment containing *ter_rrnB*
SEQ ID NO: 44: Primer for amplification of DNA fragment containing *ter*_*rrnB*
SEQ ID NO: 45: Nucleotide sequence of DNA fragment containing *ter_thrL* terminator
SEQ ID NO: 46: Primer for amplification of DNA fragment containing *ter_thrL* terminator
SEQ ID NO: 47: Primer for amplification of DNA fragment containing *ter_thrL* terminator
SEQ ID NO: 48: Amino acid sequence of *ldhA* of *Enterobacter aerogenes* AJ110637
SEQ ID NO: 49: Amino acid sequence of *adhE* of *Enterobacter aerogenes* AJ110637
SEQ ID NO: 50: Gene sequence of *pta* of *Enterobacter aerogenes* AJ110637
SEQ ID NO: 51: Amino acid sequence of *pta* of *Enterobacter aerogenes* AJ110637
SEQ ID NO: 52: Gene sequence of *α-aldC* of *Enterobacter aerogenes* AJ110637
SEQ ID NO: 53: Amino acid sequence of *α-aldC* of *Enterobacter aerogenes* AJ110637
SEQ ID NO: 54: Gene sequence of *pflB* of *Enterobacter aerogenes* AJ110637
SEQ ID NO: 55: Amino acid sequence of *pflB* of *Enterobacter aerogenes* AJ110637
SEQ ID NO: 56: Primer for deletion of *ldhA*
SEQ ID NO: 57: Primer for deletion of *ldhA*
SEQ ID NO: 58: Primer for deletion of *pta*
SEQ ID NO: 59: Primer for deletion of *pta*
SEQ ID NO : 60: Primer for deletion of *α-aldc*
SEQ ID NO: 61: Primer for deletion of *α-aldc*
SEQ ID NO: 62: Primer for deletion of *pflB*
SEQ ID NO: 63: Primer for deletion of *pflB*
SEQ ID NO: 64: Gene sequence of *pckA* of *Selenomonas ruminantium* subsp. *lactilytica* TH1
SEQ ID NO: 65: Amino acid sequence of *pckA* of *Selenomonas ruminantium* subsp. *lactilytica* TH1
SEQ ID NO: 66: Nucleotide sequence of primer intxis_f for construction of RSF-int-xis
SEQ ID NO: 67: Nucleotide sequence of primer intxis_R for construction of RSF-int-xis

### Industrial Applicability

According to the method of the present invention, an organic acid can be quickly and highly efficiently produced. When the organic acid is succinic acid, the obtained succinic acid can be used for food additives, pharmaceuticals, cosmetics, and the like. Moreover, succinic acid-containing polymers can also be produced by performing a polymerization reaction using the obtained succinic acid as a raw material.

## Claims

1. A method for producing succinic acid or malic acid comprising:
allowing a bacterium belonging to the genus *Enterobacter* which has an ability to produce succinic acid or malic acid and has been modified so that the phosphoenolpyruvate carboxykinase activity is enhanced as compared to a parent strain or a wild-type strain or immobilized cells of said bacterium obtained by immobilizing the cells of said bacterium with acrylamide or carragheenan to act on an organic raw material in a reaction mixture containing carbonate ions, bicarbonate ions, or carbon dioxide gas to produce succinic acid or malic acid, and
collecting succinic acid or malic acid,
wherein said bacterium has been modified so that expression of the *pckA* gene coding for phosphoenolpyruvate carboxykinase is enhanced by increasing the copy number of the gene and/or by modifying an expression control sequence of the gene, and
wherein the *pckA* gene is a DNA defined in (a) or (b):
(a) a DNA comprising a nucleotide sequence of SEQ ID NO: 6, 8, 10, 12, 14, 16 or 64,
(b) a DNA which hybridizes with a nucleotide sequence complementary to a nucleotide sequence of SEQ ID NO: 6, 8, 10, 12, 14, 16 or 64 under stringent conditions, and codes for a protein having the phosphoenolpyruvate carboxykinase activity.

2. The method according to claim 1, wherein the *pckA* gene codes for a protein having an amino acid sequence of SEQ ID NO: 7, 9, 11, 13, 15, 17, 65 or 24, or a protein having an amino acid sequence of SEQ ID NO: 7, 9, 11, 13, 15, 17, 65 or 24 including substitutions, deletions, insertions or additions of one or several amino acid residues.

3. The method according to claim 1 or 2, wherein the bacterium has been further modified so that one or more of enzymatic activities selected from alcohol dehydrogenase activity, lactate dehydrogenase activity, phosphate acetyltransferase activity, α-acetolactate decarboxylase activity, and pyruvate formate lyase activity are decreased.

4. The method according to claim 3, wherein the bacterium has been modified so that the alcohol dehydrogenase activity is decreased.

5. The method according to claim 3, wherein the bacterium has been modified so that the alcohol dehydrogenase activity and the lactate dehydrogenase activity are decreased.

6. The method according to claim 3, wherein the bacterium has been modified so that the alcohol dehydrogenase activity, the lactate dehydrogenase activity, and the phosphate acetyltransferase activity are decreased.

7. The method according to claim 3, wherein the bacterium has been modified so that the alcohol dehydrogenase activity, the lactate dehydrogenase activity, the phosphate acetyltransferase activity, and the α-acetolactate decarboxylase activity are decreased.

8. The method according to claim 3, wherein the bacterium has been modified so that the alcohol dehydrogenase activity, the lactate dehydrogenase activity, the phosphate acetyltransferase activity, the α-acetolactate decarboxylase activity, and the pyruvate formate lyase activity are decreased.

9. The method according to any one of claims 1 to 8, wherein the bacterium has been further modified so that pyruvate carboxylase activity is enhanced.

10. The method according to any one of claims 1 to 9, wherein succinic acid or malic acid is succinic acid.

11. The method according to claim 10, wherein the bacterium has been further modified so that the glucose PTS is decreased.

12. A method for producing a succinic acid-containing polymer comprising the steps of:
producing succinic acid by the method according to claim 10 or 11, and
polymerizing the obtained succinic acid.

## Patentansprüche

1. Verfahren zum Herstellen von Bernsteinsäure oder Äpfelsäure, welches umfasst:
das Zulassen, dass ein Bakterium der Gattung Enterobacter, das die Fähigkeit zur Produktion von Bernsteinsäure oder Äpfelsäure aufweist und so modifiziert worden ist, dass die Phosphoenolpyrovatcarboxykinaseaktivität im Vergleich zu einem Elternstamm oder einem Wildtypstamm erhöht ist, oder durch Immobilisieren der Zellen des Bakteriums mit Acrylamid oder Carraghen erhaltene immobilisierte Zellen des Bakteriums auf ein organisches Ausgangsmaterial in einem Reaktionsgemisch, das Carbonationen, Dicarbonationen oder Kohlendioxidgas enthält, einwirkt, wobei Bernsteinsäure oder Äpfelsäure hergestellt wird, und
das Gewinnen von Bernsteinsäure oder Äpfelsäure,
wobei das Bakterium so modifiziert worden ist, dass die Expression des für Phosphoenolpyrovatcarboxykinase kodierenden pckA-Gens durch Erhöhen der Kopienzahl des Gens und/oder durch Modifizieren einer Expressionskontrollsequenz des Gens erhöht ist, und
wobei das pckA-Gen eine in (a) oder in (b) definierte DNA ist:
(a) eine DNA, die eine Nukleotidsequenz der SEQ ID NO: 6, 8, 10, 12, 14, 16 oder 64 umfasst,
(b) eine DNA, die unter stringenten Bedingungen mit einer zu der Nukleotidsequenz der SEQ ID NO: 6, 8, 10, 12, 14, 16 oder 64 komplementären Nukleotidsequenz hybridisiert und für ein Protein mit Phosphoenolpyruvatcarboxykinaseaktivität kodiert.

2. Verfahren nach Anspruch 1, wobei das pckA-Gen für ein Protein mit einer Aminosäuresequenz der SEQ ID NO: 7, 9, 11, 13, 15, 17, 65 oder 24 oder für ein Protein der Aminosäuresequenz der SEQ ID NO: 7, 9, 11, 13, 15, 17, 65 oder 24, einschließlich Substitutionen, Deletionen, Insertionen oder Additionen von einem oder mehreren Aminosäureresten, kodiert.

3. Verfahren nach Anspruch 1 oder 2, wobei das Bakterium weiter so modifiziert worden ist, dass eine oder mehrere unter Alkoholdehydrogenaseaktivität, Laktatdehydrogenaseaktivität, Phosphatacetyltransferaseaktivität, α-Acetolaktatdecarboxylaseaktivität und Pyruvatformiatlyaseaktivität ausgewählte enzymatische Aktivitäten verringert ist/sind.

4. Verfahren nach Anspruch 3, wobei das Bakterium so modifiziert worden ist, dass die Alkoholdehydrogenaseaktivität verringert ist.

5. Verfahren nach Anspruch 3, wobei das Bakterium so modifiziert worden ist, dass die Alkoholdehydrogenaseaktivität und die Laktatdehydrogenaseaktivität verringert sind.

6. Verfahren nach Anspruch 3, wobei das Bakterium so modifiziert worden ist, dass die Alkoholdeyhdrogenaseaktivität, die Laktatdehydrogenaseaktivität und die Phosphatacetyltransferaseaktivität verringert sind.

7. Verfahren nach Anspruch 3, wobei das Bakterium so modifiziert worden ist, dass die Alkoholdehydrogenaseaktivität, die Laktatdehydrogenaseaktivität, die Phosphatacetyltransferaseaktivität und die α-Acetolaktatdecarboxylaseaktivität verringert sind.

8. Verfahren nach Anspruch 3, wobei das Bakterium so modifiziert worden ist, dass die Alkoholdehydrogenaseaktivität, die Laktatdehydrogenaseaktivität, die Phosphatacetyltransferaseaktivität, die α-Acetolaktatdecarboxylaseaktivität und Pyruvatformiatlyaseaktivität verringert sind.

9. Verfahren nach einem der Ansprüche 1 bis 8, wobei das Bakterium weiter so modifiziert worden ist, dass die Pyruvatcarboxylaseaktivität erhöht ist.

10. Verfahren nach einem der Ansprüche 1 bis 9, wobei Bernsteinsäure oder Äpfelsäure Bernsteinsäure ist.

11. Verfahren nach Anspruch 10, wobei das Bakterium weiter so modifiziert worden ist, dass das Glukose-PTS verringert ist.

12. Verfahren zum Herstellen eines Bernsteinsäure enthaltenden Polymers, welches die folgenden Stufen umfasst:
das Herstellen von Bernsteinsäure durch das Verfahren nach Anspruch 10 oder 11 und
das Polymerisieren der erhaltenen Bernsteinsäure.

## Revendications

1. Procédé de production d'acide succinique ou d'acide malique consistant :
à laisser une bactérie appartenant au genre *Enterobacter* qui présente une aptitude à produire de l'acide succinique ou de l'acide malique et qui a été modifiée de sorte que l'activité phosphoénolpyruvate carboxykinase est promue en comparaison à une souche parente ou à une souche de type sauvage ou des cellules immobilisées de ladite bactérie obtenues par immobilisation des cellules de ladite bactérie avec de l'acrylamide ou du carraghénane agir sur une matière première organique dans un mélange réactionnel contenant des ions carbonate, des ions bicarbonate, ou du dioxyde de carbone gazeux pour produire de l'acide succinique ou de l'acide malique, et
à recueillir de l'acide succinique ou de l'acide malique,
dans lequel ladite bactérie a été modifiée de sorte que l'expression du gène *pckA* codant la phosphoénolypyruvate carboxykinase est promue par augmentation du nombre de copies du gène et/ou par modification d'une séquence de contrôle d'expression du gène, et
dans lequel le gène *pckA* est un ADN défini dans (a) ou (b) :
(a) un ADN comprenant une séquence nucléotidique de SEQ ID NO: 6, 8, 10, 12, 14, 16 ou 64,
(b) un ADN qui s'hybride avec une séquence nucléotidique complémentaire à une séquence nucléotidique de SEQ ID NO:6, 8, 10, 12, 14, 16 ou 64 dans des conditions strictes, et code une protéine présentant l'activité phosphoénolpyruvate dicarboxykinase.

2. Procédé selon la revendication 1, dans lequel le gène *pckA* code une protéine présentant une séquence d'acide aminé de SEQ ID NO:7, 9, 11, 13, 15, 17, 65 ou 24, ou une protéine présentant une séquence d'acide aminé de SEQ ID NO: 7, 9, 11, 13, 15, 17, 65 ou 24 comprenant des substitutions, délétions, insertions ou additions d'un ou plusieurs résidus d'acides aminés.

3. Procédé selon la revendication 1 ou 2, dans lequel la bactérie a de plus été modifiée de sorte qu'une ou plusieurs activités enzymatiques choisies parmi l'activité alcool déshydrogénase, l'activité lactate déshydrogénase, l'activité phosphate acétyltransférase, l'activité α-acétolactate décarboxylase, et l'activité pyruvate formate lyase sont abaissées.

4. Procédé selon la revendication 3, dans lequel la bactérie a été modifiée de sorte que l'activité alcool déshydrogénase est abaissée.

5. Procédé selon la revendication 3, dans lequel la bactérie a été modifiée de sorte que l'activité alcool déshydrogénase et l'activité lactate déshydrogénase sont abaissées

6. Procédé selon la revendication 3, dans lequel la bactérie a été modifiée de sorte que l'activité alcool déshydrogénase, l'activité lactate déshydrogénase, et l'activité phosphate acétyltransférase sont abaissées.

7. Procédé selon la revendication 3, dans lequel la bactérie a été modifiée de sorte que l'activité alcool déshydrogénase, l'activité lactate déshydrogénase, l'activité phosphate acétyltransférase, et l'activité α-acétolactate décarboxylase sont abaissées.

8. Procédé selon la revendication 3, dans lequel la bactérie a été modifiée de sorte que l'activité alcool déshydrogénase, l'activité lactate déshydrogénase, l'activité phosphate acétyltransférase, l'activité α-acétolactate décarboxylase, et l'activité pyruvate formate lyase sont abaissées.

9. Procédé selon l'une quelconque des revendications 1 à 8, dans lequel la bactérie a de plus été modifiée de sorte que l'activité pyruvate carboxylase est promue.

10. Procédé selon l'une quelconque des revendications 1 à 9, dans lequel l'acide succinique ou l'acide malique est l'acide succinique.

11. Procédé selon la revendication 10, dans lequel la bactérie a de plus été modifiée de sorte que le glucose PTS est abaissé.

12. Procédé de production d'un polymère contenant de l'acide succinique comprenant les étapes de :
production d'acide succinique par le procédé selon la revendication 10 ou 11, et
polymérisation de l'acide succinique obtenu.


### SEQUENCE LISTING

<110> Ajinomoto Co., Inc.
<120> A method for producing an organic acid
<130> EPA-64924
<150> JP2007-315764
   <151> 2007-12-06
<160> 67
<170> patentIn version 3.5
<210> 1
   <211> 24
   <212> DNA
   <213> Artificial sequence
<220>
   <223> primer for thrLABC
<400> 1
   tggtcgactg gttacaacaa cgcc 24
<210> 2
   <211> 35
   <212> DNA
   <213> Artificial sequence
<220>
   <223> primer for thrLABC
<400> 2
   acgtcattcc tccttgtcgc ctatattggt taaag 35
<210> 3
   <211> 290
   <212> DNA
   <213> Escherichia coli
<400> 3
   tggtcgactg gttacaacaa cgcctggggc ttttagagca acgagacacg gcaatgttgc    60
   accgtttgct gcatgatatt gaaaaaaata tcaccaaata aaaaacgcct tagtaagtat   120
   ttttcagctt ttcattctga ctgcaacggg caatatgtct ctgtgtggat taaaaaaaga   180
   gtgtctgata gcagcttctg aactggttac ctgccgtgag taaattaaaa ttttattgac   240
   ttaggtcact aaatacttta accaatatag gcgacaagga ggaatgacgt              290
<210> 4
   <211> 40
   <212> DNA
   <213> Artificial Sequence
<220>
   <223> primer for A. succinogenes pckA
<400> 4
   gacaaggagg aatgacgtat gactgactta aacaaactcg 40
<210> 5
   <211> 28
   <212> DNA
   <213> Artificial Sequence
<220>
   <223> primer for A. succinogenes pckA
<400> 5
   acgcgtcgac ctcagcctta tttttcag 28
<210> 6
   <211> 1713
   <212> DNA
   <213> Actinobacillus succinogenes
<220>
   <221> CDS
   <222> (19)..(1635)
<400> 6
   gacaaggagg aatgacgt atg act gac tta aac aaa ctc gtt aaa gaa ctt      51
                       Met Thr Asp Leu Asn Lys Leu Val Lys Glu Leu
                       1               5                   10
   aat gac tta ggg ctt acc gat gtt aag gaa att gtg tat aac ccg agt      99
   Asn Asp Leu Gly Leu Thr Asp Val Lys Glu Ile Val Tyr Asn Pro Ser
               15                  20                  25
   tat gaa caa ctt ttc gag gaa gaa acc aaa ccg ggt ttg gag ggt ttc     147
   Tyr Glu Gln Leu Phe Glu Glu Glu Thr Lys Pro Gly Leu Glu Gly Phe
           30                  35                  40
   gat aaa ggg acg tta acc acg ctt ggc gcg gtt gcc gtc gat acg ggg     195
   Asp Lys Gly Thr Leu Thr Thr Leu Gly Ala Val Ala Val Asp Thr Gly
       45                  50                  55
   att ttt acc ggt cgt tca ccg aaa gat aaa tat atc gtt tgc gat gaa     243
   Ile Phe Thr Gly Arg Ser Pro Lys Asp Lys Tyr Ile Val Cys Asp Glu
   60                  65                  70                  75
   act acg aaa gac acc gtt tgg tgg aac agc gaa gcg gcg aaa aac gat     291
   Thr Thr Lys Asp Thr Val Trp Trp Asn Ser Glu Ala Ala Lys Asn Asp
                   80                  85                  90
   aac aaa ccg atg acg caa gaa act tgg aaa agt ttg aga gaa tta gtg     339
   Asn Lys Pro Met Thr Gln Glu Thr Trp Lys Ser Leu Arg Glu Leu Val
               95                  100                 105
   gcg aaa caa ctt tcc ggt aaa cgt tta ttc gtg gta gaa ggt tac tgc     387
   Ala Lys Gln Leu Ser Gly Lys Arg Leu Phe Val Val Glu Gly Tyr Cys
           110                 115                 120
   ggc gcc agt gaa aaa cac cgt atc ggt gtg cgt atg gtt act gaa gtg     435
   Gly Ala Ser Glu Lys His Arg Ile Gly Val Arg Met Val Thr Glu Val
       125                 130                 135
   gca tgg cag gcg cat ttt gtg aaa aac atg ttt atc cga ccg acc gat     483
   Ala Trp Gln Ala His Phe Val Lys Asn Met Phe Ile Arg Pro Thr Asp
   140                 145                 150                 155
   gaa gag ttg aaa aat ttc aaa gcg gat ttt acc gtg tta aac ggt gct     531
   Glu Glu Leu Lys Asn Phe Lys Ala Asp Phe Thr Val Leu Asn Gly Ala
                   160                 165                 170
   aaa tgt act aat ccg aac tgg aaa gaa caa ggt ttg aac agt gaa aac     579
   Lys Cys Thr Asn Pro Asn Trp Lys Glu Gln Gly Leu Asn Ser Glu Asn
               175                 180                 185
   ttt gtc gct ttc aat att acc gaa ggt att cag ctt atc ggc ggt act     627
   Phe Val Ala Phe Asn Ile Thr Glu Gly Ile Gln Leu Ile Gly Gly Thr
           190                 195                 200
   tgg tac ggc ggt gaa atg aaa aaa ggt atg ttc tca atg atg aac tac     675
   Trp Tyr Gly Gly Glu Met Lys Lys Gly Met Phe Ser Met Met Asn Tyr
               205                                210                                215
   ttc ctg ccg tta aaa ggt gtg gct tcc atg cac tgt tcc gcc aac gta      723
   Phe Leu Pro Leu Lys Gly Val Ala Ser Met His Cys Ser Ala Asn Val
   220                     225                     230                     235
   ggt aaa gac ggt gac gtg gct att ttc ttc ggt tta tcc ggt acg ggt      771
   Gly Lys Asp Gly Asp Val Ala Ile Phe Phe Gly Leu Ser Gly Thr Gly
                   240                     245                     250
   aaa aca acg ctt tcg acc gat cct aaa cgc caa tta atc ggt gat gac      819
   Lys Thr Thr Leu Ser Thr Asp Pro Lys Arg Gln Leu Ile Gly Asp Asp
               255                     260                     265
   gaa cac ggt tgg gat gaa tcc ggc gta ttt aac ttt gaa ggc ggt tgt      867
   Glu His Gly Trp Asp Glu Ser Gly Val Phe Asn Phe Glu Gly Gly Cys
           270                     275                     280
   tac gcg aaa acc att aac tta tct caa gaa aac gaa ccg gat att tac      915
   Tyr Ala Lys Thr Ile Asn Leu Ser Gln Glu Asn Glu Pro Asp Ile Tyr
       285                     290                     295
   ggc gca atc cgt cgt gac gca tta tta gaa aac gtc gtg gtt cgt gca      963
   Gly Ala Ile Arg Arg Asp Ala Leu Leu Glu Asn Val Val Val Arg Ala
   300                     305                     310                     315
   gac ggt tcc gtt gac ttt gac gac ggt tca aaa aca gaa aat acc cgt      1011
   Asp Gly Ser Val Asp Phe Asp Asp Gly Ser Lys Thr Glu Asn Thr Arg
                   320                     325                     330
   gtt tca tat ccg att tac cac atc gac aac atc gtt cgt ccg gta tcg      1059
   Val Ser Tyr Pro Ile Tyr His Ile Asp Asn Ile Val Arg Pro Val Ser
               335                     340                     345
   aaa gcc ggt cat gca acc aaa gtg att ttc tta acc gcg gac gca ttc      1107
   Lys Ala Gly His Ala Thr Lys Val Ile Phe Leu Thr Ala Asp Ala Phe
           350                     355                     360
   ggc gta ttg ccg ccg gtt tca aaa ctg act ccg gaa caa acc gaa tac      1155
   Gly Val Leu Pro Pro Val Ser Lys Leu Thr Pro Glu Gln Thr Glu Tyr
       365                     370                     375
   tac ttc tta tcc ggc ttt act gca aaa tta gcg ggt acg gaa cgc ggc      1203
   Tyr Phe Leu Ser Gly Phe Thr Ala Lys Leu Ala Gly Thr Glu Arg Gly
   380                     385                     390                     395
   gta acc gaa ccg act ccg aca ttc tcg gcc tgt ttc ggt gcg gca ttc      1251
   Val Thr Glu Pro Thr Pro Thr Phe Ser Ala Cys Phe Gly Ala Ala Phe
                   400                     405                     410
   tta agc ctg cat ccg att caa tat gcg gac gtg ttg gtc gaa cgc atg      1299
   Leu Ser Leu His Pro Ile Gln Tyr Ala Asp Val Leu Val Glu Arg Met
               415                     420                     425
   aaa gcc tcc ggt gcg gaa gct tat ttg gtg aac acc ggt tgg aac ggc      1347
   Lys Ala Ser Gly Ala Glu Ala Tyr Leu Val Asn Thr Gly Trp Asn Gly
           430                     435                     440
   acg ggt aaa cgt att tca atc aaa gat acc cgc ggt att atc gat gcg      1395
   Thr Gly Lys Arg Ile Ser Ile Lys Asp Thr Arg Gly Ile Ile Asp Ala
       445                     450                     455
   att ttg gac ggt tca atc gaa aaa gcg gaa atg ggc gaa ttg cca atc      1443
   Ile Leu Asp Gly Ser Ile Glu Lys Ala Glu Met Gly Glu Leu Pro Ile
   460                     465                     470                     475
   ttt aat tta gcg att cct aaa gca tta ccg ggt gtt gat cct gct att      1491
   Phe Asn Leu Ala Ile Pro Lys Ala Leu Pro Gly Val Asp Pro Ala Ile
                   480                     485                     490
   ttg gat ccg cgc gat act tac gca gac aaa gcg caa tgg caa gtt aaa      1539
   Leu Asp Pro Arg Asp Thr Tyr Ala Asp Lys Ala Gln Trp Gln Val Lys
               495                     500                     505
   gcg gaa gat ttg gca aac cgt ttc gtg aaa aac ttt gtg aaa tat acg      1587
   Ala Glu Asp Leu Ala Asn Arg Phe Val Lys Asn Phe Val Lys Tyr Thr
           510                     515                     520
   gcg aat ccg gaa gcg gct aaa tta gtt ggc gcc ggt cca aaa gca taa      1635
   Ala Asn Pro Glu Ala Ala Lys Leu Val Gly Ala Gly Pro Lys Ala
       525                     530                     535
   aactgtaaaa gcatagtatg tgcatgattc ggtaaactac cgaataaaat ctgaaaaata   1695
   aggctgaggt cgacgcgt                                                 1713
<210> 7
   <211> 538
   <212> PRT
   <213> Actinobacillus succinogenes
<400> 7
   Met Thr Asp Leu Asn Lys Leu Val Lys Glu Leu Asn Asp Leu Gly Leu
   1               5                   10                  15
   Thr Asp Val Lys Glu Ile Val Tyr Asn Pro Ser Tyr Glu Gln Leu Phe
               20                  25                  30
   Glu Glu Glu Thr Lys Pro Gly Leu Glu Gly Phe Asp Lys Gly Thr Leu
           35                  40                  45
   Thr Thr Leu Gly Ala Val Ala Val Asp Thr Gly Ile Phe Thr Gly Arg
       50                  55                  60
   Ser Pro Lys Asp Lys Tyr Ile Val Cys Asp Glu Thr Thr Lys Asp Thr
   65                  70                  75                  80
   Val Trp Trp Asn Ser Glu Ala Ala Lys Asn Asp Asn Lys Pro Met Thr
                   85                  90                  95
   Gln Glu Thr Trp Lys Ser Leu Arg Glu Leu Val Ala Lys Gln Leu Ser
               100                 105                 110
   Gly Lys Arg Leu Phe Val Val Glu Gly Tyr Cys Gly Ala Ser Glu Lys
           115                 120                 125
   His Arg Ile Gly Val Arg Met Val Thr Glu Val Ala Trp Gln Ala His
       130                 135                 140
   Phe Val Lys Asn Met Phe Ile Arg Pro Thr Asp Glu Glu Leu Lys Asn
   145                 150                 155                 160
   Phe Lys Ala Asp Phe Thr Val Leu Asn Gly Ala Lys Cys Thr Asn Pro
                   165                 170                 175
   Asn Trp Lys Glu Gln Gly Leu Asn Ser Glu Asn Phe Val Ala Phe Asn
               180                 185                 190
   Ile Thr Glu Gly Ile Gln Leu Ile Gly Gly Thr Trp Tyr Gly Gly Glu
           195                 200                 205
   Met Lys Lys Gly Met Phe Ser Met Met Asn Tyr Phe Leu Pro Leu Lys
       210                 215                 220
   Gly Val Ala Ser Met His Cys Ser Ala Asn Val Gly Lys Asp Gly Asp
   225                 230                 235                 240
   Val Ala Ile Phe Phe Gly Leu Ser Gly Thr Gly Lys Thr Thr Leu Ser
                   245                 250                 255
   Thr Asp Pro Lys Arg Gln Leu Ile Gly Asp Asp Glu His Gly Trp Asp
               260                 265                 270
   Glu Ser Gly Val Phe Asn Phe Glu Gly Gly Cys Tyr Ala Lys Thr Ile
           275                 280                 285
   Asn Leu Ser Gln Glu Asn Glu Pro Asp Ile Tyr Gly Ala Ile Arg Arg
       290                 295                 300
   Asp Ala Leu Leu Glu Asn Val Val Val Arg Ala Asp Gly Ser Val Asp
   305                 310                 315                 320
   Phe Asp Asp Gly Ser Lys Thr Glu Asn Thr Arg Val Ser Tyr Pro Ile
                   325                 330                 335
   Tyr His Ile Asp Asn Ile Val Arg Pro Val Ser Lys Ala Gly His Ala
               340                 345                 350
   Thr Lys Val Ile Phe Leu Thr Ala Asp Ala Phe Gly Val Leu Pro Pro
           355                 360                 365
   Val Ser Lys Leu Thr Pro Glu Gln Thr Glu Tyr Tyr Phe Leu Ser Gly
       370                 375                 380
   Phe Thr Ala Lys Leu Ala Gly Thr Glu Arg Gly Val Thr Glu Pro Thr
   385                 390                 395                 400
   Pro Thr Phe Ser Ala Cys Phe Gly Ala Ala Phe Leu Ser Leu His Pro
                   405                 410                 415
   Ile Gln Tyr Ala Asp Val Leu Val Glu Arg Met Lys Ala Ser Gly Ala
               420                 425                 430
   Glu Ala Tyr Leu Val Asn Thr Gly Trp Asn Gly Thr Gly Lys Arg Ile
           435                 440                 445
   Ser Ile Lys Asp Thr Arg Gly Ile Ile Asp Ala Ile Leu Asp Gly Ser
   450 455 460
   Ile Glu Lys Ala Glu Met Gly Glu Leu Pro Ile Phe Asn Leu Ala Ile
   465 470 475 480
   Pro Lys Ala Leu Pro Gly Val Asp Pro Ala Ile Leu Asp Pro Arg Asp
   485 490 495
   Thr Tyr Ala Asp Lys Ala Gln Trp Gln Val Lys Ala Glu Asp Leu Ala
   500 505 510
   Asn Arg Phe Val Lys Asn Phe Val Lys Tyr Thr Ala Asn Pro Glu Ala
   515 520 525
   Ala Lys Leu Val Gly Ala Gly Pro Lys Ala
   530 535
<210> 8
   <211> 1617
   <212> DNA
   <213> Haemophilus influenzae
<220>
   <221> CDS
   <222> (1)..(1617)
<400> 8
   atg aca gac tta aat aaa gtg gta aaa gaa ctt gaa gct ctt ggc att 48
   Met Thr Asp Leu Asn Lys Val Val Lys Glu Leu Glu Ala Leu Gly Ile
   1 5 10 15
   tat gac gta aaa gaa gtt gtt tac aat cca agc tac gag caa ttg ttc 96
   Tyr Asp Val Lys Glu Val Val Tyr Asn Pro Ser Tyr Glu Gln Leu Phe
   20 25 30
   gaa gaa gaa act aaa cca ggc tta gaa ggc ttt gaa aaa ggt act tta 144
   Glu Glu Glu Thr Lys Pro Gly Leu Glu Gly Phe Glu Lys Gly Thr Leu
   35 40 45
   act acg act ggt gca gtg gca gta gat aca ggt atc ttc aca ggt cgt 192
   Thr Thr Thr Gly Ala Val Ala Val Asp Thr Gly Ile Phe Thr Gly Arg
   50 55 60
   tct cca aaa gat aaa tat atc gtg tta gat gaa aaa acc aaa gat act 240
   Ser Pro Lys Asp Lys Tyr Ile Val Leu Asp Glu Lys Thr Lys Asp Thr
   65 70 75 80
   gtt tgg tgg aca tct gaa aca gca aaa aac gac aac aag cca atg aac 288
   Val Trp Trp Thr Ser Glu Thr Ala Lys Asn Asp Asn Lys Pro Met Asn
   85 90 95
   caa gct aca tgg caa agc tta aaa gac ttg gta acc aac caa ctt tct 336
   Gln Ala Thr Trp Gln Ser Leu Lys Asp Leu Val Thr Asn Gln Leu Ser
   100 105 110
   cgt aaa cgc tta ttt gta gtt gat ggt ttc tgt ggt gcg agc gaa cac 384
   Arg Lys Arg Leu Phe Val Val Asp Gly Phe Cys Gly Ala Ser Glu His
   115 120 125
   gac cgt att gca gta cgt att gtc act gaa gta gcg tgg caa gca cat 432
   Asp Arg Ile Ala Val Arg Ile Val Thr Glu Val Ala Trp Gln Ala His
   130 135 140
   ttt gta aaa aat atg ttt att cgc cca act gaa gaa caa ctc aaa aat 480
   Phe Val Lys Asn Met Phe Ile Arg Pro Thr Glu Glu Gln Leu Lys Asn
   145 150 155 160
   ttt gaa cca gat ttc gtt gta atg aac ggt tct aaa gta acc aat cca 528
   Phe Glu Pro Asp Phe Val Val Met Asn Gly Ser Lys Val Thr Asn Pro
   165 170 175
   aac tgg aaa gaa caa ggt tta aat tca gaa aac ttt gtt gct ttc aac 576
   Asn Trp Lys Glu Gln Gly Leu Asn Ser Glu Asn Phe Val Ala Phe Asn
   180 185 190
   ttg act gaa cgc att caa tta atc ggc ggt act tgg tac ggc ggc gaa 624
   Leu Thr Glu Arg Ile Gln Leu Ile Gly Gly Thr Trp Tyr Gly Gly Glu
   195 200 205
   atg aaa aaa ggt atg ttc tca atg atg aac tac ttc cta cct ctc aaa 672
   Met Lys Lys Gly Met Phe Ser Met Met Asn Tyr Phe Leu Pro Leu Lys
       210                     215                     220
   ggt gtt ggt gca atg cac tgc tca gct aac gtt ggt aaa gat ggt gat      720
   Gly Val Gly Ala Met His Cys Ser Ala Asn Val Gly Lys Asp Gly Asp
   225                     230                     235                 240
   gta gca atc ttc ttc ggc tta tct ggc aca ggt aaa aca acc ctt tca      768
   Val Ala Ile Phe Phe Gly Leu Ser Gly Thr Gly Lys Thr Thr Leu Ser
                   245                     250                     255
   acg gat cca aaa cgt gaa tta atc ggt gac gat gaa cac ggt tgg gat      816
   Thr Asp Pro Lys Arg Glu Leu Ile Gly Asp Asp Glu His Gly Trp Asp
               260                     265                     270
   gat gtt ggt atc ttt aac ttt gaa ggt ggt tgt tat gcg aaa acc att      864
   Asp Val Gly Ile Phe Asn Phe Glu Gly Gly Cys Tyr Ala Lys Thr Ile
           275                     280                     285
   cat ctt tca gaa gaa aat gaa cca gat att tac cac gct atc cgt cgc      912
   His Leu Ser Glu Glu Asn Glu Pro Asp Ile Tyr His Ala Ile Arg Arg
       290                     295                     300
   gac gca tta tta gaa aac gtg gtt gtt cgt tca gat ggt tct gtt gat      960
   Asp Ala Leu Leu Glu Asn Val Val Val Arg Ser Asp Gly Ser Val Asp
   305                     310                     315                 320
   ttc gat gat ggt tca aaa aca gaa aat act cgc gtg tct tac cca att     1008
   Phe Asp Asp Gly Ser Lys Thr Glu Asn Thr Arg Val Ser Tyr Pro Ile
                   325                     330                     335
   tat cac atc gat aac att gta aaa cca gtt tct cgt gca ggt cac gca     1056
   Tyr His Ile Asp Asn Ile Val Lys Pro Val Ser Arg Ala Gly His Ala
               340                     345                     350
   act aaa gtg att ttc tta act gca gat gcg ttt ggt gta tta cct cca     1104
   Thr Lys Val Ile Phe Leu Thr Ala Asp Ala Phe Gly Val Leu Pro Pro
           355                     360                     365
   gta tct aaa ttg aca cca gaa caa act aaa tac tac ttc tta tct ggt     1152
   Val Ser Lys Leu Thr Pro Glu Gln Thr Lys Tyr Tyr Phe Leu Ser Gly
       370                     375                     380
   ttc aca gcg aaa tta gca ggg act gaa cgt ggt att act gaa cca acg     1200
   Phe Thr Ala Lys Leu Ala Gly Thr Glu Arg Gly Ile Thr Glu Pro Thr
   385                     390                     395                 400
   cca act ttc tca gca tgt ttc ggt gca gca ttt tta acg ctt cat cca     1248
   Pro Thr Phe Ser Ala Cys Phe Gly Ala Ala Phe Leu Thr Leu His Pro
                   405                     410                     415
   act caa tat gca gaa gtg tta gta aaa cgt atg caa gca gca ggt gct     1296
   Thr Gln Tyr Ala Glu Val Leu Val Lys Arg Met Gln Ala Ala Gly Ala
               420                     425                     430
   gaa gct tac tta gtg aat act ggt tgg aat ggc aca ggc aaa cgt atc     1344
   Glu Ala Tyr Leu Val Asn Thr Gly Trp Asn Gly Thr Gly Lys Arg Ile
           435                     440                     445
   tca atc aaa gat act cgc gga atc att gat gca atc tta gat ggc tca     1392
   Ser Ile Lys Asp Thr Arg Gly Ile Ile Asp Ala Ile Leu Asp Gly Ser
       450                     455                     460
   att gaa aaa gct gaa atg ggc gaa ttg cca atc ttc aac tta gca att     1440
   Ile Glu Lys Ala Glu Met Gly Glu Leu Pro Ile Phe Asn Leu Ala Ile
   465                     470                     475                 480
   cct aaa gca tta cca ggt gta gat tct gca atc tta gat cct cgc gat     1488
   Pro Lys Ala Leu Pro Gly Val Asp Ser Ala Ile Leu Asp Pro Arg Asp
                   485                     490                     495
   act tac gca gat aaa gca caa tgg caa tca aaa gct gaa gac tta gca     1536
   Thr Tyr Ala Asp Lys Ala Gln Trp Gln Ser Lys Ala Glu Asp Leu Ala
               500                     505                     510
   ggt cgt ttt gtg aaa aac ttt gtt aaa tat gca act aac gaa gaa ggc     1584
   Gly Arg Phe Val Lys Asn Phe Val Lys Tyr Ala Thr Asn Glu Glu Gly
           515                     520                     525
   aaa gct tta att gca gct ggt cct aaa gct taa                         1617
   Lys Ala Leu Ile Ala Ala Gly Pro Lys Ala
       530                     535
<210> 9
   <211> 538
   <212> PRT
   <213> Haemophilus influenzae
<400> 9
   Met Thr Asp Leu Asn Lys Val Val Lys Glu Leu Glu Ala Leu Gly Ile
   1               5                   10                  15
   Tyr Asp Val Lys Glu Val Val Tyr Asn Pro Ser Tyr Glu Gln Leu Phe
               20                  25                  30
   Glu Glu Glu Thr Lys Pro Gly Leu Glu Gly Phe Glu Lys Gly Thr Leu
           35                  40                  45
   Thr Thr Thr Gly Ala Val Ala Val Asp Thr Gly Ile Phe Thr Gly Arg
       50                  55                  60
   Ser Pro Lys Asp Lys Tyr Ile Val Leu Asp Glu Lys Thr Lys Asp Thr
   65                  70                  75                  80
   Val Trp Trp Thr Ser Glu Thr Ala Lys Asn Asp Asn Lys Pro Met Asn
                   85                  90                  95
   Gln Ala Thr Trp Gln Ser Leu Lys Asp Leu Val Thr Asn Gln Leu Ser
               100                 105                 110
   Arg Lys Arg Leu Phe Val Val Asp Gly Phe Cys Gly Ala Ser Glu His
           115                 120                 125
   Asp Arg Ile Ala Val Arg Ile Val Thr Glu Val Ala Trp Gln Ala His
       130                 135                 140
   Phe Val Lys Asn Met Phe Ile Arg Pro Thr Glu Glu Gln Leu Lys Asn
   145                 150                 155                 160
   Phe Glu Pro Asp Phe Val Val Met Asn Gly Ser Lys Val Thr Asn Pro
                   165                 170                 175
   Asn Trp Lys Glu Gln Gly Leu Asn Ser Glu Asn Phe Val Ala Phe Asn
               180                 185                 190
   Leu Thr Glu Arg Ile Gln Leu Ile Gly Gly Thr Trp Tyr Gly Gly Glu
           195                 200                 205
   Met Lys Lys Gly Met Phe Ser Met Met Asn Tyr Phe Leu Pro Leu Lys
       210                 215                 220
   Gly Val Gly Ala Met His Cys Ser Ala Asn Val Gly Lys Asp Gly Asp
   225                 230                 235                 240
   Val Ala Ile Phe Phe Gly Leu Ser Gly Thr Gly Lys Thr Thr Leu Ser
                   245                 250                 255
   Thr Asp Pro Lys Arg Glu Leu Ile Gly Asp Asp Glu His Gly Trp Asp
               260                 265                 270
   Asp Val Gly Ile Phe Asn Phe Glu Gly Gly Cys Tyr Ala Lys Thr Ile
           275                 280                 285
   His Leu Ser Glu Glu Asn Glu Pro Asp Ile Tyr His Ala Ile Arg Arg
       290                 295                 300
   Asp Ala Leu Leu Glu Asn Val Val Val Arg Ser Asp Gly Ser Val Asp
   305                 310                 315                 320
   Phe Asp Asp Gly Ser Lys Thr Glu Asn Thr Arg Val Ser Tyr Pro Ile
                   325                 330                 335
   Tyr His Ile Asp Asn Ile Val Lys Pro Val Ser Arg Ala Gly His Ala
               340                 345                 350
   Thr Lys Val Ile Phe Leu Thr Ala Asp Ala Phe Gly Val Leu Pro Pro
           355                 360                 365
   Val Ser Lys Leu Thr Pro Glu Gln Thr Lys Tyr Tyr Phe Leu Ser Gly
       370                 375                 380
   Phe Thr Ala Lys Leu Ala Gly Thr Glu Arg Gly Ile Thr Glu Pro Thr
   385                 390                 395                 400
   Pro Thr Phe Ser Ala Cys Phe Gly Ala Ala Phe Leu Thr Leu His Pro
                   405                 410                 415
   Thr Gln Tyr Ala Glu Val Leu Val Lys Arg Met Gln Ala Ala Gly Ala
               420                 425                 430
   Glu Ala Tyr Leu Val Asn Thr Gly Trp Asn Gly Thr Gly Lys Arg Ile
           435                 440                 445
   Ser Ile Lys Asp Thr Arg Gly Ile Ile Asp Ala Ile Leu Asp Gly Ser
       450                 455                 460
   Ile Glu Lys Ala Glu Met Gly Glu Leu Pro Ile Phe Asn Leu Ala Ile
   465                 470                 475                 480
   Pro Lys Ala Leu Pro Gly Val Asp Ser Ala Ile Leu Asp Pro Arg Asp
                   485                 490                 495
   Thr Tyr Ala Asp Lys Ala Gln Trp Gln Ser Lys Ala Glu Asp Leu Ala
               500                 505                 510
   Gly Arg Phe Val Lys Asn Phe Val Lys Tyr Ala Thr Asn Glu Glu Gly
           515                 520                 525
   Lys Ala Leu Ile Ala Ala Gly Pro Lys Ala
       530                 535
<210> 10
   <211> 1617
   <212> DNA
   <213> Pasteurella multocida
<220>
   <221> CDS
   <222> (1)..(1617)
<400> 10
   atg act gac tta aat aaa gta atc aat gaa ctt ggt gca ctt ggt att      48
   Met Thr Asp Leu Asn Lys Val Ile Asn Glu Leu Gly Ala Leu Gly Ile
   1               5                   10                  15
   cat gat gta aaa gaa atc gtt tat aac cca agt tat gag caa ttg ttc      96
   His Asp Val Lys Glu Ile Val Tyr Asn Pro Ser Tyr Glu Gln Leu Phe
               20                  25                  30
   gaa gag gaa acc aaa cca ggt tta gaa ggt tac gaa aaa ggt atc gtg     144
   Glu Glu Glu Thr Lys Pro Gly Leu Glu Gly Tyr Glu Lys Gly Ile Val
           35                  40                  45
   aca caa tca ggc gcg gtg gcg gtt gat acc ggt atc ttt aca ggt cgt     192
   Thr Gln Ser Gly Ala Val Ala Val Asp Thr Gly Ile Phe Thr Gly Arg
       50                  55                  60
   tcg cca aaa gat aaa tac att gtg ctt gat gac aaa acc aaa gac act     240
   Ser Pro Lys Asp Lys Tyr Ile Val Leu Asp Asp Lys Thr Lys Asp Thr
   65                  70                  75                  80
   gtt tgg tgg aca agc gat gcg gcg aaa aac gat aac aaa cca atg aca     288
   Val Trp Trp Thr Ser Asp Ala Ala Lys Asn Asp Asn Lys Pro Met Thr
                   85                  90                  95
   caa gat acg tgg aaa agc tta aaa ggc tta gtg aca gaa caa ctt tct     336
   Gln Asp Thr Trp Lys Ser Leu Lys Gly Leu Val Thr Glu Gln Leu Ser
               100                 105                 110
   ggc aaa cgt tta ttt gtg atc gac gca ttc tgt ggt gca aac gct gac     384
   Gly Lys Arg Leu Phe Val Ile Asp Ala Phe Cys Gly Ala Asn Ala Asp
           115                 120                 125
   acc cgt tta tca gtg cgt atc gtg aca gaa gta gca tgg caa gca cac     432
   Thr Arg Leu Ser Val Arg Ile Val Thr Glu Val Ala Trp Gln Ala His
       130                 135                 140
   ttt gtg aaa aac atg ttc att cgt cca aca gaa gca gaa tta gtt ggc     480
   Phe Val Lys Asn Met Phe Ile Arg Pro Thr Glu Ala Glu Leu Val Gly
   145                 150                 155                 160
   ttc aaa cca gat ttc gta gta atg aac ggt tct aaa gta acc aac ccg     528
   Phe Lys Pro Asp Phe Val Val Met Asn Gly Ser Lys Val Thr Asn Pro
                   165                 170                 175
   aac tgg aaa gaa caa ggt cta aat tct gaa aac ttt gtg gca ttt aac     576
   Asn Trp Lys Glu Gln Gly Leu Asn Ser Glu Asn Phe Val Ala Phe Asn
               180                 185                 190
   tta act gaa ggc gtg caa tta atc ggt ggt act tgg tac ggc ggt gaa     624
   Leu Thr Glu Gly Val Gln Leu Ile Gly Gly Thr Trp Tyr Gly Gly Glu
           195                 200                 205
   atg aaa aaa ggt atg ttc tca atg atg aac tac ttc tta cca tta aaa     672
   Met Lys Lys Gly Met Phe Ser Met Met Asn Tyr Phe Leu Pro Leu Lys
       210                 215                 220
   ggc atc gca tct atg cac tgt tca gcc aac gtg ggt gaa aaa ggc gac     720
   Gly Ile Ala Ser Met His Cys Ser Ala Asn Val Gly Glu Lys Gly Asp
   225 230 235 240
   gtt gct gtg ttc ttc ggt tta tca ggt aca ggt aaa acc acc ctt tca 768
   Val Ala Val Phe Phe Gly Leu Ser Gly Thr Gly Lys Thr Thr Leu Ser
   245 250 255
   aca gat cca aaa cgt caa tta atc ggt gac gat gag cac ggt tgg gat 816
   Thr Asp Pro Lys Arg Gln Leu Ile Gly Asp Asp Glu His Gly Trp Asp
   260 265 270
   gat gat ggc gta ttc aac tac gaa ggt ggt tgc tat gcg aaa acg atc 864
   Asp Asp Gly Val Phe Asn Tyr Glu Gly Gly Cys Tyr Ala Lys Thr Ile
   275 280 285
   aaa ctg tct cca gaa aac gaa cca gat atc tat aaa gcc atc aaa cgt 912
   Lys Leu Ser Pro Glu Asn Glu Pro Asp Ile Tyr Lys Ala Ile Lys Arg
   290 295 300
   gat gcc tta tta gaa aac gtt gta gta cgt gca gat ggt tca gtg gat 960
   Asp Ala Leu Leu Glu Asn Val Val Val Arg Ala Asp Gly Ser Val Asp
   305 310 315 320
   tac gat gat ggt tca aaa aca gaa aac acc cgt gtt tct tac cca att 1008
   Tyr Asp Asp Gly Ser Lys Thr Glu Asn Thr Arg Val Ser Tyr Pro Ile
   325 330 335
   tac cac atc gac aac atc gta aca ccg gta tca aaa gca ggt cat gcg 1056
   Tyr His Ile Asp Asn Ile Val Thr Pro Val Ser Lys Ala Gly His Ala
   340 345 350
   aaa aaa gtg atc ttc tta act gcg gac gca ttc ggt gtg tta cca cca 1104
   Lys Lys Val Ile Phe Leu Thr Ala Asp Ala Phe Gly Val Leu Pro Pro
   355 360 365
   gta tct aaa tta acg cca gaa caa act aaa tac tac ttc tta tct ggt 1152
   Val Ser Lys Leu Thr Pro Glu Gln Thr Lys Tyr Tyr Phe Leu Ser Gly
   370 375 380
   ttc acc gcg aaa tta gcc ggt act gag cgt ggt atc aca gaa cca aca 1200
   Phe Thr Ala Lys Leu Ala Gly Thr Glu Arg Gly Ile Thr Glu Pro Thr
   385 390 395 400
   cca acg ttc tct gca tgt ttc ggt gca gcg ttc tta tca ctt cac cca 1248
   Pro Thr Phe Ser Ala Cys Phe Gly Ala Ala Phe Leu Ser Leu His Pro
   405 410 415
   aca caa tat gcg gaa gtg tta gtg aaa cgt atg gaa gca gcg ggt gcg 1296
   Thr Gln Tyr Ala Glu Val Leu Val Lys Arg Met Glu Ala Ala Gly Ala
   420 425 430
   gaa gct tac tta gtg aac aca ggt tgg aac ggt aca ggt aaa cgt atc 1344
   Glu Ala Tyr Leu Val Asn Thr Gly Trp Asn Gly Thr Gly Lys Arg Ile
   435 440 445
   tca atc aaa gat acg cgc ggt atc atc gat gca atc tta gac ggt tca 1392
   Ser Ile Lys Asp Thr Arg Gly Ile Ile Asp Ala Ile Leu Asp Gly Ser
   450 455 460
   atc gaa aaa gca gaa atg ggc aaa tta cca atc ttt gat tta gcg atc 1440
   Ile Glu Lys Ala Glu Met Gly Lys Leu Pro Ile Phe Asp Leu Ala Ile
   465 470 475 480
   cca act gca tta cca ggt gtt gac cct gca atc tta gat cca cgt gat 1488
   Pro Thr Ala Leu Pro Gly Val Asp Pro Ala Ile Leu Asp Pro Arg Asp
   485 490 495
   act tat gca gac aaa gca caa tgg caa gcg aaa gca gaa gac tta gct 1536
   Thr Tyr Ala Asp Lys Ala Gln Trp Gln Ala Lys Ala Glu Asp Leu Ala
   500 505 510
   ggt cgt ttc gtg aaa aac ttc gaa aaa tac acc act aac gat gaa ggt 1584
   Gly Arg Phe Val Lys Asn Phe Glu Lys Tyr Thr Thr Asn Asp Glu Gly
   515 520 525
   aaa gca tta gtg gca gca ggt cca aaa gcg taa 1617
   Lys Ala Leu Val Ala Ala Gly Pro Lys Ala
   530 535
<210> 11
   <211> 538
   <212> PRT
   <213> Pasteurella multocida
<400> 11
   Met Thr Asp Leu Asn Lys Val Ile Asn Glu Leu Gly Ala Leu Gly Ile
   1               5                   10                  15
   His Asp Val Lys Glu Ile Val Tyr Asn Pro Ser Tyr Glu Gln Leu Phe
               20                  25                  30
   Glu Glu Glu Thr Lys Pro Gly Leu Glu Gly Tyr Glu Lys Gly Ile Val
           35                  40                  45
   Thr Gln Ser Gly Ala Val Ala Val Asp Thr Gly Ile Phe Thr Gly Arg
       50                  55                  60
   Ser Pro Lys Asp Lys Tyr Ile Val Leu Asp Asp Lys Thr Lys Asp Thr
   65                  70                  75                  80
   Val Trp Trp Thr Ser Asp Ala Ala Lys Asn Asp Asn Lys Pro Met Thr
                   85                  90                  95
   Gln Asp Thr Trp Lys Ser Leu Lys Gly Leu Val Thr Glu Gln Leu Ser
               100                 105                 110
   Gly Lys Arg Leu Phe Val Ile Asp Ala Phe Cys Gly Ala Asn Ala Asp
           115                 120                 125
   Thr Arg Leu Ser Val Arg Ile Val Thr Glu Val Ala Trp Gln Ala His
       130                 135                 140
   Phe Val Lys Asn Met Phe Ile Arg Pro Thr Glu Ala Glu Leu Val Gly
   145                 150                 155                 160
   Phe Lys Pro Asp Phe Val Val Met Asn Gly Ser Lys Val Thr Asn Pro
                   165                 170                 175
   Asn Trp Lys Glu Gln Gly Leu Asn Ser Glu Asn Phe Val Ala Phe Asn
               180                 185                 190
   Leu Thr Glu Gly Val Gln Leu Ile Gly Gly Thr Trp Tyr Gly Gly Glu
           195                 200                 205
   Met Lys Lys Gly Met Phe Ser Met Met Asn Tyr Phe Leu Pro Leu Lys
       210                 215                 220
   Gly Ile Ala Ser Met His Cys Ser Ala Asn Val Gly Glu Lys Gly Asp
   225                 230                 235                 240
   Val Ala Val Phe Phe Gly Leu Ser Gly Thr Gly Lys Thr Thr Leu Ser
                   245                 250                 255
   Thr Asp Pro Lys Arg Gln Leu Ile Gly Asp Asp Glu His Gly Trp Asp
               260                 265                 270
   Asp Asp Gly Val Phe Asn Tyr Glu Gly Gly Cys Tyr Ala Lys Thr Ile
           275                 280                 285
   Lys Leu Ser Pro Glu Asn Glu Pro Asp Ile Tyr Lys Ala Ile Lys Arg
       290                 295                 300
   Asp Ala Leu Leu Glu Asn Val Val Val Arg Ala Asp Gly Ser Val Asp
   305                 310                 315                 320
   Tyr Asp Asp Gly Ser Lys Thr Glu Asn Thr Arg Val Ser Tyr Pro Ile
                   325                 330                 335
   Tyr His Ile Asp Asn Ile Val Thr Pro Val Ser Lys Ala Gly His Ala
               340                 345                 350
   Lys Lys Val Ile Phe Leu Thr Ala Asp Ala Phe Gly Val Leu Pro Pro
           355                 360                 365
   Val Ser Lys Leu Thr Pro Glu Gln Thr Lys Tyr Tyr Phe Leu Ser Gly
       370                 375                 380
   Phe Thr Ala Lys Leu Ala Gly Thr Glu Arg Gly Ile Thr Glu Pro Thr
   385                 390                 395                 400
   Pro Thr Phe Ser Ala Cys Phe Gly Ala Ala Phe Leu Ser Leu His Pro
                   405                 410                 415
   Thr Gln Tyr Ala Glu Val Leu Val Lys Arg Met Glu Ala Ala Gly Ala
               420                 425                 430
   Glu Ala Tyr Leu Val Asn Thr Gly Trp Asn Gly Thr Gly Lys Arg Ile
           435                 440                 445
   Ser Ile Lys Asp Thr Arg Gly Ile Ile Asp Ala Ile Leu Asp Gly Ser
       450                 455                 460
   Ile Glu Lys Ala Glu Met Gly Lys Leu Pro Ile Phe Asp Leu Ala Ile
   465                 470                 475                 480
   Pro Thr Ala Leu Pro Gly Val Asp Pro Ala Ile Leu Asp Pro Arg Asp
                           485                     490                     495
   Thr Tyr Ala Asp Lys Ala Gln Trp Gln Ala Lys Ala Glu Asp Leu Ala
                   500                     505                     510
   Gly Arg Phe Val Lys Asn Phe Glu Lys Tyr Thr Thr Asn Asp Glu Gly
               515                     520                     525
   Lys Ala Leu Val Ala Ala Gly Pro Lys Ala
       530                     535
<210> 12
   <211> 1617
   <212> DNA
   <213> Mannheimia succiniciproducens
<220>
   <221> CDS
   <222> (1)..(1617)
<400> 12
   atg aca gat ctt aat caa tta act caa gaa ctt ggt gct tta ggt att      48
   Met Thr Asp Leu Asn Gln Leu Thr Gln Glu Leu Gly Ala Leu Gly Ile
   1               5                   10                  15
   cat gat gta caa gaa gtt gtg tat aac ccg agc tat gaa ctt ctt ttt      96
   His Asp Val Gln Glu Val Val Tyr Asn Pro Ser Tyr Glu Leu Leu Phe
               20                  25                  30
   gcg gaa gaa acc aaa cca ggt tta gaa ggt tat gaa aaa ggt act gtg     144
   Ala Glu Glu Thr Lys Pro Gly Leu Glu Gly Tyr Glu Lys Gly Thr Val
           35                  40                  45
   act aat caa gga gcg gtt gct gta aat acc ggt att ttc acc ggt cgt     192
   Thr Asn Gln Gly Ala Val Ala Val Asn Thr Gly Ile Phe Thr Gly Arg
       50                  55                  60
   tct ccg aaa gat aaa tat atc gtt tta gac gac aaa act aaa gat acc     240
   Ser Pro Lys Asp Lys Tyr Ile Val Leu Asp Asp Lys Thr Lys Asp Thr
   65                  70                  75                  80
   gta tgg tgg acc agc gaa aaa gtt aaa aac gat aac aaa cca atg agc     288
   Val Trp Trp Thr Ser Glu Lys Val Lys Asn Asp Asn Lys Pro Met Ser
                   85                  90                  95
   caa gat acc tgg aac agt ttg aaa ggt tta gtt gcc gat caa ctt tcc     336
   Gln Asp Thr Trp Asn Ser Leu Lys Gly Leu Val Ala Asp Gln Leu Ser
               100                 105                 110
   ggt aaa cgt tta ttt gtt gtt gac gca ttc tgc ggc gcg aat aaa gat     384
   Gly Lys Arg Leu Phe Val Val Asp Ala Phe Cys Gly Ala Asn Lys Asp
           115                 120                 125
   acg cgt tta gct gtt cgt gtg gtt act gaa gtt gca tgg cag gcg cat     432
   Thr Arg Leu Ala Val Arg Val Val Thr Glu Val Ala Trp Gln Ala His
       130                 135                 140
   ttt gta aca aat atg ttt atc cgc cct tca gcg gaa gaa tta aaa ggt     480
   Phe Val Thr Asn Met Phe Ile Arg Pro Ser Ala Glu Glu Leu Lys Gly
   145                 150                 155                 160
   ttc aaa cct gat ttc gtg gta atg aac ggt gca aaa tgt aca aat cct     528
   Phe Lys Pro Asp Phe Val Val Met Asn Gly Ala Lys Cys Thr Asn Pro
                   165                 170                 175
   aac tgg aaa gaa caa ggg tta aat tcc gaa aac ttc gtt gcg ttc aac     576
   Asn Trp Lys Glu Gln Gly Leu Asn Ser Glu Asn Phe Val Ala Phe Asn
               180                 185                 190
   att aca gaa ggc gtt caa tta atc ggc ggt act tgg tac ggt ggt gaa     624
   Ile Thr Glu Gly Val Gln Leu Ile Gly Gly Thr Trp Tyr Gly Gly Glu
           195                 200                 205
   atg aaa aaa ggt atg ttc tca atg atg aac tac ttc tta ccg ctt cgt     672
   Met Lys Lys Gly Met Phe Ser Met Met Asn Tyr Phe Leu Pro Leu Arg
       210                 215                 220
   ggt att gca tca atg cac tgt tcc gca aac gtt ggt aaa gac ggc gat     720
   Gly Ile Ala Ser Met His Cys Ser Ala Asn Val Gly Lys Asp Gly Asp
   225                 230                 235                 240
   acc gca att ttc ttc ggt ttg tca ggc aca ggt aaa acg aca tta tca      768
   Thr Ala Ile Phe Phe Gly Leu Ser Gly Thr Gly Lys Thr Thr Leu Ser
                   245                 250                 255
   aca gat cct aaa cgt caa cta atc ggt gat gac gaa cac ggt tgg gac      816
   Thr Asp Pro Lys Arg Gln Leu Ile Gly Asp Asp Glu His Gly Trp Asp
               260                 265                 270
   gat gaa ggc gta ttt aac ttc gaa ggt ggt tgc tac gcg aaa acc att      864
   Asp Glu Gly Val Phe Asn Phe Glu Gly Gly Cys Tyr Ala Lys Thr Ile
           275                 280                 285
   aac tta tcc gct gaa aac gag ccg gat atc tat ggc gct atc aaa cgt      912
   Asn Leu Ser Ala Glu Asn Glu Pro Asp Ile Tyr Gly Ala Ile Lys Arg
       290                 295                 300
   gac gca tta ttg gaa aac gtg gtt gtt tta gat aac ggt gac gtt gac      960
   Asp Ala Leu Leu Glu Asn Val Val Val Leu Asp Asn Gly Asp Val Asp
   305                 310                 315                 320
   tat gca gac ggt tcc aaa aca gaa aat aca cgt gtt tct tat ccg att     1008
   Tyr Ala Asp Gly Ser Lys Thr Glu Asn Thr Arg Val Ser Tyr Pro Ile
                   325                 330                 335
   tat cac att caa aat atc gtt aaa cct gtt tct aaa gct ggt ccg gca     1056
   Tyr His Ile Gln Asn Ile Val Lys Pro Val Ser Lys Ala Gly Pro Ala
               340                 345                 350
   act aaa gtt atc ttc ttg tct gcc gat gca ttc ggt gta tta ccg ccg     1104
   Thr Lys Val Ile Phe Leu Ser Ala Asp Ala Phe Gly Val Leu Pro Pro
           355                 360                 365
   gtg tct aaa tta act ccg gaa caa acc aaa tac tat ttc tta tcc ggt     1152
   Val Ser Lys Leu Thr Pro Glu Gln Thr Lys Tyr Tyr Phe Leu Ser Gly
       370                 375                 380
   ttc act gcg aaa tta gcg ggt acg gaa cgc ggt att aca gag cct aca     1200
   Phe Thr Ala Lys Leu Ala Gly Thr Glu Arg Gly Ile Thr Glu Pro Thr
   385                 390                 395                 400
   cca aca ttc tct gca tgt ttt ggt gcg gct ttt tta agc ttg cat ccg     1248
   Pro Thr Phe Ser Ala Cys Phe Gly Ala Ala Phe Leu Ser Leu His Pro
                   405                 410                 415
   aca caa tat gcc gaa gtg tta gta aaa cgt atg caa gaa tca ggt gcg     1296
   Thr Gln Tyr Ala Glu Val Leu Val Lys Arg Met Gln Glu Ser Gly Ala
               420                 425                 430
   gaa gcg tat ctt gtt aat aca ggt tgg aac ggt acc ggc aaa cgt atc     1344
   Glu Ala Tyr Leu Val Asn Thr Gly Trp Asn Gly Thr Gly Lys Arg Ile
           435                 440                 445
   tca att aaa gat acc cgt ggt att att gat gca att tta gac ggc tca     1392
   Ser Ile Lys Asp Thr Arg Gly Ile Ile Asp Ala Ile Leu Asp Gly Ser
       450                 455                 460
   att gat aaa gcg gaa atg ggc tca tta cca atc ttc gat ttc tca att     1440
   Ile Asp Lys Ala Glu Met Gly Ser Leu Pro Ile Phe Asp Phe Ser Ile
   465                 470                 475                 480
   cct aaa gca tta cct ggt gtt aac cct gca atc tta gat ccg cgc gat     1488
   Pro Lys Ala Leu Pro Gly Val Asn Pro Ala Ile Leu Asp Pro Arg Asp
                   485                 490                 495
   act tat gcg gat aaa gcg caa tgg gaa gaa aaa gct caa gat ctt gca     1536
   Thr Tyr Ala Asp Lys Ala Gln Trp Glu Glu Lys Ala Gln Asp Leu Ala
               500                 505                 510
   ggt cgc ttt gtg aaa aac ttt gaa aaa tat acc ggt acg gcg gaa ggt     1584
   Gly Arg Phe Val Lys Asn Phe Glu Lys Tyr Thr Gly Thr Ala Glu Gly
           515                 520                 525
   cag gca tta gtt gct gcc ggt cct aaa gca taa                         1617
   Gln Ala Leu Val Ala Ala Gly Pro Lys Ala
       530                 535
<210> 13
   <211> 538
   <212> PRT
   <213> Mannheimia succiniciproducens
<400> 13
   Met Thr Asp Leu Asn Gln Leu Thr Gln Glu Leu Gly Ala Leu Gly Ile
   1               5                   10                  15
   His Asp Val Gln Glu Val Val Tyr Asn Pro Ser Tyr Glu Leu Leu Phe
               20                  25                  30
   Ala Glu Glu Thr Lys Pro Gly Leu Glu Gly Tyr Glu Lys Gly Thr Val
           35                  40                  45
   Thr Asn Gln Gly Ala Val Ala Val Asn Thr Gly Ile Phe Thr Gly Arg
       50                  55                  60
   Ser Pro Lys Asp Lys Tyr Ile Val Leu Asp Asp Lys Thr Lys Asp Thr
   65                  70                  75                  80
   Val Trp Trp Thr Ser Glu Lys Val Lys Asn Asp Asn Lys Pro Met Ser
                   85                  90                  95
   Gln Asp Thr Trp Asn Ser Leu Lys Gly Leu Val Ala Asp Gln Leu Ser
               100                 105                 110
   Gly Lys Arg Leu Phe Val Val Asp Ala Phe Cys Gly Ala Asn Lys Asp
           115                 120                 125
   Thr Arg Leu Ala Val Arg Val Val Thr Glu Val Ala Trp Gln Ala His
       130                 135                 140
   Phe Val Thr Asn Met Phe Ile Arg Pro Ser Ala Glu Glu Leu Lys Gly
   145                 150                 155                 160
   Phe Lys Pro Asp Phe Val Val Met Asn Gly Ala Lys Cys Thr Asn Pro
                   165                 170                 175
   Asn Trp Lys Glu Gln Gly Leu Asn Ser Glu Asn Phe Val Ala Phe Asn
               180                 185                 190
   Ile Thr Glu Gly Val Gln Leu Ile Gly Gly Thr Trp Tyr Gly Gly Glu
           195                 200                 205
   Met Lys Lys Gly Met Phe Ser Met Met Asn Tyr Phe Leu Pro Leu Arg
       210                 215                 220
   Gly Ile Ala Ser Met His Cys Ser Ala Asn Val Gly Lys Asp Gly Asp
   225                 230                 235                 240
   Thr Ala Ile Phe Phe Gly Leu Ser Gly Thr Gly Lys Thr Thr Leu Ser
                   245                 250                 255
   Thr Asp Pro Lys Arg Gln Leu Ile Gly Asp Asp Glu His Gly Trp Asp
               260                 265                 270
   Asp Glu Gly Val Phe Asn Phe Glu Gly Gly Cys Tyr Ala Lys Thr Ile
           275                 280                 285
   Asn Leu Ser Ala Glu Asn Glu Pro Asp Ile Tyr Gly Ala Ile Lys Arg
       290                 295                 300
   Asp Ala Leu Leu Glu Asn Val Val Val Leu Asp Asn Gly Asp Val Asp
   305                 310                 315                 320
   Tyr Ala Asp Gly Ser Lys Thr Glu Asn Thr Arg Val Ser Tyr Pro Ile
                   325                 330                 335
   Tyr His Ile Gln Asn Ile Val Lys Pro Val Ser Lys Ala Gly Pro Ala
               340                 345                 350
   Thr Lys Val Ile Phe Leu Ser Ala Asp Ala Phe Gly Val Leu Pro Pro
           355                 360                 365
   Val Ser Lys Leu Thr Pro Glu Gln Thr Lys Tyr Tyr Phe Leu Ser Gly
       370                 375                 380
   Phe Thr Ala Lys Leu Ala Gly Thr Glu Arg Gly Ile Thr Glu Pro Thr
   385                 390                 395                 400
   Pro Thr Phe Ser Ala Cys Phe Gly Ala Ala Phe Leu Ser Leu His Pro
                   405                 410                 415
   Thr Gln Tyr Ala Glu Val Leu Val Lys Arg Met Gln Glu Ser Gly Ala
               420                 425                 430
   Glu Ala Tyr Leu Val Asn Thr Gly Trp Asn Gly Thr Gly Lys Arg Ile
           435                 440                 445
   Ser Ile Lys Asp Thr Arg Gly Ile Ile Asp Ala Ile Leu Asp Gly Ser
       450                 455                 460
   Ile Asp Lys Ala Glu Met Gly Ser Leu Pro Ile Phe Asp Phe Ser Ile
   465                 470                 475                 480
   Pro Lys Ala Leu Pro Gly Val Asn Pro Ala Ile Leu Asp Pro Arg Asp
                   485                 490                 495
   Thr Tyr Ala Asp Lys Ala Gln Trp Glu Glu Lys Ala Gln Asp Leu Ala
                       500                 505                 510
   Gly Arg Phe Val Lys Asn Phe Glu Lys Tyr Thr Gly Thr Ala Glu Gly
               515                 520                 525
   Gln Ala Leu Val Ala Ala Gly Pro Lys Ala
           530                 535
<210> 14
   <211> 1620
   <212> DNA
   <213> Yersinia pseudotuberculosis
<220>
   <221> CDS
   <222> (1)..(1620)
<400> 14
   atg agt gtt aaa gga att acc ccg cag gag ctc gcc gcc tat ggc atc      48
   Met Ser Val Lys Gly Ile Thr Pro Gln Glu Leu Ala Ala Tyr Gly Ile
   1               5                   10                  15
   cat aac gtc agc gag att gtt tac aac cca agc tat gat tta ctg ttt      96
   His Asn Val Ser Glu Ile Val Tyr Asn Pro Ser Tyr Asp Leu Leu Phe
               20                  25                  30
   gaa gaa gag acc aaa ccc acg ttg gaa gga tac gaa cgt ggc aca ctg     144
   Glu Glu Glu Thr Lys Pro Thr Leu Glu Gly Tyr Glu Arg Gly Thr Leu
           35                  40                  45
   acg act acc ggc gca ata gcg gta gat acc ggt att ttt acc ggg cgt     192
   Thr Thr Thr Gly Ala Ile Ala Val Asp Thr Gly Ile Phe Thr Gly Arg
       50                  55                  60
   tca ccc aaa gat aaa tat att gtc cgc gat gct atc act cag gat acc     240
   Ser Pro Lys Asp Lys Tyr Ile Val Arg Asp Ala Ile Thr Gln Asp Thr
   65                  70                  75                  80
   gtg tgg tgg gcc gat cag ggc aaa ggt aaa aat gat aat aag cct ctg     288
   Val Trp Trp Ala Asp Gln Gly Lys Gly Lys Asn Asp Asn Lys Pro Leu
                   85                  90                  95
   agc caa gag atc tgg agc cat ttg aaa ggt ctg gtg acg gaa caa ctc     336
   Ser Gln Glu Ile Trp Ser His Leu Lys Gly Leu Val Thr Glu Gln Leu
               100                 105                 110
   tct ggc aaa cgc ctc ttt gtt gtc gat aca ttc tgc ggt gct aat gcg     384
   Ser Gly Lys Arg Leu Phe Val Val Asp Thr Phe Cys Gly Ala Asn Ala
           115                 120                 125
   gat acc cgc ctg caa gtc cgc ttt atc aca gaa gtc gct tgg cag gca     432
   Asp Thr Arg Leu Gln Val Arg Phe Ile Thr Glu Val Ala Trp Gln Ala
       130                 135                 140
   cac ttc gtc aaa aat atg ttt atc cgt cca tca gat gaa gaa ctg gct     480
   His Phe Val Lys Asn Met Phe Ile Arg Pro Ser Asp Glu Glu Leu Ala
   145                 150                 155                 160
   cgg ttt gaa cct gac ttt atc gtg atg aac ggt gcc aaa tgc act aac     528
   Arg Phe Glu Pro Asp Phe Ile Val Met Asn Gly Ala Lys Cys Thr Asn
                   165                 170                 175
   cca caa tgg aaa gag cag ggc ctg aat tca gaa aac ttt gtc gcc ttt     576
   Pro Gln Trp Lys Glu Gln Gly Leu Asn Ser Glu Asn Phe Val Ala Phe
               180                 185                 190
   aat ctg aca gaa cgt atg cag ttg att ggt ggc acg tgg tat ggc ggc     624
   Asn Leu Thr Glu Arg Met Gln Leu Ile Gly Gly Thr Trp Tyr Gly Gly
           195                 200                 205
   gaa atg aag aaa ggg atg ttc tca atg atg aac tac ctg ctg cca ctg     672
   Glu Met Lys Lys Gly Met Phe Ser Met Met Asn Tyr Leu Leu Pro Leu
       210                 215                 220
   aaa ggc att gct tca atg cat tgt tca gct aac gtc ggc gaa aaa ggc     720
   Lys Gly Ile Ala Ser Met His Cys Ser Ala Asn Val Gly Glu Lys Gly
   225                 230                 235                 240
   gat gtt gcc atc ttc ttc ggt ctg tcg ggt acc ggt aaa acc act tta     768
   Asp Val Ala Ile Phe Phe Gly Leu Ser Gly Thr Gly Lys Thr Thr Leu
                   245                 250                 255
   tct acc gat cca aaa cgc aag ttg atc ggt gat gat gaa cat ggc tgg      816
   Ser Thr Asp Pro Lys Arg Lys Leu Ile Gly Asp Asp Glu His Gly Trp
               260                 265                 270
   gat gat gat ggc gtc ttt aac ttc gag ggg ggg tgc tac gct aaa acc      864
   Asp Asp Asp Gly Val Phe Asn Phe Glu Gly Gly Cys Tyr Ala Lys Thr
           275                 280                 285
   atc aag tta tct gaa gaa gca gag cca gat att tac cac gcc att aaa      912
   Ile Lys Leu Ser Glu Glu Ala Glu Pro Asp Ile Tyr His Ala Ile Lys
       290                 295                 300
   cgc gac gcc ttg ctg gaa aac gtg gtg gtg cta gca gac ggt acc gtt      960
   Arg Asp Ala Leu Leu Glu Asn Val Val Val Leu Ala Asp Gly Thr Val
   305                 310                 315                 320
   gat ttt aat gac ggt tct aaa act gaa aac acc cgt gtc tct tat ccg     1008
   Asp Phe Asn Asp Gly Ser Lys Thr Glu Asn Thr Arg Val Ser Tyr Pro
                   325                 330                 335
   att tac cac att gat aac att gtt aaa ccg gtg tcc aaa gca ggc cat     1056
   Ile Tyr His Ile Asp Asn Ile Val Lys Pro Val Ser Lys Ala Gly His
               340                 345                 350
   gcg acc aag gtt atc ttc ctg act gcc gat gcc ttt ggt gtg ctc ccc     1104
   Ala Thr Lys Val Ile Phe Leu Thr Ala Asp Ala Phe Gly Val Leu Pro
           355                 360                 365
   cca gta tct cgt ctg acc gca aac cag acg caa tat cac ttc ctc tct     1152
   Pro Val Ser Arg Leu Thr Ala Asn Gln Thr Gln Tyr His Phe Leu Ser
       370                 375                 380
   ggc ttt act gcc aaa ctg gca ggg aca gag cgt ggc gtc acg gag cca     1200
   Gly Phe Thr Ala Lys Leu Ala Gly Thr Glu Arg Gly Val Thr Glu Pro
   385                 390                 395                 400
   aca cca acc ttc tct gct tgc ttt ggt gcg gcc ttc ctg tct ctg cac     1248
   Thr Pro Thr Phe Ser Ala Cys Phe Gly Ala Ala Phe Leu Ser Leu His
                   405                 410                 415
   cca acg cag tac gct gaa gtg ctg gtt aag cgt atg caa gcg gtt ggc     1296
   Pro Thr Gln Tyr Ala Glu Val Leu Val Lys Arg Met Gln Ala Val Gly
               420                 425                 430
   gca caa gcc tat ctg gtc aat acc ggt tgg aac ggg aca ggt aag cgt     1344
   Ala Gln Ala Tyr Leu Val Asn Thr Gly Trp Asn Gly Thr Gly Lys Arg
           435                 440                 445
   att tcc atc aag gat acc cgc gcc att att gac gca atc cta aac ggg     1392
   Ile Ser Ile Lys Asp Thr Arg Ala Ile Ile Asp Ala Ile Leu Asn Gly
       450                 455                 460
   gag att gat aag gca gaa acc ttt acg ctg cca atc ttt gat ctg gca     1440
   Glu Ile Asp Lys Ala Glu Thr Phe Thr Leu Pro Ile Phe Asp Leu Ala
   465                 470                 475                 480
   gtc cct atg gcg tta ccc ggt gtg aat ccc gat atc ctc gat cct cgc     1488
   Val Pro Met Ala Leu Pro Gly Val Asn Pro Asp Ile Leu Asp Pro Arg
                   485                 490                 495
   gac acc tac gcc gat aaa gcg caa tgg caa gag aaa gcc gaa gat ttg     1536
   Asp Thr Tyr Ala Asp Lys Ala Gln Trp Gln Glu Lys Ala Glu Asp Leu
               500                 505                 510
   gcg aaa cgc ttt gcg act aac ttt gat aaa tac act gat acc cct gcg     1584
   Ala Lys Arg Phe Ala Thr Asn Phe Asp Lys Tyr Thr Asp Thr Pro Ala
           515                 520                 525
   ggg gcc gcg ttg gtt agc gcg ggg cca aag atc taa                     1620
   Gly Ala Ala Leu Val Ser Ala Gly Pro Lys Ile
       530                 535
<210> 15
   <211> 539
   <212> PRT
   <213> Yersinia pseudotuberculosis
<400> 15
   Met Ser Val Lys Gly Ile Thr Pro Gln Glu Leu Ala Ala Tyr Gly Ile
   1               5                   10                  15
   His Asn Val Ser Glu Ile Val Tyr Asn Pro Ser Tyr Asp Leu Leu Phe
               20                  25                  30
   Glu Glu Glu Thr Lys Pro Thr Leu Glu Gly Tyr Glu Arg Gly Thr Leu
           35                  40                  45
   Thr Thr Thr Gly Ala Ile Ala Val Asp Thr Gly Ile Phe Thr Gly Arg
       50                  55                  60
   Ser Pro Lys Asp Lys Tyr Ile Val Arg Asp Ala Ile Thr Gln Asp Thr
   65                  70                  75                  80
   Val Trp Trp Ala Asp Gln Gly Lys Gly Lys Asn Asp Asn Lys Pro Leu
                   85                  90                  95
   Ser Gln Glu Ile Trp Ser His Leu Lys Gly Leu Val Thr Glu Gln Leu
               100                 105                 110
   Ser Gly Lys Arg Leu Phe Val Val Asp Thr Phe Cys Gly Ala Asn Ala
           115                 120                 125
   Asp Thr Arg Leu Gln Val Arg Phe Ile Thr Glu Val Ala Trp Gln Ala
       130                 135                 140
   His Phe Val Lys Asn Met Phe Ile Arg Pro Ser Asp Glu Glu Leu Ala
   145                 150                 155                 160
   Arg Phe Glu Pro Asp Phe Ile Val Met Asn Gly Ala Lys Cys Thr Asn
                   165                 170                 175
   Pro Gln Trp Lys Glu Gln Gly Leu Asn Ser Glu Asn Phe Val Ala Phe
               180                 185                 190
   Asn Leu Thr Glu Arg Met Gln Leu Ile Gly Gly Thr Trp Tyr Gly Gly
           195                 200                 205
   Glu Met Lys Lys Gly Met Phe Ser Met Met Asn Tyr Leu Leu Pro Leu
       210                 215                 220
   Lys Gly Ile Ala Ser Met His Cys Ser Ala Asn Val Gly Glu Lys Gly
   225                 230                 235                 240
   Asp Val Ala Ile Phe Phe Gly Leu Ser Gly Thr Gly Lys Thr Thr Leu
                   245                 250                 255
   Ser Thr Asp Pro Lys Arg Lys Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Lys Leu Ser Glu Glu Ala Glu Pro Asp Ile Tyr His Ala Ile Lys
       290                 295                 300
   Arg Asp Ala Leu Leu Glu Asn Val Val Val Leu Ala Asp Gly Thr Val
   305                 310                 315                 320
   Asp Phe Asn Asp Gly Ser Lys Thr Glu Asn Thr Arg Val Ser Tyr Pro
                   325                 330                 335
   Ile Tyr His Ile Asp Asn Ile Val Lys Pro Val Ser Lys Ala Gly His
               340                 345                 350
   Ala Thr Lys Val Ile Phe Leu Thr Ala Asp Ala Phe Gly Val Leu Pro
           355                 360                 365
   Pro Val Ser Arg Leu Thr Ala Asn Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Ser Leu His
                   405                 410                 415
   Pro Thr Gln Tyr Ala Glu Val Leu Val Lys Arg Met Gln Ala Val Gly
               420                 425                 430
   Ala Gln Ala Tyr Leu Val Asn Thr Gly Trp Asn Gly Thr Gly Lys Arg
           435                 440                 445
   Ile Ser Ile Lys Asp Thr Arg Ala Ile Ile Asp Ala Ile Leu Asn Gly
       450                 455                 460
   Glu Ile Asp Lys Ala Glu Thr Phe Thr Leu Pro Ile Phe Asp Leu Ala
   465                 470                 475                 480
   Val Pro Met Ala Leu Pro Gly Val Asn Pro Asp Ile Leu Asp Pro Arg
                   485                 490                 495
   Asp Thr Tyr Ala Asp Lys Ala Gln Trp Gln Glu Lys Ala Glu Asp Leu
               500                 505                 510
   Ala Lys Arg Phe Ala Thr Asn Phe Asp Lys Tyr Thr Asp Thr Pro Ala
                       515                     520                     525
   Gly Ala Ala Leu Val Ser Ala Gly Pro Lys Ile
       530                     535
<210> 16
   <211> 1629
   <212> DNA
   <213> vibrio cholerae
<220>
   <221> CDS
   <222> (1)..(1629)
<400> 16
   atg acc gtt atg gaa cat act aag gct gca caa atc gac cta gcc caa      48
   Met Thr Val Met Glu His Thr Lys Ala Ala Gln Ile Asp Leu Ala Gln
   1               5                   10                  15
   tat ggg atc acc ggc gta act gaa ctg gtt cgt aac ccg agc tat gaa      96
   Tyr Gly Ile Thr Gly Val Thr Glu Leu Val Arg Asn Pro Ser Tyr Glu
               20                  25                  30
   atg tta ttt gcc gaa gaa acc cgt tca gat ctc gaa ggt tat gaa cgt      144
   Met Leu Phe Ala Glu Glu Thr Arg Ser Asp Leu Glu Gly Tyr Glu Arg
           35                  40                  45
   ggt gtg gtg act gaa ttg ggt gct gtt gcg gtt gat act ggc atc ttc      192
   Gly Val Val Thr Glu Leu Gly Ala Val Ala Val Asp Thr Gly Ile Phe
       50                  55                  60
   act ggc cgc tca cca aaa gat aag ttt atc gtt aaa gat gat acc act      240
   Thr Gly Arg Ser Pro Lys Asp Lys Phe Ile Val Lys Asp Asp Thr Thr
   65                  70                  75                  80
   cgc gat acg ctg tgg tgg acg tca gac aaa gcg aaa aac gac aac aaa      288
   Arg Asp Thr Leu Trp Trp Thr Ser Asp Lys Ala Lys Asn Asp Asn Lys
                   85                  90                  95
   ccg atc aat caa gaa gtg tgg aat gac ctg aaa gcc ttg gtg acc aag      336
   Pro Ile Asn Gln Glu Val Trp Asn Asp Leu Lys Ala Leu Val Thr Lys
               100                 105                 110
   cag ctt tct ggt aaa cgc gta ttt gtg ctc gat ggc tac tgt ggt gcc      384
   Gln Leu Ser Gly Lys Arg Val Phe Val Leu Asp Gly Tyr Cys Gly Ala
           115                 120                 125
   aac gcc gat act cgc tta agt gtt cgc ttc atc acc gaa gta gca tgg      432
   Asn Ala Asp Thr Arg Leu Ser Val Arg Phe Ile Thr Glu Val Ala Trp
       130                 135                 140
   caa gca cac ttt gtg aaa aac atg ttc att cgt cca agc gaa gaa gag      480
   Gln Ala His Phe Val Lys Asn Met Phe Ile Arg Pro Ser Glu Glu Glu
   145                 150                 155                 160
   ctg gca cac ttt aaa cca gac ttt gtc gta atg aac ggc gca aaa tgt      528
   Leu Ala His Phe Lys Pro Asp Phe Val Val Met Asn Gly Ala Lys Cys
                   165                 170                 175
   acc aat gcg aag tgg aaa gag cac ggt ctg aac tca gaa aac ttc act      576
   Thr Asn Ala Lys Trp Lys Glu His Gly Leu Asn Ser Glu Asn Phe Thr
               180                 185                 190
   gtg ttt aac ctg acc gag cgc atg cag ctc atc ggc ggt act tgg tac      624
   Val Phe Asn Leu Thr Glu Arg Met Gln Leu Ile Gly Gly Thr Trp Tyr
           195                 200                 205
   ggc ggt gag atg aaa aaa ggt atg ttc gcg atg atg aac tac ttc ctg      672
   Gly Gly Glu Met Lys Lys Gly Met Phe Ala Met Met Asn Tyr Phe Leu
       210                 215                 220
   ccg cta caa ggc att gcc tct atg cac tgc tct gcc aac atg ggt aaa      720
   Pro Leu Gln Gly Ile Ala Ser Met His Cys Ser Ala Asn Met Gly Lys
   225                 230                 235                 240
   gcg ggc gat gtc gcc atc ttc ttc ggt ctt tct ggt acg ggt aaa acc      768
   Ala Gly Asp Val Ala Ile Phe Phe Gly Leu Ser Gly Thr Gly Lys Thr
                   245                 250                 255
   acc cta tcc acc gat cca aaa cgt gcg tta att ggt gac gat gag cac      816
   Thr Leu Ser Thr Asp Pro Lys Arg Ala Leu Ile Gly Asp Asp Glu His
               260                 265                 270
   ggc tgg gat gat gat ggc gtg ttc aac ttt gaa ggc ggc tgc tac gcg      864
   Gly Trp Asp Asp Asp Gly Val Phe Asn Phe Glu Gly Gly Cys Tyr Ala
           275                 280                 285
   aaa acc atc aag ctg tct aaa gaa gca gag ccg gat atc tat aac gcg      912
   Lys Thr Ile Lys Leu Ser Lys Glu Ala Glu Pro Asp Ile Tyr Asn Ala
       290                 295                 300
   atc cgc cgt gat gct cta ctg gaa aac gtc acg gtt cgt agt gat ggt      960
   Ile Arg Arg Asp Ala Leu Leu Glu Asn Val Thr Val Arg Ser Asp Gly
   305                 310                 315                 320
   tcg att gat ttt gat gat ggt tca aaa acc gag aac acc cgt gtt tct     1008
   Ser Ile Asp Phe Asp Asp Gly Ser Lys Thr Glu Asn Thr Arg Val Ser
                   325                 330                 335
   tac cct att tat cac atc gac aac atc gta aaa ccc gtt tcc aaa ggc     1056
   Tyr Pro Ile Tyr His Ile Asp Asn Ile Val Lys Pro Val Ser Lys Gly
               340                 345                 350
   ggt cat gcg act aag gtg atc ttc ctg tct gcc gat gcg ttt ggc gta     1104
   Gly His Ala Thr Lys Val Ile Phe Leu Ser Ala Asp Ala Phe Gly Val
           355                 360                 365
   ttg cct cca gtt tca aaa ctg acg cca gag caa acc aag tac cac ttc     1152
   Leu Pro Pro Val Ser Lys Leu Thr Pro Glu Gln Thr Lys Tyr His Phe
       370                 375                 380
   ttg tct ggc ttt acg gct aaa ctg gca ggt act gag cgt ggc att act     1200
   Leu Ser Gly Phe Thr Ala Lys Leu Ala Gly Thr Glu Arg Gly Ile Thr
   385                 390                 395                 400
   gaa cct acc cca acc ttc tcc gcc tgt ttt ggc gca gcg ttc ctc act     1248
   Glu Pro Thr Pro Thr Phe Ser Ala Cys Phe Gly Ala Ala Phe Leu Thr
                   405                 410                 415
   ctg cac cca act cag tat gca gaa gtg ctg gta aaa cgt atg gaa gca     1296
   Leu His Pro Thr Gln Tyr Ala Glu Val Leu Val Lys Arg Met Glu Ala
               420                 425                 430
   gcg ggc gcc gaa gcc tat ctg gtt aac aca ggt tgg aac ggc agc ggt     1344
   Ala Gly Ala Glu Ala Tyr Leu Val Asn Thr Gly Trp Asn Gly Ser Gly
           435                 440                 445
   aag cgc atc tca att aaa gat acg cgc ggt att atc gat gcg att ttg     1392
   Lys Arg Ile Ser Ile Lys Asp Thr Arg Gly Ile Ile Asp Ala Ile Leu
       450                 455                 460
   gat ggt tcg atc gaa aaa gcg gaa acc aaa cag atc cca atc ttt aat     1440
   Asp Gly Ser Ile Glu Lys Ala Glu Thr Lys Gln Ile Pro Ile Phe Asn
   465                 470                 475                 480
   ctg caa gtg ccc acc gca ctg ccc ggc gtc gat cct atg atc ctc gac     1488
   Leu Gln Val Pro Thr Ala Leu Pro Gly Val Asp Pro Met Ile Leu Asp
                   485                 490                 495
   cca cgt gat act tat gtt gac cca ctg cag tgg gaa agc aaa gcc aaa     1536
   Pro Arg Asp Thr Tyr Val Asp Pro Leu Gln Trp Glu Ser Lys Ala Lys
               500                 505                 510
   gac ttg gca acg cgc ttc atc aac aac ttc gac aag tac acg gat aac     1584
   Asp Leu Ala Thr Arg Phe Ile Asn Asn Phe Asp Lys Tyr Thr Asp Asn
           515                 520                 525
   gcc gaa ggt aaa gca ctg gtt gcc gcg ggt cca aag ctc gac taa         1629
   Ala Glu Gly Lys Ala Leu Val Ala Ala Gly Pro Lys Leu Asp
       530                 535                 540
<210> 17
   <211> 542
   <212> PRT
   <213> vibrio cholerae
<400> 17
   Met Thr Val Met Glu His Thr Lys Ala Ala Gln Ile Asp Leu Ala Gln
   1               5                   10                  15
   Tyr Gly Ile Thr Gly Val Thr Glu Leu Val Arg Asn Pro Ser Tyr Glu
                   20                      25                      30
   Met Leu Phe Ala Glu Glu Thr Arg Ser Asp Leu Glu Gly Tyr Glu Arg
               35                      40                      45
   Gly Val Val Thr Glu Leu Gly Ala Val Ala Val Asp Thr Gly Ile Phe
           50                      55                      60
   Thr Gly Arg Ser Pro Lys Asp Lys Phe Ile Val Lys Asp Asp Thr Thr
   65                      70                      75                      80
   Arg Asp Thr Leu Trp Trp Thr Ser Asp Lys Ala Lys Asn Asp Asn Lys
                   85                      90                      95
   Pro Ile Asn Gln Glu Val Trp Asn Asp Leu Lys Ala Leu Val Thr Lys
               100                     105                     110
   Gln Leu Ser Gly Lys Arg Val Phe Val Leu Asp Gly Tyr Cys Gly Ala
           115                     120                     125
   Asn Ala Asp Thr Arg Leu Ser Val Arg Phe Ile Thr Glu Val Ala Trp
       130                     135                     140
   Gln Ala His Phe Val Lys Asn Met Phe Ile Arg Pro Ser Glu Glu Glu
   145                     150                     155                     160
   Leu Ala His Phe Lys Pro Asp Phe Val Val Met Asn Gly Ala Lys Cys
                   165                     170                     175
   Thr Asn Ala Lys Trp Lys Glu His Gly Leu Asn Ser Glu Asn Phe Thr
               180                     185                     190
   Val Phe Asn Leu Thr Glu Arg Met Gln Leu Ile Gly Gly Thr Trp Tyr
           195                     200                     205
   Gly Gly Glu Met Lys Lys Gly Met Phe Ala Met Met Asn Tyr Phe Leu
       210                     215                     220
   Pro Leu Gln Gly Ile Ala Ser Met His Cys Ser Ala Asn Met Gly Lys
   225                     230                     235                     240
   Ala Gly Asp Val Ala Ile Phe Phe Gly Leu Ser Gly Thr Gly Lys Thr
                   245                     250                     255
   Thr Leu Ser Thr Asp Pro Lys Arg Ala Leu Ile Gly Asp Asp Glu His
               260                     265                     270
   Gly Trp Asp Asp Asp Gly Val Phe Asn Phe Glu Gly Gly Cys Tyr Ala
           275                     280                     285
   Lys Thr Ile Lys Leu Ser Lys Glu Ala Glu Pro Asp Ile Tyr Asn Ala
       290                     295                     300
   Ile Arg Arg Asp Ala Leu Leu Glu Asn Val Thr Val Arg Ser Asp Gly
   305                     310                     315                     320
   Ser Ile Asp Phe Asp Asp Gly Ser Lys Thr Glu Asn Thr Arg Val Ser
                   325                     330                     335
   Tyr Pro Ile Tyr His Ile Asp Asn Ile Val Lys Pro Val Ser Lys Gly
               340                     345                     350
   Gly His Ala Thr Lys Val Ile Phe Leu Ser Ala Asp Ala Phe Gly Val
           355                     360                     365
   Leu Pro Pro Val Ser Lys Leu Thr Pro Glu Gln Thr Lys Tyr His Phe
       370                     375                     380
   Leu Ser Gly Phe Thr Ala Lys Leu Ala Gly Thr Glu Arg Gly Ile Thr
   385                     390                     395                     400
   Glu Pro Thr Pro Thr Phe Ser Ala Cys Phe Gly Ala Ala Phe Leu Thr
                   405                     410                     415
   Leu His Pro Thr Gln Tyr Ala Glu Val Leu Val Lys Arg Met Glu Ala
               420                     425                     430
   Ala Gly Ala Glu Ala Tyr Leu Val Asn Thr Gly Trp Asn Gly Ser Gly
           435                     440                     445
   Lys Arg Ile Ser Ile Lys Asp Thr Arg Gly Ile Ile Asp Ala Ile Leu
       450                     455                     460
   Asp Gly Ser Ile Glu Lys Ala Glu Thr Lys Gln Ile Pro Ile Phe Asn
   465                     470                     475                     480
   Leu Gln Val Pro Thr Ala Leu Pro Gly Val Asp Pro Met Ile Leu Asp
                   485                     490                     495
   Pro Arg Asp Thr Tyr Val Asp Pro Leu Gln Trp Glu Ser Lys Ala Lys
               500                     505                     510
   Asp Leu Ala Thr Arg Phe Ile Asn Asn Phe Asp Lys Tyr Thr Asp Asn
           515                     520                     525
   Ala Glu Gly Lys Ala Leu Val Ala Ala Gly Pro Lys Leu Asp
           530                     535                     540
<210> 18
   <211> 89
   <212> DNA
   <213> Artificial Sequence
<220>
   <223> primer for deleting adhE
<400> 18
   caagttgata agatcttccg tgccgccgct ctggccgctg cagatgctcg aatccctctc 60
   tgaagcctgc ttttttatac taagttggc 89
<210> 19
   <211> 89
   <212> DNA
   <213> Artificial Sequence
<220>
   <223> primer for deleting adhE
<400> 19
   acgtgagcca ggaaatcagc ttcctgaacg ccggcttcgc gaatagattt cggaataccc 60
   gctcaagtta gtataaaaaa gctgaacga 89
<210> 20
   <211> 1261
   <212> DNA
   <213> Enterobacter aerogenes
<220>
   <221> CDS
   <222> (147)..(1136)
<400> 20
   aacgtattgt cttttaaaga gatttcttaa gactgcgata tgctctagaa ttaatatcat      60
   aacctgatga ttaaactaat ttttaatatc tgtaagatta ttttaattat gctaccgtga     120
   cggtattatc actagaggaa agcctt atg aaa atc gct gtt tat agt acc aag      173
                                Met Lys Ile Ala Val Tyr Ser Thr Lys
                                1               5
   cag tac gat aaa aag tat ctg cag cat gtt aac gac gca tac ggc ttt      221
   Gln Tyr Asp Lys Lys Tyr Leu Gln His Val Asn Asp Ala Tyr Gly Phe
   10                  15                  20                  25
   gaa ctg gaa ttt ttc gac ttc ctg ctg acc gaa aag acc gcg aaa acg      269
   Glu Leu Glu Phe Phe Asp Phe Leu Leu Thr Glu Lys Thr Ala Lys Thr
                   30                  35                  40
   gcc aac ggc tgt gaa gcg gta tgt atc ttc gtt aac gac gac ggc agc      317
   Ala Asn Gly Cys Glu Ala Val Cys Ile Phe Val Asn Asp Asp Gly Ser
               45                  50                  55
   cgc ccg gtg ctg gaa gag cta aaa gcc cac ggc gtg aaa tac atc gca      365
   Arg Pro Val Leu Glu Glu Leu Lys Ala His Gly Val Lys Tyr Ile Ala
           60                  65                  70
   cta cgc tgc gcc ggc ttt aac aac gtc gat ctt gat gcg gct aaa gag      413
   Leu Arg Cys Ala Gly Phe Asn Asn Val Asp Leu Asp Ala Ala Lys Glu
       75                  80                  85
   ctg ggc ctg cgc gtc gtg cgc gtc ccg gcc tac tcg ccg gaa gcc gtt      461
   Leu Gly Leu Arg Val Val Arg Val Pro Ala Tyr Ser Pro Glu Ala Val
   90                  95                  100                 105
   gct gaa cac gcc atc ggt atg atg atg tcg ttg aac cgt cgc att cac      509
   Ala Glu His Ala Ile Gly Met Met Met Ser Leu Asn Arg Arg Ile His
                       110                     115                     120
   cgc gcc tat cag cgt acc cgc gat gcc aac ttc tcg ctg gaa ggg ctg      557
   Arg Ala Tyr Gln Arg Thr Arg Asp Ala Asn Phe Ser Leu Glu Gly Leu
                   125                     130                     135
   acc ggc ttc acg atg tac ggc aaa acc gca ggg gtg atc ggc acc ggt      605
   Thr Gly Phe Thr Met Tyr Gly Lys Thr Ala Gly Val Ile Gly Thr Gly
               140                     145                     150
   aaa atc ggc gtc gcg acg ctg cgg atc ctc aaa ggt ttc ggt atg cgc      653
   Lys Ile Gly Val Ala Thr Leu Arg Ile Leu Lys Gly Phe Gly Met Arg
           155                     160                     165
   ctg ctg gcg ttt gat ccc tac ccg agc gcg gcg gcg ctg gat ctc ggc      701
   Leu Leu Ala Phe Asp Pro Tyr Pro Ser Ala Ala Ala Leu Asp Leu Gly
       170                     175                     180                     185
   gtt gaa tat gtc gac ctg ccg acg ctg tac gcg cag tcc gac gtc atc      749
   Val Glu Tyr Val Asp Leu Pro Thr Leu Tyr Ala Gln Ser Asp Val Ile
                       190                     195                     200
   tcc ctg cac tgc ccg ctt acc aac gaa aac tat cac ctg ctc aac cag      797
   Ser Leu His Cys Pro Leu Thr Asn Glu Asn Tyr His Leu Leu Asn Gln
                   205                     210                     215
   gcg gca ttc gat cag atg aaa gac ggc gtg atg gtc att aat acc agc      845
   Ala Ala Phe Asp Gln Met Lys Asp Gly Val Met Val Ile Asn Thr Ser
               220                     225                     230
   cgc ggc gcg cta ata gat tca caa gcg gct atc gac gcg ctg aag cat      893
   Arg Gly Ala Leu Ile Asp Ser Gln Ala Ala Ile Asp Ala Leu Lys His
           235                     240                     245
   cag aaa atc ggc gca ctg gga atg gac gtg tat gaa aat gaa cgc gat      941
   Gln Lys Ile Gly Ala Leu Gly Met Asp Val Tyr Glu Asn Glu Arg Asp
       250                     255                     260                     265
   ctg ttc ttt gaa gat aaa tcg aat gat gtc atc cag gat gac gtg ttc      989
   Leu Phe Phe Glu Asp Lys Ser Asn Asp Val Ile Gln Asp Asp Val Phe
                       270                     275                     280
   cgc cgg ctc tcc gcc tgc cac aac gtc ctg ttt acc ggg cac cag gca      1037
   Arg Arg Leu Ser Ala Cys His Asn Val Leu Phe Thr Gly His Gln Ala
                   285                     290                     295
   ttc ctg acg gct gag gcg ctg atc ggt att tcc gag aca acg ctt ggc      1085
   Phe Leu Thr Ala Glu Ala Leu Ile Gly Ile Ser Glu Thr Thr Leu Gly
               300                     305                     310
   aat ctg cag cag gta gct aag ggc gaa acc tgc ccg aac gcg ctg gtc      1133
   Asn Leu Gln Gln Val Ala Lys Gly Glu Thr Cys Pro Asn Ala Leu Val
           315                     320                     325
   taa gcttctttcc ccttttgtgc tccccgctaa cggggggcac attcagacaa           1186
   tccccacaga tcctgcctgc attacagtta cactgttttt cgttttattg atatgacaaa    1246
   tgttttggag tacga                                                     1261
<210> 21
   <211> 3348
   <212> DNA
   <213> Enterobacter aerogenes
<220>
   <221> CDS
   <222> (301)..(3048)
<400> 21
   atcattaacc ttgacttgac tggttaatta ttgacctgct gaaaggtatc agcatcgcca      60
   aatattgatc caaatcacgt aattaatact cagaagtgag taatcttggc tacatcaaca     120
   gagtgaaagg ccgaagtacc tgagtgcgta gtgagacaaa gcttttttag taaatcagtg      180
   ggttcaaagc gttagaaatc tttacgttaa catttacatg caagctgtta acagcctgtc     240
   tataatgtcg aatcgagcta ctgttttact aaaaaagttt aacattatca ggagagcatt     300
   atg gct gtt act aat gtc gct gaa ctt aac gca ctt gta gag cgc gta      348
   Met Ala Val Thr Asn Val Ala Glu Leu Asn Ala Leu Val Glu Arg Val
   1               5                   10                  15
   aaa aaa gcc cag cgt gaa tat gcc agt ttc act caa gaa caa gtt gat      396
   Lys Lys Ala Gln Arg Glu Tyr Ala Ser Phe Thr Gln Glu Gln Val Asp
               20                  25                  30
   aag atc ttc cgt gcc gcc gct ctg gcc gct gca gat gct cga atc cct      444
   Lys Ile Phe Arg Ala Ala Ala Leu Ala Ala Ala Asp Ala Arg Ile Pro
           35                  40                  45
   ctc gct aaa atg gct gtt gcc gaa tcc ggc atg ggt att att gaa gat      492
   Leu Ala Lys Met Ala Val Ala Glu Ser Gly Met Gly Ile Ile Glu Asp
       50                  55                  60
   aaa gtg atc aaa aac cac ttc gct tcc gaa tat atc tac aac gcc tat      540
   Lys Val Ile Lys Asn His Phe Ala Ser Glu Tyr Ile Tyr Asn Ala Tyr
   65                  70                  75                  80
   aaa gat gaa aag acc tgt ggc gtc ctg tct gaa gac gac act ttc ggt      588
   Lys Asp Glu Lys Thr Cys Gly Val Leu Ser Glu Asp Asp Thr Phe Gly
                   85                  90                  95
   acc atc acc att gct gag cca atc ggt att att tgc ggt atc gtc ccg      636
   Thr Ile Thr Ile Ala Glu Pro Ile Gly Ile Ile Cys Gly Ile Val Pro
               100                 105                 110
   act acc aac ccg act tct act gct atc ttc aaa tcg ctg atc agc ctg      684
   Thr Thr Asn Pro Thr Ser Thr Ala Ile Phe Lys Ser Leu Ile Ser Leu
           115                 120                 125
   aag acg cgt aac gcc atc atc ttc tct ccg cac ccg cgt gct aaa gac      732
   Lys Thr Arg Asn Ala Ile Ile Phe Ser Pro His Pro Arg Ala Lys Asp
       130                 135                 140
   gcg act aac aaa gcg gcg gac atc gta ttg cag gca gct att gcc gca      780
   Ala Thr Asn Lys Ala Ala Asp Ile Val Leu Gln Ala Ala Ile Ala Ala
   145                 150                 155                 160
   ggc gcg ccg aaa gat ctg atc ggt tgg atc gac cag cct tcc gta gaa      828
   Gly Ala Pro Lys Asp Leu Ile Gly Trp Ile Asp Gln Pro Ser Val Glu
                   165                 170                 175
   ctg tct aac gca ctg atg cat cat cct gac atc aac ctg atc ctc gcc      876
   Leu Ser Asn Ala Leu Met His His Pro Asp Ile Asn Leu Ile Leu Ala
               180                 185                 190
   acc ggc ggc cca ggt atg gtt aag gcc gct tat agc tcc ggt aaa cca      924
   Thr Gly Gly Pro Gly Met Val Lys Ala Ala Tyr Ser Ser Gly Lys Pro
           195                 200                 205
   gct atc ggc gtt ggc gcg ggc aac acg ccg gtt gtt att gat gaa acg      972
   Ala Ile Gly Val Gly Ala Gly Asn Thr Pro Val Val Ile Asp Glu Thr
       210                 215                 220
   gct gat atc aaa cgc gct gtg gcg tcc gta ctg atg tca aaa acc ttc     1020
   Ala Asp Ile Lys Arg Ala Val Ala Ser Val Leu Met Ser Lys Thr Phe
   225                 230                 235                 240
   gat aac ggc gtt atc tgt gct tct gaa caa tcc gtg gtg gtt gtc gac     1068
   Asp Asn Gly Val Ile Cys Ala Ser Glu Gln Ser Val Val Val Val Asp
                   245                 250                 255
   tcc gtc tac gac gcc gtc cgc gag cgt ttt gcc agc cat ggc ggc tac     1116
   Ser Val Tyr Asp Ala Val Arg Glu Arg Phe Ala Ser His Gly Gly Tyr
               260                 265                 270
   ctg ctg cag ggt aaa gag ctg aaa gcc gtt cag gac atc atc ctg aaa     1164
   Leu Leu Gln Gly Lys Glu Leu Lys Ala Val Gln Asp Ile Ile Leu Lys
           275                 280                 285
   aat ggc gcg ctg aat gcg gcg atc gtt ggt caa cca gcg gca aaa atc     1212
   Asn Gly Ala Leu Asn Ala Ala Ile Val Gly Gln Pro Ala Ala Lys Ile
       290                 295                 300
   gct gaa ctg gca ggc ttc acc gtg cca gcc acc act aag att ctg atc     1260
   Ala Glu Leu Ala Gly Phe Thr Val Pro Ala Thr Thr Lys Ile Leu Ile
   305                 310                 315                 320
   ggc gaa gtt acc gac gtt gac gaa agc gaa ccg ttc gct cac gaa aaa     1308
   Gly Glu Val Thr Asp Val Asp Glu Ser Glu Pro Phe Ala His Glu Lys
                   325                 330                 335
   ctg tct ccg acg ctg gca atg tac cgt gcg aaa gat ttc gaa gac gcg     1356
   Leu Ser Pro Thr Leu Ala Met Tyr Arg Ala Lys Asp Phe Glu Asp Ala
               340                 345                 350
   gtc aat aaa gca gaa aaa ctg gtc gcc atg ggc ggt atc ggt cac acc     1404
   Val Asn Lys Ala Glu Lys Leu Val Ala Met Gly Gly Ile Gly His Thr
           355                             360                             365
   tct tgc ctg tac acc gac cag gac aac cag ccg gct cgc gtg gcc tac      1452
   Ser Cys Leu Tyr Thr Asp Gln Asp Asn Gln Pro Ala Arg Val Ala Tyr
       370                     375                     380
   ttc ggc cag atg atg aaa acc gcg cgt atc ctg atc aac acc ccg gct      1500
   Phe Gly Gln Met Met Lys Thr Ala Arg Ile Leu Ile Asn Thr Pro Ala
   385                 390                     395                 400
   tcc cag ggt ggt atc ggc gac ctg tat aac ttc aag ctc gca cct tcc      1548
   Ser Gln Gly Gly Ile Gly Asp Leu Tyr Asn Phe Lys Leu Ala Pro Ser
                   405                     410                 415
   ctg act ctg ggt tgt ggt tcc tgg ggt ggt aac tcc atc tct gaa aac      1596
   Leu Thr Leu Gly Cys Gly Ser Trp Gly Gly Asn Ser Ile Ser Glu Asn
               420                 425                 430
   gtt ggt ccg aaa cac ctg atc aac aag aaa acc gtt gct aag cga gct      1644
   Val Gly Pro Lys His Leu Ile Asn Lys Lys Thr Val Ala Lys Arg Ala
           435                     440                     445
   gaa aac atg ttg tgg cat aaa ctt ccg aaa tct atc tac ttc cgt cgt      1692
   Glu Asn Met Leu Trp His Lys Leu Pro Lys Ser Ile Tyr Phe Arg Arg
       450                     455                     460
   ggc tca ctg cca atc gca ctg gat gaa gtg att act gat ggt cac aaa      1740
   Gly Ser Leu Pro Ile Ala Leu Asp Glu Val Ile Thr Asp Gly His Lys
   465                 470                     475                 480
   cgc gcg ctg att gtg act gac cgc ttc ctg ttc aac aac ggt tac gcg      1788
   Arg Ala Leu Ile Val Thr Asp Arg Phe Leu Phe Asn Asn Gly Tyr Ala
                   485                     490                 495
   gac cag atc act tcc gta ctg aaa gcg gct ggc gta gaa acc gaa gtg      1836
   Asp Gln Ile Thr Ser Val Leu Lys Ala Ala Gly Val Glu Thr Glu Val
               500                     505                     510
   ttc ttt gaa gtt gaa gct gac cca acg ctg act atc gtg cgt aaa ggc      1884
   Phe Phe Glu Val Glu Ala Asp Pro Thr Leu Thr Ile Val Arg Lys Gly
           515                     520                     525
   gcg gat ctg gcc aac tcc ttc aaa cca gac gta atc atc gcc ctg ggc      1932
   Ala Asp Leu Ala Asn Ser Phe Lys Pro Asp Val Ile Ile Ala Leu Gly
       530                     535                     540
   ggc ggt tcc ccg atg gat gcg gca aaa atc atg tgg gtc atg tac gag      1980
   Gly Gly Ser Pro Met Asp Ala Ala Lys Ile Met Trp Val Met Tyr Glu
   545                 550                     555                 560
   cac ccg gaa acc cac ttc gaa gaa ctg gcg ctg cgc ttt atg gat atc      2028
   His Pro Glu Thr His Phe Glu Glu Leu Ala Leu Arg Phe Met Asp Ile
                   565                     570                 575
   cgt aaa cgt atc tac aag ttc ccg aaa atg ggc gtc aaa gcc aag atg      2076
   Arg Lys Arg Ile Tyr Lys Phe Pro Lys Met Gly Val Lys Ala Lys Met
               580                     585                 590
   gtt gcc att acc acc acc tcc ggt acc ggt tct gaa gtt acc ccg ttc      2124
   Val Ala Ile Thr Thr Thr Ser Gly Thr Gly Ser Glu Val Thr Pro Phe
           595                     600                     605
   gcc gta gta acc gac gat gca act gga cag aaa tat ccg ctg gct gac      2172
   Ala Val Val Thr Asp Asp Ala Thr Gly Gln Lys Tyr Pro Leu Ala Asp
       610                     615                     620
   tac gct ctg act ccg gat atg gcg att gtc gat gcc aac ctg gtc atg      2220
   Tyr Ala Leu Thr Pro Asp Met Ala Ile Val Asp Ala Asn Leu Val Met
   625                 630                     635                 640
   gat atg ccg aag tct ctc tgt gcc ttc ggt ggt ctg gat gcc gtg act      2268
   Asp Met Pro Lys Ser Leu Cys Ala Phe Gly Gly Leu Asp Ala Val Thr
                   645                     650                 655
   cac gct ctg gaa gct tac gtc tcc gta ctg gct tct gag ttc tcc gat      2316
   His Ala Leu Glu Ala Tyr Val Ser Val Leu Ala Ser Glu Phe Ser Asp
               660                     665                 670
   ggt cag gct ctg cag gcg ctg aaa ctg ctg aaa gag tat ctg ccg gcc      2364
   Gly Gln Ala Leu Gln Ala Leu Lys Leu Leu Lys Glu Tyr Leu Pro Ala
           675                     680                     685
   tct tac cat gaa ggt tct aag aac ccg gta gcc cgc gaa cgt gtg cac      2412
   Ser Tyr His Glu Gly Ser Lys Asn Pro Val Ala Arg Glu Arg Val His
       690                     695                     700
   agc gcc gcc act atc gcc ggt atc gcg ttt gct aac gcc ttc ctc ggc      2460
   Ser Ala Ala Thr Ile Ala Gly Ile Ala Phe Ala Asn Ala Phe Leu Gly
   705                 710                 715                 720
   gtg tgc cac tca atg gcg cac aaa ctg ggc tcg cag ttc cac att cct      2508
   Val Cys His Ser Met Ala His Lys Leu Gly Ser Gln Phe His Ile Pro
                   725                 730                 735
   cac ggt ctg gca aac gcc ctg ctg atc agc aac gtt atc cgc tat aac      2556
   His Gly Leu Ala Asn Ala Leu Leu Ile Ser Asn Val Ile Arg Tyr Asn
               740                 745                 750
   gcg aat gac aac ccg acc aag cag acc gca ttc agc cag tat gac cgt      2604
   Ala Asn Asp Asn Pro Thr Lys Gln Thr Ala Phe Ser Gln Tyr Asp Arg
           755                 760                 765
   ccg cag gcg cgc cgt cgc tac gct gag atc gca gac cac ctg ggc ctg      2652
   Pro Gln Ala Arg Arg Arg Tyr Ala Glu Ile Ala Asp His Leu Gly Leu
       770                 775                 780
   agc gct ccg ggc gac cgc act gcg gcg aaa atc gag aaa ctg ctg gca      2700
   Ser Ala Pro Gly Asp Arg Thr Ala Ala Lys Ile Glu Lys Leu Leu Ala
   785                 790                 795                 800
   tgg ctg gaa agc ctc aaa gct gaa ctg ggt att ccg aaa tct att cgc      2748
   Trp Leu Glu Ser Leu Lys Ala Glu Leu Gly Ile Pro Lys Ser Ile Arg
                   805                 810                 815
   gaa gcc ggc gtt cag gaa gct gat ttc ctg gct cac gtt gat aag ctg      2796
   Glu Ala Gly Val Gln Glu Ala Asp Phe Leu Ala His Val Asp Lys Leu
               820                 825                 830
   tct gaa gac gcc ttc gat gac cag tgc acc ggc gct aac ccg cgc tac      2844
   Ser Glu Asp Ala Phe Asp Asp Gln Cys Thr Gly Ala Asn Pro Arg Tyr
           835                 840                 845
   ccg ctg atc gcc gag ctg aaa cag att ctg ctg gat acc tac tac ggt      2892
   Pro Leu Ile Ala Glu Leu Lys Gln Ile Leu Leu Asp Thr Tyr Tyr Gly
       850                 855                 860
   cgt gac ttt gta gaa ggt gaa gcg gcg gct gtg aaa aaa gaa gcg gct      2940
   Arg Asp Phe Val Glu Gly Glu Ala Ala Ala Val Lys Lys Glu Ala Ala
   865                 870                 875                 880
   ccg gcc aaa gct gag aaa aaa gac gaa aaa atc cgc tta att cgc aga      2988
   Pro Ala Lys Ala Glu Lys Lys Asp Glu Lys Ile Arg Leu Ile Arg Arg
                   885                 890                 895
   att gaa atg aac gaa gcc cca tct att gat ggg gct ttt ttt ata ttc      3036
   Ile Glu Met Asn Glu Ala Pro Ser Ile Asp Gly Ala Phe Phe Ile Phe
               900                 905                 910
   gta ctt tat tag agtttcatcc aggagtattg tatgcaatga atctggtgaa          3088
   Val Leu Tyr
           915
   gtcggaataa aggaaccgat tccataaaag acaacaggcg ccatttagac gcctgaaggt    3148
   aatgaaatta cagcattatt cgccgtggcg cagcttactt tgcacctgag ttaatgaacc    3208
   ggtgcttaac gcctctttat aatgcttacg gcatacggaa acgtaccgct cattaccgcc    3268
   aatcactacc tgttctcctt cattgtatgg ccgcccttcc tgatcaaggc gtaacaccat    3328
   gcttgcctta cggccacaga                                                3348
<210> 22
   <211> 3420
   <212> DNA
   <213> Brevibacterium fluvum
<220>
   <221> CDS
   <222> (1)..(3420)
<400> 22
   atg tcg act cac aca tct tca acg ctt cca gca ttc aaa aag atc ttg        48
   Met Ser Thr His Thr Ser Ser Thr Leu Pro Ala Phe Lys Lys Ile Leu
   1               5                   10                  15
   gta gca aac cgc ggc gaa atc gcg gtc cgt gct ttc cgt gca gca ctc        96
   Val Ala Asn Arg Gly Glu Ile Ala Val Arg Ala Phe Arg Ala Ala Leu
   20 25 30
   gaa acc ggt gca gcc acg gta gct att tac ccc cgt gaa gat cgg gga 144
   Glu Thr Gly Ala Ala Thr Val Ala Ile Tyr Pro Arg Glu Asp Arg Gly
   35 40 45
   tca ttc cac cgc tct ttt gct tct gaa gct gtc cgc att ggt act gaa 192
   Ser Phe His Arg Ser Phe Ala Ser Glu Ala Val Arg Ile Gly Thr Glu
   50 55 60
   ggc tca cca gtc aag gcg tac ctg gac atc gat gaa att atc ggt gca 240
   Gly Ser Pro Val Lys Ala Tyr Leu Asp Ile Asp Glu Ile Ile Gly Ala
   65 70 75 80
   gct aaa aaa gtt aaa gca gat gct att tac ccg gga tat ggc ttc ctg 288
   Ala Lys Lys Val Lys Ala Asp Ala Ile Tyr Pro Gly Tyr Gly Phe Leu
   85 90 95
   tct gaa aat gcc cag ctt gcc cgc gag tgc gcg gaa aac ggc att act 336
   Ser Glu Asn Ala Gln Leu Ala Arg Glu Cys Ala Glu Asn Gly Ile Thr
   100 105 110
   ttt att ggc cca acc cca gag gtt ctt gat ctc acc ggt gat aag tct 384
   Phe Ile Gly Pro Thr Pro Glu Val Leu Asp Leu Thr Gly Asp Lys Ser
   115 120 125
   cgt gcg gta acc gcc gcg aag aag gct ggt ctg cca gtt ttg gcg gaa 432
   Arg Ala Val Thr Ala Ala Lys Lys Ala Gly Leu Pro Val Leu Ala Glu
   130 135 140
   tcc acc ccg agc aaa aac atc gat gac atc gtt aaa agc gct gaa ggc 480
   Ser Thr Pro Ser Lys Asn Ile Asp Asp Ile Val Lys Ser Ala Glu Gly
   145 150 155 160
   cag act tac ccc atc ttt gta aag gca gtt gcc ggt ggt ggc gga cgc 528
   Gln Thr Tyr Pro Ile Phe Val Lys Ala Val Ala Gly Gly Gly Gly Arg
   165 170 175
   ggt atg cgc ttt gtt tct tca cct gat gag ctt cgc aaa ttg gca aca 576
   Gly Met Arg Phe Val Ser Ser Pro Asp Glu Leu Arg Lys Leu Ala Thr
   180 185 190
   gaa gca tct cgt gaa gct gaa gcg gca ttc ggc gac ggt tcg gta tat 624
   Glu Ala Ser Arg Glu Ala Glu Ala Ala Phe Gly Asp Gly Ser Val Tyr
   195 200 205
   gtc gag cgt gct gtg att aac ccc cag cac att gaa gtg cag atc ctt 672
   Val Glu Arg Ala Val Ile Asn Pro Gln His Ile Glu Val Gln Ile Leu
   210 215 220
   ggc gat cgc act gga gaa gtt gta cac ctt tat gaa cgt gac tgc tca 720
   Gly Asp Arg Thr Gly Glu Val Val His Leu Tyr Glu Arg Asp Cys Ser
   225 230 235 240
   ctg cag cgt cgt cac caa aaa gtt gtc gaa att gcg cca gca cag cat 768
   Leu Gln Arg Arg His Gln Lys Val Val Glu Ile Ala Pro Ala Gln His
   245 250 255
   ttg gat cca gaa ctg cgt gat cgc att tgt gcg gat gca gta aag ttc 816
   Leu Asp Pro Glu Leu Arg Asp Arg Ile Cys Ala Asp Ala Val Lys Phe
   260 265 270
   tgc cgc tcc att ggt tac cag ggc gcg gga act gtg gaa ttc ttg gtc 864
   Cys Arg Ser Ile Gly Tyr Gln Gly Ala Gly Thr Val Glu Phe Leu Val
   275 280 285
   gat gaa aag ggc aac cac gtt ttc atc gaa atg aac cca cgt atc cag 912
   Asp Glu Lys Gly Asn His Val Phe Ile Glu Met Asn Pro Arg Ile Gln
   290 295 300
   gtt gag cac acc gtg act gaa gaa gtc acc gag gtg gac ctg gtg aag 960
   Val Glu His Thr Val Thr Glu Glu Val Thr Glu Val Asp Leu Val Lys
   305 310 315 320
   gcg cag atg cgc ttg gct gct ggt gca acc ttg aag gaa ttg ggt ctg 1008
   Ala Gln Met Arg Leu Ala Ala Gly Ala Thr Leu Lys Glu Leu Gly Leu
   325 330 335
   acc caa gat aag atc aag acc cac ggt gcg gca ctg cag tgc cgc atc 1056
   Thr Gln Asp Lys Ile Lys Thr His Gly Ala Ala Leu Gln Cys Arg Ile
   340 345 350
   acc acg gaa gat cca aac aac ggc ttc cgc cca gat acc gga act atc 1104
   Thr Thr Glu Asp Pro Asn Asn Gly Phe Arg Pro Asp Thr Gly Thr Ile
   355 360 365
   acc gcg tac cgc tca cca ggc gga gct ggc gtt cgt ctt gac ggt gca      1152
   Thr Ala Tyr Arg Ser Pro Gly Gly Ala Gly Val Arg Leu Asp Gly Ala
       370                 375                 380
   gct cag ctc ggt ggc gaa atc acc gca cac ttt gac tcc atg ctg gtg      1200
   Ala Gln Leu Gly Gly Glu Ile Thr Ala His Phe Asp Ser Met Leu Val
   385                 390                 395                 400
   aaa atg acc tgc cgt ggt tcc gat ttt gaa act gct gtt gct cgt gca      1248
   Lys Met Thr Cys Arg Gly Ser Asp Phe Glu Thr Ala Val Ala Arg Ala
                   405                 410                 415
   cag cgc gcg ttg gct gag ttc acc gtg tct ggt gtt gca acc aac att      1296
   Gln Arg Ala Leu Ala Glu Phe Thr Val Ser Gly Val Ala Thr Asn Ile
               420                 425                 430
   ggt ttc ttg cgt gcg ttg ctg cgt gaa gag gac ttt act tcc aag cgc      1344
   Gly Phe Leu Arg Ala Leu Leu Arg Glu Glu Asp Phe Thr Ser Lys Arg
           435                 440                 445
   atc gcc acc gga ttt atc ggc gat cac cca cac ctc ctt cag gct cca      1392
   Ile Ala Thr Gly Phe Ile Gly Asp His Pro His Leu Leu Gln Ala Pro
       450                 455                 460
   cct gcg gat gat gag cag gga cgc atc ctg gat tac ttg gca gat gtc      1440
   Pro Ala Asp Asp Glu Gln Gly Arg Ile Leu Asp Tyr Leu Ala Asp Val
   465                 470                 475                 480
   acc gtg aac aag cct cat ggt gtg cgt cca aag gat gtt gca gca cca      1488
   Thr Val Asn Lys Pro His Gly Val Arg Pro Lys Asp Val Ala Ala Pro
                   485                 490                 495
   atc gat aag ctg ccc aac atc aag gat ctg cca ctg cca cgc ggt tcc      1536
   Ile Asp Lys Leu Pro Asn Ile Lys Asp Leu Pro Leu Pro Arg Gly Ser
               500                 505                 510
   cgt gac cgc ctg aag cag ctt gga cca gca gcg ttt gcc cgc gat ctc      1584
   Arg Asp Arg Leu Lys Gln Leu Gly Pro Ala Ala Phe Ala Arg Asp Leu
           515                 520                 525
   cgt gag cag gac gca ctg gca gtt act gat acc acc ttc cgc gat gca      1632
   Arg Glu Gln Asp Ala Leu Ala Val Thr Asp Thr Thr Phe Arg Asp Ala
       530                 535                 540
   cac cag tct ttg ctt gcg acc cga gtc cgc tca ttc gca ctg aag cct      1680
   His Gln Ser Leu Leu Ala Thr Arg Val Arg Ser Phe Ala Leu Lys Pro
   545                 550                 555                 560
   gcg gca gag gcc gtc gca aag ctg act cct gag ctt ttg tcc gtg gag      1728
   Ala Ala Glu Ala Val Ala Lys Leu Thr Pro Glu Leu Leu Ser Val Glu
                   565                 570                 575
   gcc tgg ggc ggt gcg acc tac gat gtg gcg atg cgt ttc ctc ttt gag      1776
   Ala Trp Gly Gly Ala Thr Tyr Asp Val Ala Met Arg Phe Leu Phe Glu
               580                 585                 590
   gat ccg tgg gac agg ctc gac gag ctg cgc gag gcg atg ccg aat gtg      1824
   Asp Pro Trp Asp Arg Leu Asp Glu Leu Arg Glu Ala Met Pro Asn Val
           595                 600                 605
   aac att cag atg ctg ctt cgc ggc cgc aac acc gtg gga tac acc cca      1872
   Asn Ile Gln Met Leu Leu Arg Gly Arg Asn Thr Val Gly Tyr Thr Pro
       610                 615                 620
   tac cca gac tcc gtc tgt cgc gcg ttt gtt aag gaa gct gcc acc tcc      1920
   Tyr Pro Asp Ser Val Cys Arg Ala Phe Val Lys Glu Ala Ala Thr Ser
   625                 630                 635                 640
   ggc gtg gac atc ttc cgc atc ttc gac gcg ctt aac gac gtc tcc cag      1968
   Gly Val Asp Ile Phe Arg Ile Phe Asp Ala Leu Asn Asp Val Ser Gln
                   645                 650                 655
   atg cgt cca gca atc gac gca gtc ctg gag acc aac acc gcg gtc gct      2016
   Met Arg Pro Ala Ile Asp Ala Val Leu Glu Thr Asn Thr Ala Val Ala
               660                 665                 670
   gaa gtg gct atg gct tat tct ggt gat ctt tcc gat ccg aat gaa aag      2064
   Glu Val Ala Met Ala Tyr Ser Gly Asp Leu Ser Asp Pro Asn Glu Lys
           675                 680                 685
   ctc tac acc ctg gat tac tac ctg aag atg gca gag gag atc gtc aag      2112
   Leu Tyr Thr Leu Asp Tyr Tyr Leu Lys Met Ala Glu Glu Ile Val Lys
       690                 695                 700
   tct ggc gct cac att ctg gct att aag gat atg gct ggt ctg ctt cgc      2160
   Ser Gly Ala His Ile Leu Ala Ile Lys Asp Met Ala Gly Leu Leu Arg
   705                 710                 715                 720
   cca gct gca gcc acc aag ctg gtc acc gca ctg cgc cgt gaa ttt gat      2208
   Pro Ala Ala Ala Thr Lys Leu Val Thr Ala Leu Arg Arg Glu Phe Asp
                   725                 730                 735
   ctg cca gtg cac gtg cac acc cac gac act gcg ggt ggc cag ctg gca      2256
   Leu Pro Val His Val His Thr His Asp Thr Ala Gly Gly Gln Leu Ala
               740                 745                 750
   acc tac ttt gct gca gct caa gct ggt gca gat gct gtt gac ggt gct      2304
   Thr Tyr Phe Ala Ala Ala Gln Ala Gly Ala Asp Ala Val Asp Gly Ala
           755                 760                 765
   tcc gca cca ctg tct ggc acc acc tcc cag cca tcc ctg tct gcc att      2352
   Ser Ala Pro Leu Ser Gly Thr Thr Ser Gln Pro Ser Leu Ser Ala Ile
       770                 775                 780
   gtt gct gca ttc gcg cac acc cgt cgc gat acc ggt ttg agc ctc gag      2400
   Val Ala Ala Phe Ala His Thr Arg Arg Asp Thr Gly Leu Ser Leu Glu
   785                 790                 795                 800
   gct gtt tct gac ctc gag cca tac tgg gaa gca gtg cgc gga ctg tac      2448
   Ala Val Ser Asp Leu Glu Pro Tyr Trp Glu Ala Val Arg Gly Leu Tyr
                   805                 810                 815
   ctg cca ttt gag tct gga acc cca ggc cca acc ggt cgc gtc tac cgc      2496
   Leu Pro Phe Glu Ser Gly Thr Pro Gly Pro Thr Gly Arg Val Tyr Arg
               820                 825                 830
   cac gaa atc cca ggc gga cag ctg tcc aac ctg cgt gca cag gcc acc      2544
   His Glu Ile Pro Gly Gly Gln Leu Ser Asn Leu Arg Ala Gln Ala Thr
           835                 840                 845
   gca ctg ggc ctt gcg gat cgt ttc gaa ctc atc gaa gac aac tac gcg      2592
   Ala Leu Gly Leu Ala Asp Arg Phe Glu Leu Ile Glu Asp Asn Tyr Ala
       850                 855                 860
   gca gtt aat gag atg ctg gga cgc cca acc aag gtc acc cca tcc tcc      2640
   Ala Val Asn Glu Met Leu Gly Arg Pro Thr Lys Val Thr Pro Ser Ser
   865                 870                 875                 880
   aag gtt gtt ggc gac ctc gca ctc cac ctc gtt ggt gcg ggt gtg gat      2688
   Lys Val Val Gly Asp Leu Ala Leu His Leu Val Gly Ala Gly Val Asp
                   885                 890                 895
   cca gca gac ttt gct gca gat cca caa aag tac gac atc cca gac tct      2736
   Pro Ala Asp Phe Ala Ala Asp Pro Gln Lys Tyr Asp Ile Pro Asp Ser
               900                 905                 910
   gtc atc gcg ttc ctg cgc ggc gag ctt ggt aac cct cca ggt ggc tgg      2784
   Val Ile Ala Phe Leu Arg Gly Glu Leu Gly Asn Pro Pro Gly Gly Trp
           915                 920                 925
   cca gag cca ctg cgc acc cgc gca ctg gaa ggc cgc tcc gaa ggc aag      2832
   Pro Glu Pro Leu Arg Thr Arg Ala Leu Glu Gly Arg Ser Glu Gly Lys
       930                 935                 940
   gca cct ctg acg gaa gtt cct gag gaa gag cag gcg cac ctc gac gct      2880
   Ala Pro Leu Thr Glu Val Pro Glu Glu Glu Gln Ala His Leu Asp Ala
   945                 950                 955                 960
   gat gat tcc aag gaa cgt cgc aac agc ctc aac cgc ctg ctg ttc ccg      2928
   Asp Asp Ser Lys Glu Arg Arg Asn Ser Leu Asn Arg Leu Leu Phe Pro
                   965                 970                 975
   aag cca acc gaa gag ttc ctc gag cac cgt cgc cgc ttc ggc aac acc      2976
   Lys Pro Thr Glu Glu Phe Leu Glu His Arg Arg Arg Phe Gly Asn Thr
               980                 985                 990
   tct gcg ctg gat gat cgt gaa ttc  ttc tac ggc ctg gtc  gaa ggc cgc    3024
   Ser Ala Leu Asp Asp Arg Glu Phe  Phe Tyr Gly Leu Val  Glu Gly Arg
           995                 1000                1005
   gag act  ttg atc cgc ctg cca  gat gtg cgc acc cca  ctg ctt gtt      3069
   Glu Thr  Leu Ile Arg Leu Pro  Asp Val Arg Thr Pro  Leu Leu Val
       1010                1015                1020
   cgc ctg  gat gcg atc tcc gag  cca gac gat aag ggt  atg cgc aat      3114
   Arg Leu  Asp Ala Ile Ser Glu  Pro Asp Asp Lys Gly  Met Arg Asn
       1025                1030                1035
   gtt gtg  gcc aac gtc aac ggc  cag atc cgc cca atg  cgt gtg cgt      3159
   Val Val  Ala Asn Val Asn Gly  Gln Ile Arg Pro Met  Arg Val Arg
       1040                    1045                    1050
   gac cgc  tcc gtt gag tct gtc  acc gca acc gca gaa  aag gca gat       3204
   Asp Arg  Ser Val Glu Ser Val  Thr Ala Thr Ala Glu  Lys Ala Asp
       1055                    1060                    1065
   tcc tcc  aac aag ggc cat gtt  gct gca cca ttc gct  ggt gtt gtc       3249
   Ser Ser  Asn Lys Gly His Val  Ala Ala Pro Phe Ala  Gly Val Val
       1070                    1075                    1080
   act gtg  act gtt gct gaa ggt  gat gag gtc aag gct  gga gat gca       3294
   Thr Val  Thr Val Ala Glu Gly  Asp Glu Val Lys Ala  Gly Asp Ala
       1085                    1090                    1095
   gtc gca  atc atc gag gct atg  aag atg gaa gca aca  atc act gct       3339
   Val Ala  Ile Ile Glu Ala Met  Lys Met Glu Ala Thr  Ile Thr Ala
       1100                    1105                    1110
   tct gtt  gac ggc aaa atc gat  cgc gtt gtg gtt cct  gct gca acg       3384
   Ser Val  Asp Gly Lys Ile Asp  Arg Val Val Val Pro  Ala Ala Thr
       1115                    1120                    1125
   aag gtg  gaa ggt ggc gac ttg  atc gtc gtc gtt tcc                    3420
   Lys Val  Glu Gly Gly Asp Leu  Ile Val Val Val Ser
       1130                    1135                    1140
<210> 23
   <211> 1140
   <212> PRT
   <213> Brevibacterium fluvum
<400> 23
   Met Ser Thr His Thr Ser Ser Thr Leu Pro Ala Phe Lys Lys Ile Leu
   1               5                   10                  15
   Val Ala Asn Arg Gly Glu Ile Ala Val Arg Ala Phe Arg Ala Ala Leu
               20                  25                  30
   Glu Thr Gly Ala Ala Thr Val Ala Ile Tyr Pro Arg Glu Asp Arg Gly
           35                  40                  45
   Ser Phe His Arg Ser Phe Ala Ser Glu Ala Val Arg Ile Gly Thr Glu
       50                  55                  60
   Gly Ser Pro Val Lys Ala Tyr Leu Asp Ile Asp Glu Ile Ile Gly Ala
   65                  70                  75                  80
   Ala Lys Lys Val Lys Ala Asp Ala Ile Tyr Pro Gly Tyr Gly Phe Leu
                   85                  90                  95
   Ser Glu Asn Ala Gln Leu Ala Arg Glu Cys Ala Glu Asn Gly Ile Thr
               100                 105                 110
   Phe Ile Gly Pro Thr Pro Glu Val Leu Asp Leu Thr Gly Asp Lys Ser
           115                 120                 125
   Arg Ala Val Thr Ala Ala Lys Lys Ala Gly Leu Pro Val Leu Ala Glu
       130                 135                 140
   Ser Thr Pro Ser Lys Asn Ile Asp Asp Ile Val Lys Ser Ala Glu Gly
   145                 150                 155                 160
   Gln Thr Tyr Pro Ile Phe Val Lys Ala Val Ala Gly Gly Gly Gly Arg
                   165                 170                 175
   Gly Met Arg Phe Val Ser Ser Pro Asp Glu Leu Arg Lys Leu Ala Thr
               180                 185                 190
   Glu Ala Ser Arg Glu Ala Glu Ala Ala Phe Gly Asp Gly Ser Val Tyr
           195                 200                 205
   Val Glu Arg Ala Val Ile Asn Pro Gln His Ile Glu Val Gln Ile Leu
       210                 215                 220
   Gly Asp Arg Thr Gly Glu Val Val His Leu Tyr Glu Arg Asp Cys Ser
   225                 230                 235                 240
   Leu Gln Arg Arg His Gln Lys Val Val Glu Ile Ala Pro Ala Gln His
                   245                 250                 255
   Leu Asp Pro Glu Leu Arg Asp Arg Ile Cys Ala Asp Ala Val Lys Phe
               260                 265                 270
   Cys Arg Ser Ile Gly Tyr Gln Gly Ala Gly Thr Val Glu Phe Leu Val
           275                 280                 285
   Asp Glu Lys Gly Asn His Val Phe Ile Glu Met Asn Pro Arg Ile Gln
       290                 295                 300
   Val Glu His Thr Val Thr Glu Glu Val Thr Glu Val Asp Leu Val Lys
   305                 310                 315                 320
   Ala Gln Met Arg Leu Ala Ala Gly Ala Thr Leu Lys Glu Leu Gly Leu
                   325                 330                 335
   Thr Gln Asp Lys Ile Lys Thr His Gly Ala Ala Leu Gln Cys Arg Ile
               340                 345                 350
   Thr Thr Glu Asp Pro Asn Asn Gly Phe Arg Pro Asp Thr Gly Thr Ile
           355                 360                 365
   Thr Ala Tyr Arg Ser Pro Gly Gly Ala Gly Val Arg Leu Asp Gly Ala
       370                 375                 380
   Ala Gln Leu Gly Gly Glu Ile Thr Ala His Phe Asp Ser Met Leu Val
   385                 390                 395                 400
   Lys Met Thr Cys Arg Gly Ser Asp Phe Glu Thr Ala Val Ala Arg Ala
                   405                 410                 415
   Gln Arg Ala Leu Ala Glu Phe Thr Val Ser Gly Val Ala Thr Asn Ile
               420                 425                 430
   Gly Phe Leu Arg Ala Leu Leu Arg Glu Glu Asp Phe Thr Ser Lys Arg
           435                 440                 445
   Ile Ala Thr Gly Phe Ile Gly Asp His Pro His Leu Leu Gln Ala Pro
       450                 455                 460
   Pro Ala Asp Asp Glu Gln Gly Arg Ile Leu Asp Tyr Leu Ala Asp Val
   465                 470                 475                 480
   Thr Val Asn Lys Pro His Gly Val Arg Pro Lys Asp Val Ala Ala Pro
                   485                 490                 495
   Ile Asp Lys Leu Pro Asn Ile Lys Asp Leu Pro Leu Pro Arg Gly Ser
               500                 505                 510
   Arg Asp Arg Leu Lys Gln Leu Gly Pro Ala Ala Phe Ala Arg Asp Leu
           515                 520                 525
   Arg Glu Gln Asp Ala Leu Ala Val Thr Asp Thr Thr Phe Arg Asp Ala
       530                 535                 540
   His Gln Ser Leu Leu Ala Thr Arg Val Arg Ser Phe Ala Leu Lys Pro
   545                 550                 555                 560
   Ala Ala Glu Ala Val Ala Lys Leu Thr Pro Glu Leu Leu Ser Val Glu
                   565                 570                 575
   Ala Trp Gly Gly Ala Thr Tyr Asp Val Ala Met Arg Phe Leu Phe Glu
               580                 585                 590
   Asp Pro Trp Asp Arg Leu Asp Glu Leu Arg Glu Ala Met Pro Asn Val
           595                 600                 605
   Asn Ile Gln Met Leu Leu Arg Gly Arg Asn Thr Val Gly Tyr Thr Pro
       610                 615                 620
   Tyr Pro Asp Ser Val Cys Arg Ala Phe Val Lys Glu Ala Ala Thr Ser
   625                 630                 635                 640
   Gly Val Asp Ile Phe Arg Ile Phe Asp Ala Leu Asn Asp Val Ser Gln
                   645                 650                 655
   Met Arg Pro Ala Ile Asp Ala Val Leu Glu Thr Asn Thr Ala Val Ala
               660                 665                 670
   Glu Val Ala Met Ala Tyr Ser Gly Asp Leu Ser Asp Pro Asn Glu Lys
           675                 680                 685
   Leu Tyr Thr Leu Asp Tyr Tyr Leu Lys Met Ala Glu Glu Ile Val Lys
       690                 695                 700
   Ser Gly Ala His Ile Leu Ala Ile Lys Asp Met Ala Gly Leu Leu Arg
   705                 710                 715                 720
   Pro Ala Ala Ala Thr Lys Leu Val Thr Ala Leu Arg Arg Glu Phe Asp
                   725                 730                 735
   Leu Pro Val His Val His Thr His Asp Thr Ala Gly Gly Gln Leu Ala
               740                 745                 750
   Thr Tyr Phe Ala Ala Ala Gln Ala Gly Ala Asp Ala Val Asp Gly Ala
           755                 760                 765
   Ser Ala Pro Leu Ser Gly Thr Thr Ser Gln Pro Ser Leu Ser Ala Ile
       770                 775                 780
   Val Ala Ala Phe Ala His Thr Arg Arg Asp Thr Gly Leu Ser Leu Glu
   785                 790                 795                 800
   Ala Val Ser Asp Leu Glu Pro Tyr Trp Glu Ala Val Arg Gly Leu Tyr
                   805                 810                 815
   Leu Pro Phe Glu Ser Gly Thr Pro Gly Pro Thr Gly Arg Val Tyr Arg
               820                 825                 830
   His Glu Ile Pro Gly Gly Gln Leu Ser Asn Leu Arg Ala Gln Ala Thr
           835                 840                 845
   Ala Leu Gly Leu Ala Asp Arg Phe Glu Leu Ile Glu Asp Asn Tyr Ala
       850                 855                 860
   Ala Val Asn Glu Met Leu Gly Arg Pro Thr Lys Val Thr Pro Ser Ser
   865                 870                 875                 880
   Lys Val Val Gly Asp Leu Ala Leu His Leu Val Gly Ala Gly Val Asp
                   885                 890                 895
   Pro Ala Asp Phe Ala Ala Asp Pro Gln Lys Tyr Asp Ile Pro Asp Ser
               900                 905                 910
   Val Ile Ala Phe Leu Arg Gly Glu Leu Gly Asn Pro Pro Gly Gly Trp
           915                 920                 925
   Pro Glu Pro Leu Arg Thr Arg Ala Leu Glu Gly Arg Ser Glu Gly Lys
       930                 935                 940
   Ala Pro Leu Thr Glu Val Pro Glu Glu Glu Gln Ala His Leu Asp Ala
   945                 950                 955                 960
   Asp Asp Ser Lys Glu Arg Arg Asn Ser Leu Asn Arg Leu Leu Phe Pro
                   965                 970                 975
   Lys Pro Thr Glu Glu Phe Leu Glu His Arg Arg Arg Phe Gly Asn Thr
               980                 985                 990
   Ser Ala Leu Asp Asp Arg Glu Phe  Phe Tyr Gly Leu Val  Glu Gly Arg
           995                 1000                1005
   Glu Thr  Leu Ile Arg Leu Pro  Asp Val Arg Thr Pro  Leu Leu Val
       1010                1015                1020
   Arg Leu  Asp Ala Ile Ser Glu  Pro Asp Asp Lys Gly  Met Arg Asn
       1025                1030                1035
   Val Val  Ala Asn Val Asn Gly  Gln Ile Arg Pro Met  Arg Val Arg
       1040                1045                1050
   Asp Arg  Ser Val Glu Ser Val  Thr Ala Thr Ala Glu  Lys Ala Asp
       1055                1060                1065
   Ser Ser  Asn Lys Gly His Val  Ala Ala Pro Phe Ala  Gly Val Val
       1070                1075                1080
   Thr Val  Thr Val Ala Glu Gly  Asp Glu Val Lys Ala  Gly Asp Ala
       1085                1090                1095
   Val Ala  Ile Ile Glu Ala Met  Lys Met Glu Ala Thr  Ile Thr Ala
       1100                1105                1110
   Ser Val  Asp Gly Lys Ile Asp  Arg Val Val Val Pro  Ala Ala Thr
       1115                1120                1125
   Lys Val  Glu Gly Gly Asp Leu  Ile Val Val Val Ser
       1130                1135                1140
<210> 24
   <211> 545
   <212> PRT
   <213> Artificial Sequence
<220>
   <223> consensus
<220>
   <221> misc_feature
   <222> (1)..(10)
   <223> Xaa can be any naturally occurring amino acid
<220>
   <221> misc_feature
   <222> (12)..(14)
   <223> Xaa can be any naturally occurring amino acid
<220>
   <221> misc_feature
   <222> (16)..(24)
   <223> Xaa can be any naturally occurring amino acid
<220>
   <221> misc_feature
   <222> (29)..(30)
   <223> Xaa can be any naturally occurring amino acid
<220>
   <221> misc_feature
   <222> (33)..(33)
   <223> Xaa can be any naturally occurring amino acid
<220>
   <221> misc_feature
   <222> (37)..(39)
   <223> Xaa can be any naturally occurring amino acid
<220>
   <221> misc_feature
   <222> (41)..(41)
   <223> Xaa can be any naturally occurring amino acid
<220>
   <221> misc_feature
   <222> (43)..(45)
   <223> Xaa can be any naturally occurring amino acid
<220>
   <221> misc_feature
   <222> (47)..(51)
   <223> Xaa can be any naturally occurring amino acid
<220>
   <221> misc_feature
   <222> (54)..(55)
   <223> Xaa can be any naturally occurring amino acid
<220>
   <221> misc_feature
   <222> (57)..(57)
   <223> Xaa can be any naturally occurring amino acid
<220>
   <221> misc_feature
   <222> (70)..(70)
   <223> Xaa can be any naturally occurring amino acid
<220>
   <221> misc_feature
   <222> (73)..(73)
   <223> Xaa can be any naturally occurring amino acid
<220>
   <221> misc_feature
   <222> (75)..(78)
   <223> Xaa can be any naturally occurring amino acid
<220>
   <221> misc_feature
   <222> (81)..(81)
   <223> Xaa can be any naturally occurring amino acid
<220>
   <221> misc_feature
   <222> (84)..(90)
   <223> Xaa can be any naturally occurring amino acid
<220>
   <221> misc_feature
   <222> (94)..(100)
   <223> Xaa can be any naturally occurring amino acid
<220>
   <221> misc_feature
   <222> (102)..(103)
   <223> Xaa can be any naturally occurring amino acid
<220>
   <221> misc_feature
   <222> (105)..(111)
   <223> Xaa can be any naturally occurring amino acid
<220>
   <221> misc_feature
   <222> (114)..(114)
   <223> Xaa can be any naturally occurring amino acid
<220>
   <221> misc_feature
   <222> (116)..(118)
   <223> Xaa can be any naturally occurring amino acid
<220>
   <221> misc_feature
   <222> (120)..(123)
   <223> Xaa can be any naturally occurring amino acid
<220>
   <221> misc_feature
   <222> (127)..(130)
   <223> Xaa can be any naturally occurring amino acid
<220>
   <221> misc_feature
   <222> (132)..(133)
   <223> Xaa can be any naturally occurring amino acid
<220>
   <221> misc_feature
   <222> (136)..(138)
   <223> Xaa can be any naturally occurring amino acid
<220>
   <221> misc_feature
   <222> (148)..(148)
   <223> Xaa can be any naturally occurring amino acid
<220>
   <221> misc_feature
   <222> (153)..(153)
   <223> Xaa can be any naturally occurring amino acid
<220>
   <221> misc_feature
   <222> (155)..(158)
   <223> Xaa can be any naturally occurring amino acid
<220>
   <221> misc_feature
   <222> (160)..(161)
   <223> Xaa can be any naturally occurring amino acid
<220>
   <221> misc_feature
   <222> (163)..(164)
   <223> xaa can be any naturally occurring amino acid
<220>
   <221> misc_feature
   <222> (167)..(167)
   <223> Xaa can be any naturally occurring amino acid
<220>
   <221> misc_feature
   <222> (169)..(169)
   <223> Xaa can be any naturally occurring amino acid
<220>
   <221> misc_feature
   <222> (171)..(172)
   <223> Xaa can be any naturally occurring amino acid
<220>
   <221> misc_feature
   <222> (174)..(179)
   <223> Xaa can be any naturally occurring amino acid
<220>
   <221> misc_feature
   <222> (182)..(182)
   <223> Xaa can be any naturally occurring amino acid
<220>
   <221> misc_feature
   <222> (185)..(185)
   <223> Xaa can be any naturally occurring amino acid
<220>
   <221> misc_feature
   <222> (188)..(191)
   <223> Xaa can be any naturally occurring amino acid
<220>
   <221> misc_feature
   <222> (194)..(194)
   <223> Xaa can be any naturally occurring amino acid
<220>
   <221> misc_feature
   <222> (196)..(198)
   <223> Xaa can be any naturally occurring amino acid
<220>
   <221> misc_feature
   <222> (200)..(200)
   <223> Xaa can be any naturally occurring amino acid
<220>
   <221> misc_feature
   <222> (202)..(203)
   <223> Xaa can be any naturally occurring amino acid
<220>
   <221> misc_feature
   <222> (216)..(216)
   <223> Xaa can be any naturally occurring amino acid
<220>
   <221> misc_feature
   <222> (221)..(221)
   <223> Xaa can be any naturally occurring amino acid
<220>
   <221> misc_feature
   <222> (225)..(225)
   <223> Xaa can be any naturally occurring amino acid
<220>
   <221> misc_feature
   <222> (227)..(229)
   <223> Xaa can be any naturally occurring amino acid
<220>
   <221> misc_feature
   <222> (236)..(239)
   <223> Xaa can be any naturally occurring amino acid
<220>
   <221> misc_feature
   <222> (241)..(243)
   <223> Xaa can be any naturally occurring amino acid
<220>
   <221> misc_feature
   <222> (245)..(245)
   <223> Xaa can be any naturally occurring amino acid
<220>
   <221> misc_feature
   <222> (264)..(264)
   <223> Xaa can be any naturally occurring amino acid
<220>
   <221> misc_feature
   <222> (275)..(276)
   <223> Xaa can be any naturally occurring amino acid
<220>
   <221> misc_feature
   <222> (278)..(278)
   <223> Xaa can be any naturally occurring amino acid
<220>
   <221> misc_feature
   <222> (281)..(281)
   <223> Xaa can be any naturally occurring amino acid
<220>
   <221> misc_feature
   <222> (289)..(289)
   <223> Xaa can be any naturally occurring amino acid
<220>
   <221> misc_feature
   <222> (291)..(291)
   <223> Xaa can be any naturally occurring amino acid
<220>
   <221> misc_feature
   <222> (293)..(294)
   <223> Xaa can be any naturally occurring amino acid
<220>
   <221> misc_feature
   <222> (296)..(296)
   <223> Xaa can be any naturally occurring amino acid
<220>
   <221> misc_feature
   <222> (302)..(302)
   <223> Xaa can be any naturally occurring amino acid
<220>
   <221> misc_feature
   <222> (305)..(305)
   <223> Xaa can be any naturally occurring amino acid
<220>
   <221> misc_feature
   <222> (307)..(307)
   <223> Xaa can be any naturally occurring amino acid
<220>
   <221> misc_feature
   <222> (314)..(318)
   <223> Xaa can be any naturally occurring amino acid
<220>
   <221> misc_feature
   <222> (320)..(321)
   <223> Xaa can be any naturally occurring amino acid
<220>
   <221> misc_feature
   <222> (323)..(324)
   <223> Xaa can be any naturally occurring amino acid
<220>
   <221> misc_feature
   <222> (326)..(328)
   <223> Xaa can be any naturally occurring amino acid
<220>
   <221> misc_feature
   <222> (339)..(339)
   <223> Xaa can be any naturally occurring amino acid
<220>
   <221> misc_feature
   <222> (342)..(344)
   <223> Xaa can be any naturally occurring amino acid
<220>
   <221> misc_feature
   <222> (346)..(352)
   <223> Xaa can be any naturally occurring amino acid
<220>
   <221> misc_feature
   <222> (354)..(357)
   <223> Xaa can be any naturally occurring amino acid
<220>
   <221> misc_feature
   <222> (359)..(360)
   <223> Xaa can be any naturally occurring amino acid
<220>
   <221> misc_feature
   <222> (365)..(365)
   <223> Xaa can be any naturally occurring amino acid
<220>
   <221> misc_feature
   <222> (377)..(377)
   <223> Xaa can be any naturally occurring amino acid
<220>
   <221> misc_feature
   <222> (380)..(381)
   <223> Xaa can be any naturally occurring amino acid
<220>
   <221> misc_feature
   <222> (384)..(384)
   <223> Xaa can be any naturally occurring amino acid
<220>
   <221> misc_feature
   <222> (386)..(386)
   <223> Xaa can be any naturally occurring amino acid
<220>
   <221> misc_feature
   <222> (402)..(402)
   <223> Xaa can be any naturally occurring amino acid
<220>
   <221> misc_feature
   <222> (415)..(415)
   <223> Xaa can be any naturally occurring amino acid
<220>
   <221> misc_feature
   <222> (419)..(419)
   <223> xaa can be any naturally occurring amino acid
<220>
   <221> misc_feature
   <222> (423)..(424)
   <223> Xaa can be any naturally occurring amino acid
<220>
   <221> misc_feature
   <222> (427)..(428)
   <223> Xaa can be any naturally occurring amino acid
<220>
   <221> misc_feature
   <222> (431)..(432)
   <223> Xaa can be any naturally occurring amino acid
<220>
   <221> misc_feature
   <222> (434)..(436)
   <223> Xaa can be any naturally occurring amino acid
<220>
   <221> misc_feature
   <222> (438)..(439)
   <223> Xaa can be any naturally occurring amino acid
<220>
   <221> misc_feature
   <222> (450)..(450)
   <223> Xaa can be any naturally occurring amino acid
<220>
   <221> misc_feature
   <222> (461)..(461)
   <223> Xaa can be any naturally occurring amino acid
<220>
   <221> misc_feature
   <222> (467)..(468)
   <223> Xaa can be any naturally occurring amino acid
<220>
   <221> misc_feature
   <222> (470)..(470)
   <223> Xaa can be any naturally occurring amino acid
<220>
   <221> misc_feature
   <222> (472)..(472)
   <223> Xaa can be any naturally occurring amino acid
<220>
   <221> misc_feature
   <222> (475)..(479)
   <223> Xaa can be any naturally occurring amino acid
<220>
   <221> misc_feature
   <222> (481)..(481)
   <223> Xaa can be any naturally occurring amino acid
<220>
   <221> misc_feature
   <222> (483)..(486)
   <223> Xaa can be any naturally occurring amino acid
<220>
   <221> misc_feature
   <222> (488)..(489)
   <223> Xaa can be any naturally occurring amino acid
<220>
   <221> misc_feature
   <222> (491)..(491)
   <223> xaa can be any naturally occurring amino acid
<220>
   <221> misc_feature
   <222> (494)..(496)
   <223> Xaa can be any naturally occurring amino acid
<220>
   <221> misc_feature
   <222> (501)..(501)
   <223> Xaa can be any naturally occurring amino acid
<220>
   <221> misc_feature
   <222> (505)..(509)
   <223> Xaa can be any naturally occurring amino acid
<220>
   <221> misc_feature
   <222> (511)..(512)
   <223> Xaa can be any naturally occurring amino acid
<220>
   <221> misc_feature
   <222> (515)..(515)
   <223> Xaa can be any naturally occurring amino acid
<220>
   <221> misc_feature
   <222> (519)..(519)
   <223> Xaa can be any naturally occurring amino acid
<220>
   <221> misc_feature
   <222> (522)..(523)
   <223> Xaa can be any naturally occurring amino acid
<220>
   <221> misc_feature
   <222> (526)..(526)
   <223> Xaa can be any naturally occurring amino acid
<220>
   <221> misc_feature
   <222> (529)..(537)
   <223> Xaa can be any naturally occurring amino acid
<220>
   <221> misc_feature
   <222> (539)..(540)
   <223> Xaa can be any naturally occurring amino acid
<220>
   <221> misc_feature
   <222> (544)..(545)
   <223> Xaa can be any naturally occurring amino acid
<400> 24
   Xaa Xaa Xaa Xaa Xaa Xaa Xaa Xaa Xaa Xaa Leu Xaa Xaa Xaa Gly Xaa
   1               5                   10                  15
   Xaa Xaa Xaa Xaa Xaa Xaa Xaa Xaa Asn Pro Ser Tyr Xaa Xaa Leu Glu
               20                  25                  30
   Xaa Glu Glu Thr Xaa Xaa Xaa Leu Xaa Gly Xaa Xaa Xaa Gly Xaa Xaa
           35                  40                  45
   Xaa Xaa Xaa Gly Ala Xaa Xaa Val Xaa Thr Gly Ile Phe Thr Gly Arg
       50                  55                  60
   Ser Pro Lys Asp Lys Xaa Ile Val Xaa Asp Xaa Xaa Xaa Xaa Asp Thr
   65                  70                  75                  80
   Xaa Trp Trp Xaa Xaa Xaa Xaa Xaa Xaa Xaa Asn Asp Asn Xaa Xaa Xaa
                   85                  90                  95
   Xaa Xaa Xaa Xaa Trp Xaa Xaa Leu Xaa Xaa Xaa Xaa Xaa Xaa Xaa Leu
               100                 105                 110
   Ser Xaa Lys Xaa Xaa Xaa Val Xaa Xaa Xaa Xaa Cys Gly Ala Xaa Xaa
           115                 120                 125
   Xaa Xaa Arg Xaa Xaa Val Arg Xaa Xaa Xaa Glu Val Ala Trp Gln Ala
       130                 135                 140
   His Phe Val Xaa Asn Met Phe Ile Xaa Pro Xaa Xaa Xaa Xaa Leu Xaa
   145                 150                 155                 160
   Xaa Phe Xaa Xaa Asp Phe Xaa Val Xaa Asn Xaa Xaa Lys Xaa Xaa Xaa
                   165                 170                 175
   Xaa Xaa Xaa Lys Glu Xaa Gly Leu Xaa Ser Glu Xaa Xaa Xaa Xaa Phe
               180                 185                 190
   Asn Xaa Thr Xaa Xaa Xaa Gln Xaa Ile Xaa Xaa Thr Trp Tyr Gly Gly
           195                 200                 205
   Glu Met Lys Lys Gly Met Phe Xaa Met Met Asn Tyr Xaa Leu Pro Leu
       210                 215                 220
   Xaa Gly Xaa Xaa Xaa Met His Cys Ser Ala Asn Xaa Xaa Xaa Xaa Gly
   225                 230                 235                 240
   Xaa Xaa Xaa Ala Xaa Phe Phe Gly Leu Ser Gly Thr Gly Lys Thr Thr
                   245                 250                 255
   Leu Ser Thr Asp Pro Lys Arg Xaa Leu Ile Gly Asp Asp Glu His Gly
               260                 265                 270
   Trp Asp Xaa Xaa Gly Xaa Phe Asn Xaa Glu Gly Gly Cys Tyr Ala Lys
           275                 280                 285
   Xaa Ile Xaa Leu Xaa Xaa Glu Xaa Glu Pro Asp Ile Tyr Xaa Ala Ile
       290                 295                 300
   Xaa Arg Xaa Ala Leu Leu Glu Asn Val Xaa Xaa Xaa Xaa Xaa Gly Xaa
   305                 310                 315                 320
   Xaa Asp Xaa Xaa Asp Xaa Xaa Xaa Thr Glu Asn Thr Arg Val Ser Tyr
                   325                 330                 335
   Pro Ile Xaa His Ile Xaa Xaa Xaa Val Xaa Xaa Xaa Xaa Xaa Xaa Xaa
               340                 345                 350
   Ser Xaa Xaa Xaa Xaa Ala Xaa Xaa Val Ile Phe Leu Xaa Ala Asp Ala
           355                 360                 365
   Phe Gly Val Leu Pro Pro Val Ser Xaa Leu Thr Xaa Xaa Gln Thr Xaa
       370                 375                 380
   Tyr Xaa Phe Leu Ser Gly Phe Thr Ala Lys Leu Ala Gly Thr Glu Arg
   385                 390                 395                 400
   Gly Xaa Thr Glu Pro Thr Pro Thr Phe Ser Ala Cys Phe Gly Xaa Ala
                   405                 410                 415
   Phe Leu Xaa Leu His Pro Xaa Xaa Tyr Ala Xaa Xaa Leu Val Xaa Xaa
               420                 425                 430
   Met Xaa Xaa Xaa Gly Xaa Xaa Ala Tyr Leu Val Asn Thr Gly Trp Asn
           435                 440                 445
   Gly Xaa Gly Lys Arg Ile Ser Ile Lys Asp Thr Arg Xaa Ile Ile Asp
       450                 455                 460
   Ala Ile Xaa Xaa Gly Xaa Ile Xaa Lys Ala Xaa Xaa Xaa Xaa Xaa Pro
   465                 470                 475                 480
   Xaa Phe Xaa Xaa Xaa Xaa Pro Xaa Xaa Leu Xaa Gly Val Xaa Xaa Xaa
                   485                 490                 495
   Ile Leu Asp Pro Xaa Asp Thr Tyr Xaa Xaa Xaa Xaa Xaa Trp Xaa Xaa
               500                 505                 510
   Lys Ala Xaa Asp Leu Ala Xaa Arg Phe Xaa Xaa Asn Phe Xaa Lys Tyr
           515                 520                 525
   Xaa Xaa Xaa Xaa Xaa Xaa Xaa Xaa Xaa Leu Xaa Xaa Ala Gly Pro Xaa
       530                 535                 540
   Xaa
   545
<210> 25
   <211> 33
   <212> DNA
   <213> Artificial Sequence
<220>
   <223> primer
<400> 25
   tagcgagatc tctgatgtcc ggcggtgctt ttg 33
<210> 26
   <211> 32
   <212> DNA
   <213> Artificial Sequence
<220>
   <223> primer
<400> 26
   aaaaagagct cttacgcccc gccctgccac tc 32
<210> 27
   <211> 34
   <212> DNA
   <213> Artificial Sequence
<220>
   <223> primer
<400> 27
   caggatctag aaggagacat gaacgatgaa catc 34
<210> 28
   <211> 36
   <212> DNA
   <213> Artificial Sequence
<220>
   <223> primer
<400> 28
   gataaggatc cgaaataaaa gaaaatgcca atagga 36
<210> 29
   <211> 31
   <212> DNA
   <213> Artificial Sequence
<220>
   <223> primer
<400> 29
   cctttgagct cgcgggcagt gagcgcaacg c 31
<210> 30
   <211> 48
   <212> DNA
   <213> Artificial sequence
<220>
   <223> primer
<400> 30
   ctagagcggc cgccgatcgg gatcctcctg tgtgaaattg ttatccgc 48
<210> 31
   <211> 42
   <212> DNA
   <213> Artificial Sequence
<220>
   <223> primer
<400> 31
   ctctacgatc gaggaggtta taaaaaatgg atattaatac tg 42
<210> 32
   <211> 36
   <212> DNA
   <213> Artificial Sequence
<220>
   <223> primer
<400> 32
   tcaaagcggc cgcttcttcg tctgtttcta ctggta 36
<210> 33
   <211> 31
   <212> DNA
   <213> Artificial Sequence
<220>
   <223> primer
<400> 33
   cctttggtac cgcgggcagt gagcgcaacg c 31
<210> 34
   <211> 34
   <212> DNA
   <213> Artificial Sequence
<220>
   <223> primer
<400> 34
   aacaggaatt ctttgcctgg cggcagtagc gcgg 34
<210> 35
   <211> 40
   <212> DNA
   <213> Artificial Sequence
<220>
   <223> primer P1
<400> 35
   ctagtaagat cttgaagcct gcttttttat actaagttgg 40
<210> 36
   <211> 41
   <212> DNA
   <213> Artificial Sequence
<220>
   <223> primer P2
<400> 36
   atgatcgaat tcgaaatcaa ataatgattt tattttgact g 41
<210> 37
   <211> 120
   <212> DNA
   <213> Artificial sequence
<220>
   <223> DNA fragment containing attL
<400> 37
   agatcttgaa gcctgctttt ttatactaag ttggcattat aaaaaagcat tgcttatcaa 60
   tttgttgcaa cgaacaggtc actatcagtc aaaataaaat cattatttga tttcgaattc 120
<210> 38
   <211> 41
   <212> DNA
   <213> Artificial sequence
<220>
   <223> primer P3
<400> 38
   atgccactgc agtctgttac aggtcactaa taccatctaa g 41
<210> 39
   <211> 46
   <212> DNA
   <213> Artificial Sequence
<220>
   <223> primer P4
<400> 39
   accgttaagc tttctagacg ctcaagttag tataaaaaag ctgaac 46
<210> 40
   <211> 184
   <212> DNA
   <213> Artificial Sequence
<220>
   <223> DNA fragment containing attR
<400> 40
   ctgcagtctg ttacaggtca ctaataccat ctaagtagtt gattcatagt gactgcatat 60
   gttgtgtttt acagtattat gtagtctgtt ttttatgcaa aatctaattt aatatattga 120
   tatttatatc attttacgtt tctcgttcag cttttttata ctaacttgag cgtctagaaa 180
   gctt 184
<210> 41
   <211> 38
   <212> DNA
   <213> Artificial Sequence
<220>
   <223> primer P5
<400> 41
   ttcttagacg tcaggtggca cttttcgggg aaatgtgc 38
<210> 42
   <211> 37
   <212> DNA
   <213> Artificial sequence
<220>
   <223> primer P6
<400> 42
   taacagagat ctcgcgcaga aaaaaaggat ctcaaga 37
<210> 43
   <211> 46
   <212> DNA
   <213> Artificial Sequence
<220>
   <223> primer P7
<400> 43
   aacagagatc taagcttaga tcctttgcct ggcggcagta gcgcgg 46
<210> 44
   <211> 35
   <212> DNA
   <213> Artificial sequence
<220>
   <223> primer P8
<400> 44
   ataaactgca gcaaaaagag tttgtagaaa cgcaa 35
<210> 45
   <211> 1388
   <212> DNA
   <213> Artificial Sequence
<220>
   <223> DNA fragment containing Tc gene and ter_thrL
<400> 45
   gaattctcat gtttgacagc ttatcatcga taagctttaa tgcggtagtt tatcacagtt 60
   aaattgctaa cgcagtcagg caccgtgtat gaaatctaac aatgcgctca tcgtcatcct 120
   cggcaccgtc accctggatg ctgtaggcat aggcttggtt atgccggtac tgccgggcct 180
   cttgcgggat atcgtccatt ccgacagcat cgccagtcac tatggcgtgc tgctagcgct 240
   atatgcgttg atgcaatttc tatgcgcacc cgttctcgga gcactgtccg accgctttgg 300
   ccgccgccca gtcctgctcg cttcgctact tggagccact atcgactacg cgatcatggc 360
   gaccacaccc gtcctgtgga tcctctacgc cggacgcatc gtggccggca tcaccggcgc 420
   cacaggtgcg gttgctggcg cctatatcgc cgacatcacc gatggggaag atcgggctcg 480
   ccacttcggg ctcatgagcg cttgtttcgg cgtgggtatg gtggcaggcc ccgtggccgg 540
   gggactgttg ggcgccatct ccttgcatgc accattcctt gcggcggcgg tgctcaacgg 600
   cctcaaccta ctactgggct gcttcctaat gcaggagtcg cataagggag agcgtcgacc 660
   gatgcccttg agagccttca acccagtcag ctccttccgg tgggcgcggg gcatgactat 720
   cgtcgccgca cttatgactg tcttctttat catgcaactc gtaggacagg tgccggcagc 780
   gctctgggtc attttcggcg aggaccgctt tcgctggagc gcgacgatga tcggcctgtc 840
   gcttgcggta ttcggaatct tgcacgccct cgctcaagcc ttcgtcactg gtcccgccac 900
   caaacgtttc ggcgagaagc aggccattat cgccggcatg gcggccgacg cgctgggcta 960
   cgtcttgctg gcgttcgcga cgcgaggctg gatggccttc cccattatga ttcttctcgc 1020
   ttccggcggc atcgggatgc ccgcgttgca ggccatgctg tccaggcagg tagatgacga 1080
   ccatcaggga cagcttcaag gatcgctcgc ggctcttacc agcctaactt cgatcactgg 1140
   accgctgatc gtcacggcga tttatgccgc ctcggcgagc acatggaacg ggttggcatg 1200
   gattgtaggc gccgccctat accttgtctg cctccccgcg ttgcgtcgcg gtgcatggag 1260
   ccgggccacc tcgacctgaa tggaagccgg cggcacctcg ctaacggatt caccactcca 1320
   actagaaagc ttaacacaga aaaaagcccg cacctgacag tgcgggcttt ttttttcgac 1380
   cactgcag 1388
<210> 46
   <211> 36
   <212> DNA
   <213> Artificial sequence
<220>
   <223> primer P9
<400> 46
   agtaattcta gaaagcttaa cacagaaaaa agcccg 36
<210> 47
   <211> 43
   <212> DNA
   <213> Artificial sequence
<220>
   <223> primer P10
<400> 47
   ctagtaggat ccctgcagtg gtcgaaaaaa aaagcccgca ctg 43
<210> 48
   <211> 329
   <212> PRT
   <213> Enterobacter aerogenes
<400> 48
   Met Lys Ile Ala Val Tyr Ser Thr Lys Gln Tyr Asp Lys Lys Tyr Leu
   1 5 10 15
   Gln His Val Asn Asp Ala Tyr Gly Phe Glu Leu Glu Phe Phe Asp Phe
   20 25 30
   Leu Leu Thr Glu Lys Thr Ala Lys Thr Ala Asn Gly Cys Glu Ala Val
   35 40 45
   Cys Ile Phe Val Asn Asp Asp Gly Ser Arg Pro Val Leu Glu Glu Leu
   50 55 60
   Lys Ala His Gly Val Lys Tyr Ile Ala Leu Arg Cys Ala Gly Phe Asn
   65 70 75 80
   Asn Val Asp Leu Asp Ala Ala Lys Glu Leu Gly Leu Arg Val Val Arg
   85 90 95
   Val Pro Ala Tyr Ser Pro Glu Ala Val Ala Glu His Ala Ile Gly Met
   100 105 110
   Met Met Ser Leu Asn Arg Arg Ile His Arg Ala Tyr Gln Arg Thr Arg
   115 120 125
   Asp Ala Asn Phe Ser Leu Glu Gly Leu Thr Gly Phe Thr Met Tyr Gly
   130 135 140
   Lys Thr Ala Gly Val Ile Gly Thr Gly Lys Ile Gly Val Ala Thr Leu
   145 150 155 160
   Arg Ile Leu Lys Gly Phe Gly Met Arg Leu Leu Ala Phe Asp Pro Tyr
   165 170 175
   Pro Ser Ala Ala Ala Leu Asp Leu Gly Val Glu Tyr Val Asp Leu Pro
   180 185 190
   Thr Leu Tyr Ala Gln Ser Asp Val Ile Ser Leu His Cys Pro Leu Thr
   195 200 205
   Asn Glu Asn Tyr His Leu Leu Asn Gln Ala Ala Phe Asp Gln Met Lys
   210 215 220
   Asp Gly Val Met Val Ile Asn Thr Ser Arg Gly Ala Leu Ile Asp Ser
   225 230 235 240
   Gln Ala Ala Ile Asp Ala Leu Lys His Gln Lys Ile Gly Ala Leu Gly
   245 250 255
   Met Asp Val Tyr Glu Asn Glu Arg Asp Leu Phe Phe Glu Asp Lys Ser
   260 265 270
   Asn Asp Val Ile Gln Asp Asp Val Phe Arg Arg Leu Ser Ala Cys His
   275 280 285
   Asn Val Leu Phe Thr Gly His Gln Ala Phe Leu Thr Ala Glu Ala Leu
   290 295 300
   Ile Gly Ile Ser Glu Thr Thr Leu Gly Asn Leu Gln Gln Val Ala Lys
   305 310 315 320
   Gly Glu Thr Cys Pro Asn Ala Leu Val
   325
<210> 49
   <211> 915
   <212> PRT
   <213> Enterobacter aerogenes
<400> 49
   Met Ala Val Thr Asn Val Ala Glu Leu Asn Ala Leu Val Glu Arg Val
   1               5                   10                  15
   Lys Lys Ala Gln Arg Glu Tyr Ala Ser Phe Thr Gln Glu Gln Val Asp
               20                  25                  30
   Lys Ile Phe Arg Ala Ala Ala Leu Ala Ala Ala Asp Ala Arg Ile Pro
           35                  40                  45
   Leu Ala Lys Met Ala Val Ala Glu Ser Gly Met Gly Ile Ile Glu Asp
       50                  55                  60
   Lys Val Ile Lys Asn His Phe Ala Ser Glu Tyr Ile Tyr Asn Ala Tyr
   65                  70                  75                  80
   Lys Asp Glu Lys Thr Cys Gly Val Leu Ser Glu Asp Asp Thr Phe Gly
                   85                  90                  95
   Thr Ile Thr Ile Ala Glu Pro Ile Gly Ile Ile Cys Gly Ile Val Pro
               100                 105                 110
   Thr Thr Asn Pro Thr Ser Thr Ala Ile Phe Lys Ser Leu Ile Ser Leu
           115                 120                 125
   Lys Thr Arg Asn Ala Ile Ile Phe Ser Pro His Pro Arg Ala Lys Asp
       130                 135                 140
   Ala Thr Asn Lys Ala Ala Asp Ile Val Leu Gln Ala Ala Ile Ala Ala
   145                 150                 155                 160
   Gly Ala Pro Lys Asp Leu Ile Gly Trp Ile Asp Gln Pro Ser Val Glu
                   165                 170                 175
   Leu Ser Asn Ala Leu Met His His Pro Asp Ile Asn Leu Ile Leu Ala
               180                 185                 190
   Thr Gly Gly Pro Gly Met Val Lys Ala Ala Tyr Ser Ser Gly Lys Pro
           195                 200                 205
   Ala Ile Gly Val Gly Ala Gly Asn Thr Pro Val Val Ile Asp Glu Thr
       210                 215                 220
   Ala Asp Ile Lys Arg Ala Val Ala Ser Val Leu Met Ser Lys Thr Phe
   225                 230                 235                 240
   Asp Asn Gly Val Ile Cys Ala Ser Glu Gln Ser Val Val Val Val Asp
                   245                     250                     255
   Ser Val Tyr Asp Ala Val Arg Glu Arg Phe Ala Ser His Gly Gly Tyr
               260                     265                     270
   Leu Leu Gln Gly Lys Glu Leu Lys Ala Val Gln Asp Ile Ile Leu Lys
           275                     280                     285
   Asn Gly Ala Leu Asn Ala Ala Ile Val Gly Gln Pro Ala Ala Lys Ile
       290                     295                     300
   Ala Glu Leu Ala Gly Phe Thr Val Pro Ala Thr Thr Lys Ile Leu Ile
   305                     310                     315                     320
   Gly Glu Val Thr Asp Val Asp Glu Ser Glu Pro Phe Ala His Glu Lys
                   325                     330                     335
   Leu Ser Pro Thr Leu Ala Met Tyr Arg Ala Lys Asp Phe Glu Asp Ala
               340                     345                     350
   Val Asn Lys Ala Glu Lys Leu Val Ala Met Gly Gly Ile Gly His Thr
           355                     360                     365
   Ser Cys Leu Tyr Thr Asp Gln Asp Asn Gln Pro Ala Arg Val Ala Tyr
       370                     375                     380
   Phe Gly Gln Met Met Lys Thr Ala Arg Ile Leu Ile Asn Thr Pro Ala
   385                     390                     395                     400
   Ser Gln Gly Gly Ile Gly Asp Leu Tyr Asn Phe Lys Leu Ala Pro Ser
                   405                     410                     415
   Leu Thr Leu Gly Cys Gly Ser Trp Gly Gly Asn Ser Ile Ser Glu Asn
               420                     425                     430
   Val Gly Pro Lys His Leu Ile Asn Lys Lys Thr Val Ala Lys Arg Ala
           435                     440                     445
   Glu Asn Met Leu Trp His Lys Leu Pro Lys Ser Ile Tyr Phe Arg Arg
       450                     455                     460
   Gly Ser Leu Pro Ile Ala Leu Asp Glu Val Ile Thr Asp Gly His Lys
   465                     470                     475                     480
   Arg Ala Leu Ile Val Thr Asp Arg Phe Leu Phe Asn Asn Gly Tyr Ala
                   485                     490                     495
   Asp Gln Ile Thr Ser Val Leu Lys Ala Ala Gly Val Glu Thr Glu Val
               500                     505                     510
   Phe Phe Glu Val Glu Ala Asp Pro Thr Leu Thr Ile Val Arg Lys Gly
           515                     520                     525
   Ala Asp Leu Ala Asn Ser Phe Lys Pro Asp Val Ile Ile Ala Leu Gly
       530                     535                     540
   Gly Gly Ser Pro Met Asp Ala Ala Lys Ile Met Trp Val Met Tyr Glu
   545                     550                     555                     560
   His Pro Glu Thr His Phe Glu Glu Leu Ala Leu Arg Phe Met Asp Ile
                   565                     570                     575
   Arg Lys Arg Ile Tyr Lys Phe Pro Lys Met Gly Val Lys Ala Lys Met
               580                     585                     590
   Val Ala Ile Thr Thr Thr Ser Gly Thr Gly Ser Glu Val Thr Pro Phe
           595                     600                     605
   Ala Val Val Thr Asp Asp Ala Thr Gly Gln Lys Tyr Pro Leu Ala Asp
       610                     615                     620
   Tyr Ala Leu Thr Pro Asp Met Ala Ile Val Asp Ala Asn Leu Val Met
   625                     630                     635                     640
   Asp Met Pro Lys Ser Leu Cys Ala Phe Gly Gly Leu Asp Ala Val Thr
                   645                     650                     655
   His Ala Leu Glu Ala Tyr Val Ser Val Leu Ala Ser Glu Phe Ser Asp
               660                     665                     670
   Gly Gln Ala Leu Gln Ala Leu Lys Leu Leu Lys Glu Tyr Leu Pro Ala
           675                     680                     685
   Ser Tyr His Glu Gly Ser Lys Asn Pro Val Ala Arg Glu Arg Val His
       690                     695                     700
   Ser Ala Ala Thr Ile Ala Gly Ile Ala Phe Ala Asn Ala Phe Leu Gly
   705                     710                     715                     720
   Val Cys His Ser Met Ala His Lys Leu Gly Ser Gln Phe His Ile Pro
                   725                     730                     735
   His Gly Leu Ala Asn Ala Leu Leu Ile Ser Asn Val Ile Arg Tyr Asn
               740                     745                     750
   Ala Asn Asp Asn Pro Thr Lys Gln Thr Ala Phe Ser Gln Tyr Asp Arg
                   755                     760                     765
   Pro Gln Ala Arg Arg Arg Tyr Ala Glu Ile Ala Asp His Leu Gly Leu
           770                     775                     780
   Ser Ala Pro Gly Asp Arg Thr Ala Ala Lys Ile Glu Lys Leu Leu Ala
   785                     790                     795                     800
   Trp Leu Glu Ser Leu Lys Ala Glu Leu Gly Ile Pro Lys Ser Ile Arg
                   805                     810                     815
   Glu Ala Gly Val Gln Glu Ala Asp Phe Leu Ala His Val Asp Lys Leu
               820                     825                     830
   Ser Glu Asp Ala Phe Asp Asp Gln Cys Thr Gly Ala Asn Pro Arg Tyr
           835                     840                     845
   Pro Leu Ile Ala Glu Leu Lys Gln Ile Leu Leu Asp Thr Tyr Tyr Gly
       850                     855                     860
   Arg Asp Phe Val Glu Gly Glu Ala Ala Ala Val Lys Lys Glu Ala Ala
   865                     870                     875                     880
   Pro Ala Lys Ala Glu Lys Lys Asp Glu Lys Ile Arg Leu Ile Arg Arg
                   885                     890                     895
   Ile Glu Met Asn Glu Ala Pro Ser Ile Asp Gly Ala Phe Phe Ile Phe
               900                     905                     910
   Val Leu Tyr
           915
<210> 50
   <211> 2688
   <212> DNA
   <213> Enterobacter aerogenes
<220>
   <221> CDS
   <222> (301)..(2448)
<400> 50
   tcgtgaactg tccctgggta aactgggcgt tctgggcttc gaagttgatc acgagcgtaa      60
   cctggctgcc cgtttcggca agtctggctt catcaacaaa gaaggcaccc gccctgccgt     120
   ggttatcccg accaacgaag agctggtgat cgctcaagac gcacaccgtc tgaccgctta     180
   attccacacc gccagcgata atcctgactg cttcgagttg ccacttgaag tatgaacggt     240
   aagctggcgg tgctgttttg tatcccgcct aaaactggcg gtaacgaaag aggatatatc     300
   gtg tcc cgt act atc atg ctg atc cct acc gga acc agc gta ggc ctg       348
   Val Ser Arg Thr Ile Met Leu Ile Pro Thr Gly Thr Ser Val Gly Leu
   1               5                   10                  15
   acc agc gtc agc ctc ggt gtt atc cgc gct atg gaa cgc aaa ggc gtt       396
   Thr Ser Val Ser Leu Gly Val Ile Arg Ala Met Glu Arg Lys Gly Val
               20                  25                  30
   cgc ctg agc gtc ttt aaa cct atc gcc cag ccg cgt tcc ggt ggc gat       444
   Arg Leu Ser Val Phe Lys Pro Ile Ala Gln Pro Arg Ser Gly Gly Asp
           35                  40                  45
   act cca gac cag act acc acc atc gtt cgc gcc agc tct tca acg acg       492
   Thr Pro Asp Gln Thr Thr Thr Ile Val Arg Ala Ser Ser Ser Thr Thr
       50                  55                  60
   act gcc gct gaa ccg atg aac atg agc cac gtt gag tcg ctg ctg tcc       540
   Thr Ala Ala Glu Pro Met Asn Met Ser His Val Glu Ser Leu Leu Ser
   65                  70                  75                  80
   agc aac cag aaa gac gtg ctg atg gaa gag atc atc gcc aac tac cat       588
   Ser Asn Gln Lys Asp Val Leu Met Glu Glu Ile Ile Ala Asn Tyr His
                   85                  90                  95
   gcc aac act cag gat gct gaa gtg gtg ctg gtt gaa ggc ctg gtc ccg       636
   Ala Asn Thr Gln Asp Ala Glu Val Val Leu Val Glu Gly Leu Val Pro
               100                 105                 110
   act cgt aaa cac cag ttc gct cag tct ctg aac tac gaa atc gca aaa       684
   Thr Arg Lys His Gln Phe Ala Gln Ser Leu Asn Tyr Glu Ile Ala Lys
           115                 120                 125
   acg ctt aac gcg gaa atc gtc ttc gtg atg tct cag ggc acc gat acc       732
   Thr Leu Asn Ala Glu Ile Val Phe Val Met Ser Gln Gly Thr Asp Thr
       130                     135                     140
   ccg gaa cag ctg aac gag cgt atc gaa ctg act cgc aac agc ttc ggc      780
   Pro Glu Gln Leu Asn Glu Arg Ile Glu Leu Thr Arg Asn Ser Phe Gly
   145                 150                     155                 160
   ggc gcg aaa aac acc agc atc act ggc gtt atc gtt aac aaa ctg aat      828
   Gly Ala Lys Asn Thr Ser Ile Thr Gly Val Ile Val Asn Lys Leu Asn
                   165                     170                 175
   gct ccg gtt gat gaa caa ggc cgt aca cgc cct gac ctg tcc gaa atc      876
   Ala Pro Val Asp Glu Gln Gly Arg Thr Arg Pro Asp Leu Ser Glu Ile
               180                     185                 190
   ttt gac gac tcg tct aaa gcg aaa gtc gtg aag atc gac ccg gct caa      924
   Phe Asp Asp Ser Ser Lys Ala Lys Val Val Lys Ile Asp Pro Ala Gln
           195                     200                 205
   ctg cag aat ggc agc ccg ctg ccg gtg ctg ggc gcg gtg cca tgg agc      972
   Leu Gln Asn Gly Ser Pro Leu Pro Val Leu Gly Ala Val Pro Trp Ser
       210                     215                 220
   ttc gat ctg atc gcc acg cgc gct atc gac atg gcg cat cac ctg aat     1020
   Phe Asp Leu Ile Ala Thr Arg Ala Ile Asp Met Ala His His Leu Asn
   225                 230                     235                 240
   gcg acc gtg atc aac gaa ggc gac atc aat act cgc cgc gtg aag tct     1068
   Ala Thr Val Ile Asn Glu Gly Asp Ile Asn Thr Arg Arg Val Lys Ser
                   245                     250                 255
   gta acc ttc tgc gcg cgc agc att ccg cac atg ctg gaa cac ttc cgt     1116
   Val Thr Phe Cys Ala Arg Ser Ile Pro His Met Leu Glu His Phe Arg
               260                     265                 270
   ccg ggt tct ctg ctg gtg act tcc gca gac cgt cca gac gta ctg gtc     1164
   Pro Gly Ser Leu Leu Val Thr Ser Ala Asp Arg Pro Asp Val Leu Val
           275                     280                 285
   gct gcc tgc ctg gcg gcg atg aac ggc gta gaa atc ggc gct atc ctg     1212
   Ala Ala Cys Leu Ala Ala Met Asn Gly Val Glu Ile Gly Ala Ile Leu
       290                     295                 300
   ctg acc ggc ggc tat gaa atg gac gcg cgc atc agc aag ctg tgc gaa     1260
   Leu Thr Gly Gly Tyr Glu Met Asp Ala Arg Ile Ser Lys Leu Cys Glu
   305                 310                     315                 320
   cgt gct ttc gcc acc ggc ctg ccg gta ttc atg gtt aac acc aac acc     1308
   Arg Ala Phe Ala Thr Gly Leu Pro Val Phe Met Val Asn Thr Asn Thr
                   325                     330                 335
   tgg cag acc tct ctg agc ctg cag agc ttc aac ctg gaa gta ccg gtt     1356
   Trp Gln Thr Ser Leu Ser Leu Gln Ser Phe Asn Leu Glu Val Pro Val
               340                     345                 350
   gac gat cac gag cgc atc gag aaa gtt cag gaa tac gtt gct aac tat     1404
   Asp Asp His Glu Arg Ile Glu Lys Val Gln Glu Tyr Val Ala Asn Tyr
           355                     360                 365
   atc aac gcc gac tgg atc gaa tcg ctg acc gcg acc tcc gag cgc agc     1452
   Ile Asn Ala Asp Trp Ile Glu Ser Leu Thr Ala Thr Ser Glu Arg Ser
       370                     375                 380
   cgt cgt ctg tct ccg cct gcc ttc cgc tac cag ctg acc gag ctg gcg     1500
   Arg Arg Leu Ser Pro Pro Ala Phe Arg Tyr Gln Leu Thr Glu Leu Ala
   385                 390                     395                 400
   cgt aaa gcg ggc aaa cgc gtc gtt ctg ccg gaa ggc gac gaa ccg cgt     1548
   Arg Lys Ala Gly Lys Arg Val Val Leu Pro Glu Gly Asp Glu Pro Arg
                   405                     410                 415
   act gtt aaa gcg gcg gcc atc tgt gcc gag cgc ggt att gca acc tgc     1596
   Thr Val Lys Ala Ala Ala Ile Cys Ala Glu Arg Gly Ile Ala Thr Cys
               420                     425                 430
   gtg ctg ctg ggc aac ccg gat gag atc aac cgc gtt gcg gcc tct cag     1644
   Val Leu Leu Gly Asn Pro Asp Glu Ile Asn Arg Val Ala Ala Ser Gln
           435                     440                 445
   ggc gta gag ctg ggc gct ggt att gaa atc gtc gat ccg gaa gtg gtt     1692
   Gly Val Glu Leu Gly Ala Gly Ile Glu Ile Val Asp Pro Glu Val Val
       450                     455                 460
   cgc gaa agc tac gtg gcg cgt ctg gtc gaa ctg cgt aag aac aaa ggc     1740
   Arg Glu Ser Tyr Val Ala Arg Leu Val Glu Leu Arg Lys Asn Lys Gly
   465                 470                     475                 480
   atg acc gaa gcg gtt gcg cgc gaa cag ctg gaa gat aac gtg gtt ctc      1788
   Met Thr Glu Ala Val Ala Arg Glu Gln Leu Glu Asp Asn Val Val Leu
                   485                 490                 495
   ggc acc ctg atg ctg gaa caa gac gaa gtc gac ggc ctg gta tcc ggc      1836
   Gly Thr Leu Met Leu Glu Gln Asp Glu Val Asp Gly Leu Val Ser Gly
               500                 505                 510
   gcg gtt cac acc acc gcc aat acc atc cgt ccg ccg ctg cag ctt atc      1884
   Ala Val His Thr Thr Ala Asn Thr Ile Arg Pro Pro Leu Gln Leu Ile
           515                 520                 525
   aaa acg gcg ccg ggc agc tcg ctg gtc tct tcc gta ttc ttc atg ctg      1932
   Lys Thr Ala Pro Gly Ser Ser Leu Val Ser Ser Val Phe Phe Met Leu
       530                 535                 540
   ctg ccg gaa cag gtt tac gtt tac ggc gac tgc gcg atc aac ccg gat      1980
   Leu Pro Glu Gln Val Tyr Val Tyr Gly Asp Cys Ala Ile Asn Pro Asp
   545                 550                 555                 560
   ccg act gct gaa cag ctg gcg gaa atc gcg att cag tcc gct gat tcc      2028
   Pro Thr Ala Glu Gln Leu Ala Glu Ile Ala Ile Gln Ser Ala Asp Ser
                   565                 570                 575
   gcg att gcc ttc ggt atc gaa ccg cgc gtg gcg atg ctc tcc tac tcc      2076
   Ala Ile Ala Phe Gly Ile Glu Pro Arg Val Ala Met Leu Ser Tyr Ser
               580                 585                 590
   acc ggc acc tcc ggc gca ggt agc gat gta gaa aaa gta cgc gaa gca      2124
   Thr Gly Thr Ser Gly Ala Gly Ser Asp Val Glu Lys Val Arg Glu Ala
           595                 600                 605
   act cgt ctg gcg caa gaa aaa cgt cct gac ctg atg atc gac ggt ccg      2172
   Thr Arg Leu Ala Gln Glu Lys Arg Pro Asp Leu Met Ile Asp Gly Pro
       610                 615                 620
   ctg cag tat gat gcc gcg gta atg gcc gat gtt gcg aag tcc aaa gcg      2220
   Leu Gln Tyr Asp Ala Ala Val Met Ala Asp Val Ala Lys Ser Lys Ala
   625                 630                 635                 640
   cca aac tct ccg gta gct ggc cgc gcg acc gtg ttc atc ttc ccg gat      2268
   Pro Asn Ser Pro Val Ala Gly Arg Ala Thr Val Phe Ile Phe Pro Asp
                   645                 650                 655
   ctg aac acc ggt aac acc act tac aaa gcc gta cag cgt tcc gct gac      2316
   Leu Asn Thr Gly Asn Thr Thr Tyr Lys Ala Val Gln Arg Ser Ala Asp
               660                 665                 670
   ctg atc tcc atc ggg ccg atg ctg cag ggt atg cgt aag ccg gta aac      2364
   Leu Ile Ser Ile Gly Pro Met Leu Gln Gly Met Arg Lys Pro Val Asn
           675                 680                 685
   gac ctg tcc cgc ggc gcg ctg gta gac gat atc gtc tac acc atc gca      2412
   Asp Leu Ser Arg Gly Ala Leu Val Asp Asp Ile Val Tyr Thr Ile Ala
       690                 695                 700
   ctg acg gcg att cag tct tct cag cag cag aaa taa tctcccgctg           2458
   Leu Thr Ala Ile Gln Ser Ser Gln Gln Gln Lys
   705                 710                 715
   agacaagaaa ggcagccaaa tggctgcctt ttttatggct ctaacgcgta aaggaaatta    2518
   cgcttcgtct tcttccggct cgtcgcgatc gtggccggta ttgcccgcgt tacgggtcat    2578
   ccacagcgcc agcgctttca gtgaatcggc ggtgaactca tcgcagcgcg cggtgatctc    2638
   ttccggcgtc aaccagcata cctcgctgac ctcttcttcc tgcagggcga               2688
<210> 51
   <211> 715
   <212> PRT
   <213> Enterobacter aerogenes
<400> 51
   Val Ser Arg Thr Ile Met Leu Ile Pro Thr Gly Thr Ser Val Gly Leu
   1               5                   10                  15
   Thr Ser Val Ser Leu Gly Val Ile Arg Ala Met Glu Arg Lys Gly Val
               20                  25                  30
   Arg Leu Ser Val Phe Lys Pro Ile Ala Gln Pro Arg Ser Gly Gly Asp
           35                  40                  45
   Thr Pro Asp Gln Thr Thr Thr Ile Val Arg Ala Ser Ser Ser Thr Thr
   50 55 60
   Thr Ala Ala Glu Pro Met Asn Met Ser His Val Glu Ser Leu Leu Ser
   65 70 75 80
   Ser Asn Gln Lys Asp Val Leu Met Glu Glu Ile Ile Ala Asn Tyr His
   85 90 95
   Ala Asn Thr Gln Asp Ala Glu Val Val Leu Val Glu Gly Leu Val Pro
   100 105 110
   Thr Arg Lys His Gln Phe Ala Gln Ser Leu Asn Tyr Glu Ile Ala Lys
   115 120 125
   Thr Leu Asn Ala Glu Ile Val Phe Val Met Ser Gln Gly Thr Asp Thr
   130 135 140
   Pro Glu Gln Leu Asn Glu Arg Ile Glu Leu Thr Arg Asn Ser Phe Gly
   145 150 155 160
   Gly Ala Lys Asn Thr Ser Ile Thr Gly Val Ile Val Asn Lys Leu Asn
   165 170 175
   Ala Pro Val Asp Glu Gln Gly Arg Thr Arg Pro Asp Leu Ser Glu Ile
   180 185 190
   Phe Asp Asp Ser Ser Lys Ala Lys Val Val Lys Ile Asp Pro Ala Gln
   195 200 205
   Leu Gln Asn Gly Ser Pro Leu Pro Val Leu Gly Ala Val Pro Trp Ser
   210 215 220
   Phe Asp Leu Ile Ala Thr Arg Ala Ile Asp Met Ala His His Leu Asn
   225 230 235 240
   Ala Thr Val Ile Asn Glu Gly Asp Ile Asn Thr Arg Arg Val Lys Ser
   245 250 255
   Val Thr Phe Cys Ala Arg Ser Ile Pro His Met Leu Glu His Phe Arg
   260 265 270
   Pro Gly Ser Leu Leu Val Thr Ser Ala Asp Arg Pro Asp Val Leu Val
   275 280 285
   Ala Ala Cys Leu Ala Ala Met Asn Gly Val Glu Ile Gly Ala Ile Leu
   290 295 300
   Leu Thr Gly Gly Tyr Glu Met Asp Ala Arg Ile Ser Lys Leu Cys Glu
   305 310 315 320
   Arg Ala Phe Ala Thr Gly Leu Pro Val Phe Met Val Asn Thr Asn Thr
   325 330 335
   Trp Gln Thr Ser Leu Ser Leu Gln Ser Phe Asn Leu Glu Val Pro Val
   340 345 350
   Asp Asp His Glu Arg Ile Glu Lys Val Gln Glu Tyr Val Ala Asn Tyr
   355 360 365
   Ile Asn Ala Asp Trp Ile Glu Ser Leu Thr Ala Thr Ser Glu Arg Ser
   370 375 380
   Arg Arg Leu Ser Pro Pro Ala Phe Arg Tyr Gln Leu Thr Glu Leu Ala
   385 390 395 400
   Arg Lys Ala Gly Lys Arg Val Val Leu Pro Glu Gly Asp Glu Pro Arg
   405 410 415
   Thr Val Lys Ala Ala Ala Ile Cys Ala Glu Arg Gly Ile Ala Thr Cys
   420 425 430
   Val Leu Leu Gly Asn Pro Asp Glu Ile Asn Arg Val Ala Ala Ser Gln
   435 440 445
   Gly Val Glu Leu Gly Ala Gly Ile Glu Ile Val Asp Pro Glu Val Val
   450 455 460
   Arg Glu Ser Tyr Val Ala Arg Leu Val Glu Leu Arg Lys Asn Lys Gly
   465 470 475 480
   Met Thr Glu Ala Val Ala Arg Glu Gln Leu Glu Asp Asn Val Val Leu
   485 490 495
   Gly Thr Leu Met Leu Glu Gln Asp Glu Val Asp Gly Leu Val Ser Gly
   500 505 510
   Ala Val His Thr Thr Ala Asn Thr Ile Arg Pro Pro Leu Gln Leu Ile
   515 520 525
   Lys Thr Ala Pro Gly Ser Ser Leu Val Ser Ser Val Phe Phe Met Leu
   530 535 540
   Leu Pro Glu Gln Val Tyr Val Tyr Gly Asp Cys Ala Ile Asn Pro Asp
   545 550 555 560
   Pro Thr Ala Glu Gln Leu Ala Glu Ile Ala Ile Gln Ser Ala Asp Ser
                           565                 570                 575
   Ala Ile Ala Phe Gly Ile Glu Pro Arg Val Ala Met Leu Ser Tyr Ser
                   580                 585                 590
   Thr Gly Thr Ser Gly Ala Gly Ser Asp Val Glu Lys Val Arg Glu Ala
               595                 600                 605
   Thr Arg Leu Ala Gln Glu Lys Arg Pro Asp Leu Met Ile Asp Gly Pro
       610                 615                 620
   Leu Gln Tyr Asp Ala Ala Val Met Ala Asp Val Ala Lys Ser Lys Ala
   625                 630                 635                 640
   Pro Asn Ser Pro Val Ala Gly Arg Ala Thr Val Phe Ile Phe Pro Asp
                   645                 650                 655
   Leu Asn Thr Gly Asn Thr Thr Tyr Lys Ala Val Gln Arg Ser Ala Asp
               660                 665                 670
   Leu Ile Ser Ile Gly Pro Met Leu Gln Gly Met Arg Lys Pro Val Asn
           675                 680                 685
   Asp Leu Ser Arg Gly Ala Leu Val Asp Asp Ile Val Tyr Thr Ile Ala
       690                 695                 700
   Leu Thr Ala Ile Gln Ser Ser Gln Gln Gln Lys
   705                 710                 715
<210> 52
   <211> 1125
   <212> DNA
   <213> Enterobacter aerogenes
<220>
   <221> CDS
   <222> (181)..(846)
<400> 52
   agttctttcg ctgcccgggt gaagtgccgc gtccgggcga cggcgacaaa atagcgtaga      60
   taacgaagtt ccatatcgaa aacgtctcaa accagcatgg tttctatatt ggaactgtga     120
   gctgaatcgg gtcaaccttt atttaacctt tcttatattt gttgaacgag gaagtggctc     180
   atg aat cat gct tca gat tgc acc tgt gaa gag agt ctg tgt gaa acg      228
   Met Asn His Ala Ser Asp Cys Thr Cys Glu Glu Ser Leu Cys Glu Thr
   1               5                   10                  15
   cta cgc gcg ttt tcc gct cag cat ccc gat agc gtg ctg tat caa act      276
   Leu Arg Ala Phe Ser Ala Gln His Pro Asp Ser Val Leu Tyr Gln Thr
               20                  25                  30
   tcg ctg atg agc gcc ctg ctc agc ggc gtc tac gaa ggt acc acc act      324
   Ser Leu Met Ser Ala Leu Leu Ser Gly Val Tyr Glu Gly Thr Thr Thr
           35                  40                  45
   att gcg gac ctg ctg aag cac ggt gat ttc ggg ctc ggc act ttt aat      372
   Ile Ala Asp Leu Leu Lys His Gly Asp Phe Gly Leu Gly Thr Phe Asn
       50                  55                  60
   gaa ctc gac ggc gag ctg atc gcg ttt agc agc cag gtt tat caa ctg      420
   Glu Leu Asp Gly Glu Leu Ile Ala Phe Ser Ser Gln Val Tyr Gln Leu
   65                  70                  75                  80
   cgt gcc gac ggc agc gcg cgt aaa gcg cgt ccg gaa cag aaa acg ccg      468
   Arg Ala Asp Gly Ser Ala Arg Lys Ala Arg Pro Glu Gln Lys Thr Pro
                   85                  90                  95
   ttt gca gtg atg acc tgg ttt cag ccg cag tac cgt aaa acc ttt gac      516
   Phe Ala Val Met Thr Trp Phe Gln Pro Gln Tyr Arg Lys Thr Phe Asp
               100                 105                 110
   cat ccg gtc agc cgc cag cag ctg cat gag gtt att gac cag caa att      564
   His Pro Val Ser Arg Gln Gln Leu His Glu Val Ile Asp Gln Gln Ile
           115                 120                 125
   cct tcc gac aat ctg ttc tgc gcg ctg cga atc gat ggt cat ttc cgc      612
   Pro Ser Asp Asn Leu Phe Cys Ala Leu Arg Ile Asp Gly His Phe Arg
       130                 135                 140
   cac gcc cat acc cgc acc gtg cct cgt cag acg ccg ccc tac cgg gcg      660
   His Ala His Thr Arg Thr Val Pro Arg Gln Thr Pro Pro Tyr Arg Ala
   145                 150                 155                 160
   atg acc gac gtg ctc gac gat cag ccg gtt ttc cgc ttt aac cag cgt      708
   Met Thr Asp Val Leu Asp Asp Gln Pro Val Phe Arg Phe Asn Gln Arg
                   165                 170                 175
   gac ggc gta ctg gtc ggt ttt cgg acc ccc agc ata tgc agg gaa tta      756
   Asp Gly Val Leu Val Gly Phe Arg Thr Pro Ser Ile Cys Arg Glu Leu
               180                 185                 190
   acg tcg ccg gct atc acg aac act tca tta ccg atg acc gcc agg gcg      804
   Thr Ser Pro Ala Ile Thr Asn Thr Ser Leu Pro Met Thr Ala Arg Ala
           195                 200                 205
   gcg gcc acc tgc tgg act acc agc tcg acc atg ggg tat tga              846
   Ala Ala Thr Cys Trp Thr Thr Ser Ser Thr Met Gly Tyr
       210                 215                 220
   ccttcggcga aattcataag ctgatgatcg accttcccgc cgacagcgcg ttcctgcagg    906
   ccaatttgca tcccgataat ctcgatgccg ccatccgttc agtagaaagt taggaggttc    966
   acatggacaa acagtatccg cagcgccagt gggcgcacgg cgccgatctg gtcgtcagcc   1026
   aactggaagc gcaaggcgta cggcaggtct tcgggatccc cggcgctaaa atcgataagg   1086
   ttttcgactc gttgctggac tcctcaatcc gcattattc                          1125
<210> 53
   <211> 221
   <212> PRT
   <213> Enterobacter aerogenes
<400> 53
   Met Asn His Ala Ser Asp Cys Thr Cys Glu Glu Ser Leu Cys Glu Thr
   1               5                   10                  15
   Leu Arg Ala Phe Ser Ala Gln His Pro Asp Ser Val Leu Tyr Gln Thr
               20                  25                  30
   Ser Leu Met Ser Ala Leu Leu Ser Gly Val Tyr Glu Gly Thr Thr Thr
           35                  40                  45
   Ile Ala Asp Leu Leu Lys His Gly Asp Phe Gly Leu Gly Thr Phe Asn
       50                  55                  60
   Glu Leu Asp Gly Glu Leu Ile Ala Phe Ser Ser Gln Val Tyr Gln Leu
   65                  70                  75                  80
   Arg Ala Asp Gly Ser Ala Arg Lys Ala Arg Pro Glu Gln Lys Thr Pro
                   85                  90                  95
   Phe Ala Val Met Thr Trp Phe Gln Pro Gln Tyr Arg Lys Thr Phe Asp
               100                 105                 110
   His Pro Val Ser Arg Gln Gln Leu His Glu Val Ile Asp Gln Gln Ile
           115                 120                 125
   Pro Ser Asp Asn Leu Phe Cys Ala Leu Arg Ile Asp Gly His Phe Arg
       130                 135                 140
   His Ala His Thr Arg Thr Val Pro Arg Gln Thr Pro Pro Tyr Arg Ala
   145                 150                 155                 160
   Met Thr Asp Val Leu Asp Asp Gln Pro Val Phe Arg Phe Asn Gln Arg
                   165                 170                 175
   Asp Gly Val Leu Val Gly Phe Arg Thr Pro Ser Ile Cys Arg Glu Leu
               180                 185                 190
   Thr Ser Pro Ala Ile Thr Asn Thr Ser Leu Pro Met Thr Ala Arg Ala
           195                 200                 205
   Ala Ala Thr Cys Trp Thr Thr Ser Ser Thr Met Gly Tyr
       210                 215                 220
<210> 54
   <211> 2823
   <212> DNA
   <213> Enterobacter aerogenes
<220>
   <221> CDS
   <222> (355)..(2637)
<400> 54
   cgcagcctga tggacaaagc gatgatcatg gtgttgccgg ttgcgatgtt tgtcgccagc      60
   ggctttgaac acagcatcgc taatatgttt atgatcccga tgggtatcgt aattcgcaac     120
   tttgcaagcc cggaattctg gaccgctatc ggttcaactc cggagagttt ttctcactta     180
   accgttatga acttcatcac tgataacctg attccagtca ctatcggaaa catcatcggc     240
   ggtggtttgt tggttgggtt gacatactgg gtcatttacc tgcgtggcga cgatcatcat     300
   taatggttgt ctcaggcagt aaataaaaaa tccacttaag aaggtaggtg ttac atg       357
                                                               Met
                                                               1
   tcc gag ctt aat gaa aag tta gcc aca gcc tgg gaa ggt ttt gcg aaa       405
   Ser Glu Leu Asn Glu Lys Leu Ala Thr Ala Trp Glu Gly Phe Ala Lys
               5                   10                  15
   ggt gac tgg cag aac gaa gtc aac gta cgt gac ttt atc cag aaa aac       453
   Gly Asp Trp Gln Asn Glu Val Asn Val Arg Asp Phe Ile Gln Lys Asn
           20                  25                  30
   tac acc cca tat gaa ggt gac gaa tcc ttc ctg gct ggc gca act gat       501
   Tyr Thr Pro Tyr Glu Gly Asp Glu Ser Phe Leu Ala Gly Ala Thr Asp
       35                  40                  45
   gcg acc acc aag ctg tgg gac agc gta atg gaa ggc gtt aaa cag gaa       549
   Ala Thr Thr Lys Leu Trp Asp Ser Val Met Glu Gly Val Lys Gln Glu
   50                  55                  60                  65
   aac cgc act cac gcg cct gtt gat ttc gac act tcc ctc gca tcc acc       597
   Asn Arg Thr His Ala Pro Val Asp Phe Asp Thr Ser Leu Ala Ser Thr
                   70                  75                  80
   atc act tct cac gac gcg ggc tac atc gag aaa gcg ctc gag aaa atc       645
   Ile Thr Ser His Asp Ala Gly Tyr Ile Glu Lys Ala Leu Glu Lys Ile
               85                  90                  95
   gtt ggt ctg caa act gaa gcc ccg ctg aaa cgt gcg att atc ccg ttc       693
   Val Gly Leu Gln Thr Glu Ala Pro Leu Lys Arg Ala Ile Ile Pro Phe
           100                 105                 110
   ggc ggt atc aaa atg gtt gaa ggt tcc tgc aaa gcg tac aat cgc gaa       741
   Gly Gly Ile Lys Met Val Glu Gly Ser Cys Lys Ala Tyr Asn Arg Glu
       115                 120                 125
   ctg gac ccg atg ctg aaa aaa atc ttc acc gag tac cgt aaa act cac       789
   Leu Asp Pro Met Leu Lys Lys Ile Phe Thr Glu Tyr Arg Lys Thr His
   130                 135                 140                 145
   aac cag ggc gtt ttc gac gta tat acc ccg gac atc ctg cgc tgc cgt       837
   Asn Gln Gly Val Phe Asp Val Tyr Thr Pro Asp Ile Leu Arg Cys Arg
                   150                 155                 160
   aaa tcc ggc gta ctg acc ggt ctg ccg gat gct tac ggc cgt ggt cgt       885
   Lys Ser Gly Val Leu Thr Gly Leu Pro Asp Ala Tyr Gly Arg Gly Arg
               165                 170                 175
   atc atc ggt gac tat cgt cgc gtt gcg ctg tac ggt atc gac ttc ctg       933
   Ile Ile Gly Asp Tyr Arg Arg Val Ala Leu Tyr Gly Ile Asp Phe Leu
           180                 185                 190
   atg aaa gac aaa ttc gcc cag ttc aac tcg ctg cag gcg aaa ctg gaa       981
   Met Lys Asp Lys Phe Ala Gln Phe Asn Ser Leu Gln Ala Lys Leu Glu
       195                 200                 205
   agc ggc gaa gat ctg gaa gca acc atc cgt ctg cgt gaa gaa att gct      1029
   Ser Gly Glu Asp Leu Glu Ala Thr Ile Arg Leu Arg Glu Glu Ile Ala
   210                 215                 220                 225
   gaa cag cat cgc gcg ctg ggt cag atc aaa gag atg gcg gct aaa tat      1077
   Glu Gln His Arg Ala Leu Gly Gln Ile Lys Glu Met Ala Ala Lys Tyr
                   230                 235                 240
   ggc tac gac atc tcc ggt ccg gcg act acc gct cag gaa gct att cag      1125
   Gly Tyr Asp Ile Ser Gly Pro Ala Thr Thr Ala Gln Glu Ala Ile Gln
               245                 250                 255
   tgg acc tac ttc ggt tac ctg gcc gcc gtt aaa tct cag aac ggc gcg      1173
   Trp Thr Tyr Phe Gly Tyr Leu Ala Ala Val Lys Ser Gln Asn Gly Ala
           260                 265                 270
   gca atg tcc ttc ggt cgt act tcc agc ttc ctg gat atc tac atc gaa      1221
   Ala Met Ser Phe Gly Arg Thr Ser Ser Phe Leu Asp Ile Tyr Ile Glu
       275                 280                 285
   cgt gac ctg cag gcg ggt aaa atc acc gag caa gac gcg cag gaa atg      1269
   Arg Asp Leu Gln Ala Gly Lys Ile Thr Glu Gln Asp Ala Gln Glu Met
   290                 295                 300                 305
   gtt gac cac ctg gtc atg aaa ctg cgt atg gtt cgc ttc ctg cgt acc      1317
   Val Asp His Leu Val Met Lys Leu Arg Met Val Arg Phe Leu Arg Thr
                   310                 315                 320
   ccg gaa tat gat gaa ctg ttc tcc ggc gac ccg att tgg gca acg gaa      1365
   Pro Glu Tyr Asp Glu Leu Phe Ser Gly Asp Pro Ile Trp Ala Thr Glu
               325                 330                 335
   tct atc ggt ggt atg ggc gtt gac ggc cgt act ctg gta acc aaa aac      1413
   Ser Ile Gly Gly Met Gly Val Asp Gly Arg Thr Leu Val Thr Lys Asn
           340                 345                 350
   agc ttc cgc ttc ctg aac acc ctg tac acc atg ggg ccg tct ccg gag      1461
   Ser Phe Arg Phe Leu Asn Thr Leu Tyr Thr Met Gly Pro Ser Pro Glu
       355                 360                 365
   ccg aac atc act atc ctg tgg tct gaa aaa ctg ccg ctg agc ttt aag      1509
   Pro Asn Ile Thr Ile Leu Trp Ser Glu Lys Leu Pro Leu Ser Phe Lys
   370                 375                 380                 385
   aaa ttc gcc gct aaa gta tcc atc gat acc tct tct ctg cag tac gag      1557
   Lys Phe Ala Ala Lys Val Ser Ile Asp Thr Ser Ser Leu Gln Tyr Glu
                   390                 395                 400
   aac gat gac ctg atg cgc ccg gac ttc aac aac gac gat tac gct atc      1605
   Asn Asp Asp Leu Met Arg Pro Asp Phe Asn Asn Asp Asp Tyr Ala Ile
               405                 410                 415
   gca tgc tgc gta agc ccg atg att gtt ggt aaa caa atg cag ttc ttc      1653
   Ala Cys Cys Val Ser Pro Met Ile Val Gly Lys Gln Met Gln Phe Phe
           420                 425                 430
   ggc gct cgc gct aac ctc gcg aaa acc atg ctg tat gct atc aac ggc      1701
   Gly Ala Arg Ala Asn Leu Ala Lys Thr Met Leu Tyr Ala Ile Asn Gly
       435                 440                 445
   ggc gtt gat gaa aaa ctg aaa atg cag gtt ggt ccg aaa tct gaa ccg      1749
   Gly Val Asp Glu Lys Leu Lys Met Gln Val Gly Pro Lys Ser Glu Pro
   450                 455                 460                 465
   atc aaa ggc gac gtc ctg aac ttc gac gaa gtt atg gag cgc atg gat      1797
   Ile Lys Gly Asp Val Leu Asn Phe Asp Glu Val Met Glu Arg Met Asp
                   470                 475                 480
   cac ttc atg gac tgg ctg gct aaa cag tac gtc acc gcg ctg aac atc      1845
   His Phe Met Asp Trp Leu Ala Lys Gln Tyr Val Thr Ala Leu Asn Ile
               485                 490                 495
   att cat tac atg cat gac aag tac agc tac gaa gcc tct ctg atg gcg      1893
   Ile His Tyr Met His Asp Lys Tyr Ser Tyr Glu Ala Ser Leu Met Ala
           500                 505                 510
   ctg cac gac cgt gac gtt atc cgc acc atg gcg tgt ggt atc gca ggt      1941
   Leu His Asp Arg Asp Val Ile Arg Thr Met Ala Cys Gly Ile Ala Gly
       515                 520                 525
   ctg tcc gtt gct gct gac tcc ctg tct gct atc aaa tat gcg aaa gtt      1989
   Leu Ser Val Ala Ala Asp Ser Leu Ser Ala Ile Lys Tyr Ala Lys Val
   530                 535                 540                 545
   aaa ccg att cgt gac gaa gac ggt ctg gct gtt gac ttc gaa atc gaa      2037
   Lys Pro Ile Arg Asp Glu Asp Gly Leu Ala Val Asp Phe Glu Ile Glu
                   550                 555                 560
   ggc gaa tac ccg cag ttt ggt aac aac gat gct cgc gtc gat gac atg      2085
   Gly Glu Tyr Pro Gln Phe Gly Asn Asn Asp Ala Arg Val Asp Asp Met
               565                 570                 575
   gcc gtt gac ctg gtt gaa cgt ttc atg aag aaa att cag aaa ctg cac      2133
   Ala Val Asp Leu Val Glu Arg Phe Met Lys Lys Ile Gln Lys Leu His
           580                 585                 590
   acc tac cgc aac gct atc ccg act cag tcc gtt ctg acc atc act tct      2181
   Thr Tyr Arg Asn Ala Ile Pro Thr Gln Ser Val Leu Thr Ile Thr Ser
       595                 600                 605
   aac gtc gtg tat ggt aag aaa acc ggt aac acc cca gat ggt cgt cgc      2229
   Asn Val Val Tyr Gly Lys Lys Thr Gly Asn Thr Pro Asp Gly Arg Arg
   610                 615                 620                 625
   gct ggc gcg ccg ttc gga cca ggt gct aac ccg atg cac ggc cgt gac      2277
   Ala Gly Ala Pro Phe Gly Pro Gly Ala Asn Pro Met His Gly Arg Asp
                   630                 635                 640
   cag aaa ggt gct gta gcc tct ctg act tcc gtt gct aaa ctg ccg ttt      2325
   Gln Lys Gly Ala Val Ala Ser Leu Thr Ser Val Ala Lys Leu Pro Phe
               645                 650                 655
   gct tac gct aaa gat ggt atc tct tac acc ttc tct atc gtg ccg aac      2373
   Ala Tyr Ala Lys Asp Gly Ile Ser Tyr Thr Phe Ser Ile Val Pro Asn
           660                 665                 670
   gcg ctg ggt aaa gat gac gaa gtt cgt aag acc aac ctg gcg ggc ctg      2421
   Ala Leu Gly Lys Asp Asp Glu Val Arg Lys Thr Asn Leu Ala Gly Leu
       675                 680                 685
   atg gat ggt tac ttc cac cac gaa gcg tcc atc gaa ggt ggt cag cac      2469
   Met Asp Gly Tyr Phe His His Glu Ala Ser Ile Glu Gly Gly Gln His
   690                 695                 700                 705
   ctg aac gtg aac gtc atg aac cgc gaa atg ctg ctc gac gcg atg gaa        2517
   Leu Asn Val Asn Val Met Asn Arg Glu Met Leu Leu Asp Ala Met Glu
                   710                 715                 720
   aac ccg gaa aaa tat ccg cag ctg acc att cgt gta tct ggc tac gcg      2565
   Asn Pro Glu Lys Tyr Pro Gln Leu Thr Ile Arg Val Ser Gly Tyr Ala
               725                 730                 735
   gta cgt ttt aac tcc ctg act aaa gaa cag cag cag gat gtt att acc      2613
   Val Arg Phe Asn Ser Leu Thr Lys Glu Gln Gln Gln Asp Val Ile Thr
           740                 745                 750
   cgt acc ttc act cag acc atg taa ttccctgtct gactgaaaaa gcgtacaata     2667
   Arg Thr Phe Thr Gln Thr Met
       755                 760
   aaggccccac atcagtgggg cctttttaac acgtgattcc ctgccccagc ctgctttgcc   2727
   agttatctat actttgggta cctgtcaaaa cagacttaac acagccggtt tgagctgtgc    2787
   atcacaggcc ctggagggcc gaacccggag atatca                              2823
<210> 55
   <211> 760
   <212> PRT
   <213> Enterobacter aerogenes
<400> 55
   Met Ser Glu Leu Asn Glu Lys Leu Ala Thr Ala Trp Glu Gly Phe Ala
   1               5                   10                  15
   Lys Gly Asp Trp Gln Asn Glu Val Asn Val Arg Asp Phe Ile Gln Lys
               20                  25                  30
   Asn Tyr Thr Pro Tyr Glu Gly Asp Glu Ser Phe Leu Ala Gly Ala Thr
           35                  40                  45
   Asp Ala Thr Thr Lys Leu Trp Asp Ser Val Met Glu Gly Val Lys Gln
       50                  55                  60
   Glu Asn Arg Thr His Ala Pro Val Asp Phe Asp Thr Ser Leu Ala Ser
   65                  70                  75                  80
   Thr Ile Thr Ser His Asp Ala Gly Tyr Ile Glu Lys Ala Leu Glu Lys
                   85                  90                  95
   Ile Val Gly Leu Gln Thr Glu Ala Pro Leu Lys Arg Ala Ile Ile Pro
               100                 105                 110
   Phe Gly Gly Ile Lys Met Val Glu Gly Ser Cys Lys Ala Tyr Asn Arg
           115                 120                 125
   Glu Leu Asp Pro Met Leu Lys Lys Ile Phe Thr Glu Tyr Arg Lys Thr
       130                 135                 140
   His Asn Gln Gly Val Phe Asp Val Tyr Thr Pro Asp Ile Leu Arg Cys
   145                 150                 155                 160
   Arg Lys Ser Gly Val Leu Thr Gly Leu Pro Asp Ala Tyr Gly Arg Gly
                   165                 170                 175
   Arg Ile Ile Gly Asp Tyr Arg Arg Val Ala Leu Tyr Gly Ile Asp Phe
               180                 185                 190
   Leu Met Lys Asp Lys Phe Ala Gln Phe Asn Ser Leu Gln Ala Lys Leu
           195                 200                 205
   Glu Ser Gly Glu Asp Leu Glu Ala Thr Ile Arg Leu Arg Glu Glu Ile
       210                 215                 220
   Ala Glu Gln His Arg Ala Leu Gly Gln Ile Lys Glu Met Ala Ala Lys
   225                 230                 235                 240
   Tyr Gly Tyr Asp Ile Ser Gly Pro Ala Thr Thr Ala Gln Glu Ala Ile
                   245                 250                 255
   Gln Trp Thr Tyr Phe Gly Tyr Leu Ala Ala Val Lys Ser Gln Asn Gly
               260                 265                 270
   Ala Ala Met Ser Phe Gly Arg Thr Ser Ser Phe Leu Asp Ile Tyr Ile
           275                 280                 285
   Glu Arg Asp Leu Gln Ala Gly Lys Ile Thr Glu Gln Asp Ala Gln Glu
       290                 295                 300
   Met Val Asp His Leu Val Met Lys Leu Arg Met Val Arg Phe Leu Arg
   305                 310                 315                 320
   Thr Pro Glu Tyr Asp Glu Leu Phe Ser Gly Asp Pro Ile Trp Ala Thr
                   325                 330                 335
   Glu Ser Ile Gly Gly Met Gly Val Asp Gly Arg Thr Leu Val Thr Lys
               340                 345                 350
   Asn Ser Phe Arg Phe Leu Asn Thr Leu Tyr Thr Met Gly Pro Ser Pro
           355                 360                 365
   Glu Pro Asn Ile Thr Ile Leu Trp Ser Glu Lys Leu Pro Leu Ser Phe
       370                 375                 380
   Lys Lys Phe Ala Ala Lys Val Ser Ile Asp Thr Ser Ser Leu Gln Tyr
   385                 390                 395                 400
   Glu Asn Asp Asp Leu Met Arg Pro Asp Phe Asn Asn Asp Asp Tyr Ala
                   405                 410                 415
   Ile Ala Cys Cys Val Ser Pro Met Ile Val Gly Lys Gln Met Gln Phe
               420                 425                 430
   Phe Gly Ala Arg Ala Asn Leu Ala Lys Thr Met Leu Tyr Ala Ile Asn
           435                 440                 445
   Gly Gly Val Asp Glu Lys Leu Lys Met Gln Val Gly Pro Lys Ser Glu
       450                 455                 460
   Pro Ile Lys Gly Asp Val Leu Asn Phe Asp Glu Val Met Glu Arg Met
   465                 470                 475                 480
   Asp His Phe Met Asp Trp Leu Ala Lys Gln Tyr Val Thr Ala Leu Asn
                   485                 490                 495
   Ile Ile His Tyr Met His Asp Lys Tyr Ser Tyr Glu Ala Ser Leu Met
               500                 505                 510
   Ala Leu His Asp Arg Asp Val Ile Arg Thr Met Ala Cys Gly Ile Ala
           515                 520                 525
   Gly Leu Ser Val Ala Ala Asp Ser Leu Ser Ala Ile Lys Tyr Ala Lys
       530                 535                 540
   Val Lys Pro Ile Arg Asp Glu Asp Gly Leu Ala Val Asp Phe Glu Ile
   545                 550                 555                 560
   Glu Gly Glu Tyr Pro Gln Phe Gly Asn Asn Asp Ala Arg Val Asp Asp
                   565                 570                 575
   Met Ala Val Asp Leu Val Glu Arg Phe Met Lys Lys Ile Gln Lys Leu
               580                 585                 590
   His Thr Tyr Arg Asn Ala Ile Pro Thr Gln Ser Val Leu Thr Ile Thr
           595                 600                 605
   Ser Asn Val Val Tyr Gly Lys Lys Thr Gly Asn Thr Pro Asp Gly Arg
       610                 615                 620
   Arg Ala Gly Ala Pro Phe Gly Pro Gly Ala Asn Pro Met His Gly Arg
   625                 630                 635                 640
   Asp Gln Lys Gly Ala Val Ala Ser Leu Thr Ser Val Ala Lys Leu Pro
                   645                 650                 655
   Phe Ala Tyr Ala Lys Asp Gly Ile Ser Tyr Thr Phe Ser Ile Val Pro
               660                 665                 670
   Asn Ala Leu Gly Lys Asp Asp Glu Val Arg Lys Thr Asn Leu Ala Gly
           675                 680                 685
   Leu Met Asp Gly Tyr Phe His His Glu Ala Ser Ile Glu Gly Gly Gln
       690                 695                 700
   His Leu Asn Val Asn Val Met Asn Arg Glu Met Leu Leu Asp Ala Met
   705                 710                 715                 720
   Glu Asn Pro Glu Lys Tyr Pro Gln Leu Thr Ile Arg Val Ser Gly Tyr
                   725                 730                 735
   Ala Val Arg Phe Asn Ser Leu Thr Lys Glu Gln Gln Gln Asp Val Ile
   740 745 750
   Thr Arg Thr Phe Thr Gln Thr Met
   755 760
<210> 56
   <211> 89
   <212> DNA
   <213> Artificial Sequence
<220>
   <223> primer
<400> 56
   atgttaacga cgcatacggc tttgaactgg aatttttcga cttcctgctg accgaaaaga 60
   tgaagcctgc ttttttatac taagttggc 89
<210> 57
   <211> 90
   <212> DNA
   <213> Artificial Sequence
<220>
   <223> primer
<400> 57
   ggcaggcgga gagccggcgg aacacgtcat cctggatgac atcattcgat ttatcttcaa 60
   cgctcaagtt agtataaaaa agctgaacga 90
<210> 58
   <211> 89
   <212> DNA
   <213> Artificial Sequence
<220>
   <223> primer
<400> 58
   ctggcggtgc tgttttgtat cccgcctaaa actggcggta acgaaagagg atatatcgtg 60
   tgaagcctgc ttttttatac taagttggc 89
<210> 59
   <211> 90
   <212> DNA
   <213> Artificial Sequence
<220>
   <223> primer
<400> 59
   gcgttagagc cataaaaaag gcagccattt ggctgccttt cttgtctcag cgggagatta 60
   cgctcaagtt agtataaaaa agctgaacga 90
<210> 60
   <211> 89
   <212> DNA
   <213> Artificial Sequence
<220>
   <223> primer
<400> 60
   gaatcgggtc aacctttatt taacctttct tatatttgtt gaacgaggaa gtggctcatg 60
   tgaagcctgc ttttttatac taagttggc 89
<210> 61
   <211> 90
   <212> DNA
   <213> Artificial Sequence
<220>
   <223> primer
<400> 61
   cagatcggcg ccgtgcgccc actggcgctg cggatactgt ttgtccatgt gaacctccta 60
   cgctcaagtt agtataaaaa agctgaacga 90
<210> 62
   <211> 89
   <212> DNA
   <213> Artificial Sequence
<220>
   <223> primer
<400> 62
   cattaatggt tgtctcaggc agtaaataaa aaatccactt aagaaggtag gtgttacatg 60
   tgaagcctgc ttttttatac taagttggc 89
<210> 63
   <211> 90
   <212> DNA
   <213> Artificial sequence
<220>
   <223> primer
<400> 63
   aaaaaggccc cactgatgtg gggcctttat tgtacgcttt ttcagtcaga cagggaatta      60
   cgctcaagtt agtataaaaa agctgaacga                                       90
<210> 64
   <211> 1620
   <212> DNA
   <213> Selenomonas ruminantium
<220>
   <221> CDS
   <222> (1)..(1620)
<400> 64
   atg gca aac atc gat ctt agc caa tat ggc atc act ggt act acc gga      48
   Met Ala Asn Ile Asp Leu Ser Gln Tyr Gly Ile Thr Gly Thr Thr Gly
   1               5                   10                  15
   att ctc cac aat ccg tct tac aag acg ctt ttt gaa gaa gag act aaa      96
   Ile Leu His Asn Pro Ser Tyr Lys Thr Leu Phe Glu Glu Glu Thr Lys
               20                  25                  30
   gaa ggt tta aca ggc tac gaa cag ggt cag gtt tcc gaa ctg ggc gct      144
   Glu Gly Leu Thr Gly Tyr Glu Gln Gly Gln Val Ser Glu Leu Gly Ala
           35                  40                  45
   gtt aac gta aag act ggt att ttc acc ggc cgt tct cct aaa gat aaa      192
   Val Asn Val Lys Thr Gly Ile Phe Thr Gly Arg Ser Pro Lys Asp Lys
       50                  55                  60
   ttc atc gtg gat gat gaa act tcc cat gac act gta tgg tgg gat tcc      240
   Phe Ile Val Asp Asp Glu Thr Ser His Asp Thr Val Trp Trp Asp Ser
   65                  70                  75                  80
   gaa gat tat cac aac gat aac cac aga gct acg ccg gaa acc tgg aac      288
   Glu Asp Tyr His Asn Asp Asn His Arg Ala Thr Pro Glu Thr Trp Asn
                   85                  90                  95
   gct ctg aaa gaa atc gct aaa aag gaa ctg tcc aac aag aaa ctc tac      336
   Ala Leu Lys Glu Ile Ala Lys Lys Glu Leu Ser Asn Lys Lys Leu Tyr
               100                 105                 110
   gtt gta gat gct ttc tgc ggt gcc aac aaa gac acc cgc atg gct gtc      384
   Val Val Asp Ala Phe Cys Gly Ala Asn Lys Asp Thr Arg Met Ala Val
           115                 120                 125
   cgc ttc atc gta gaa gtt gct tgg cag gca cat ttc gta acg aat atg      432
   Arg Phe Ile Val Glu Val Ala Trp Gln Ala His Phe Val Thr Asn Met
       130                 135                 140
   ttc atc cag ccg acg gaa gaa gag ctg gct aac ttc aag ccg gac ttc      480
   Phe Ile Gln Pro Thr Glu Glu Glu Leu Ala Asn Phe Lys Pro Asp Phe
   145                 150                 155                 160
   gta gtt tac aac gct tcc aag gct aaa gtt gaa aac tac aag gaa ctt      528
   Val Val Tyr Asn Ala Ser Lys Ala Lys Val Glu Asn Tyr Lys Glu Leu
                   165                 170                 175
   ggt ctc cat tcc gaa acg gca gta gta ttt aac ctc acg agc cgc gaa      576
   Gly Leu His Ser Glu Thr Ala Val Val Phe Asn Leu Thr Ser Arg Glu
               180                 185                 190
   cag gtt atc atc aac acc tgg tac ggc ggt gaa atg aag aag ggt atg      624
   Gln Val Ile Ile Asn Thr Trp Tyr Gly Gly Glu Met Lys Lys Gly Met
           195                 200                 205
   ttt tcc atg atg aac tac ttc ctg ccg ctc aag ggg att gct gct atg      672
   Phe Ser Met Met Asn Tyr Phe Leu Pro Leu Lys Gly Ile Ala Ala Met
       210                 215                 220
   cat tgc tcc gct aat acg gac aag cag ggc cag aac acg gct atc ttc      720
   His Cys Ser Ala Asn Thr Asp Lys Gln Gly Gln Asn Thr Ala Ile Phe
   225                 230                 235                 240
   ttc ggc ctc tcc ggc acg ggt aaa acc acc ctg tcc acg gac ccg aaa      768
   Phe Gly Leu Ser Gly Thr Gly Lys Thr Thr Leu Ser Thr Asp Pro Lys
                   245                 250                 255
   cgt ctc ctg att ggt gat gat gaa cac ggc tgg gat gat gaa ggc gta      816
   Arg Leu Leu Ile Gly Asp Asp Glu His Gly Trp Asp Asp Glu Gly Val
               260                 265                 270
   ttc aac ttc gaa ggc ggc tgc tat gct aag gtt atc aac ctc gac atg      864
   Phe Asn Phe Glu Gly Gly Cys Tyr Ala Lys Val Ile Asn Leu Asp Met
           275                 280                 285
   gaa tcc gaa ccg gac atc tat ggc gcc atc aaa cgt aac gct ctg ctc      912
   Glu Ser Glu Pro Asp Ile Tyr Gly Ala Ile Lys Arg Asn Ala Leu Leu
       290                 295                 300
   gaa aac gtt acc ctc gac gac aag ggc aac atc gac ttt gcc gat aag      960
   Glu Asn Val Thr Leu Asp Asp Lys Gly Asn Ile Asp Phe Ala Asp Lys
   305                 310                 315                 320
   acc atc acg gaa aac acc cgt gta tcc tat cct atc gac cac atc aaa     1008
   Thr Ile Thr Glu Asn Thr Arg Val Ser Tyr Pro Ile Asp His Ile Lys
                   325                 330                 335
   ggc acc gtt aag ggc ttt gtt aac gac aag agc gca gct ccg gca gct     1056
   Gly Thr Val Lys Gly Phe Val Asn Asp Lys Ser Ala Ala Pro Ala Ala
               340                 345                 350
   aag agt gtt atc ttc ctg tcc gct gat gct ttc ggc gta ctg ccc ccg     1104
   Lys Ser Val Ile Phe Leu Ser Ala Asp Ala Phe Gly Val Leu Pro Pro
           355                 360                 365
   gtt tcc atc ctg act ccg gaa cag acg aag tat tac ttc ctc tcc ggc     1152
   Val Ser Ile Leu Thr Pro Glu Gln Thr Lys Tyr Tyr Phe Leu Ser Gly
       370                 375                 380
   ttc acg gct aaa ctg gct ggt acg gaa cgc ggc atc acc gaa ccg aca      1200
   Phe Thr Ala Lys Leu Ala Gly Thr Glu Arg Gly Ile Thr Glu Pro Thr
   385                 390                 395                 400
   ccg acc ttc tcc gct tgc ttc ggt cag gca ttc ctc gaa ctg cat ccg      1248
   Pro Thr Phe Ser Ala Cys Phe Gly Gln Ala Phe Leu Glu Leu His Pro
                   405                 410                 415
   acc aag tac gca gaa gaa ctc gtt aag aag atg gag gct aac ggc acg      1296
   Thr Lys Tyr Ala Glu Glu Leu Val Lys Lys Met Glu Ala Asn Gly Thr
               420                 425                 430
   aag gca tac ctc gtg aac acg ggc tgg aat ggt tcc ggc aag cgt atc      1344
   Lys Ala Tyr Leu Val Asn Thr Gly Trp Asn Gly Ser Gly Lys Arg Ile
           435                 440                 445
   tcc atc aaa gat acc cgt ggc atc atc gat gct atc cat agc ggc gct      1392
   Ser Ile Lys Asp Thr Arg Gly Ile Ile Asp Ala Ile His Ser Gly Ala
       450                 455                 460
   atc aaa aaa gct ccg acc aag aag att ccg ttc ttc aac ctc gaa gta      1440
   Ile Lys Lys Ala Pro Thr Lys Lys Ile Pro Phe Phe Asn Leu Glu Val
   465                 470                 475                 480
   ccg acg gaa ctt gag ggc gtt gac acc aac atc ctc gac ccg aag gat      1488
   Pro Thr Glu Leu Glu Gly Val Asp Thr Asn Ile Leu Asp Pro Lys Asp
                   485                 490                 495
   acc tat gct aac ccg gct gat tgg gaa gca aaa gca aaa gac ctc gct      1536
   Thr Tyr Ala Asn Pro Ala Asp Trp Glu Ala Lys Ala Lys Asp Leu Ala
               500                 505                 510
   cag cgc ttc atc aag aac ttc gac aaa tac acg aag aac aat gaa gct      1584
   Gln Arg Phe Ile Lys Asn Phe Asp Lys Tyr Thr Lys Asn Asn Glu Ala
           515                 520                 525
   ggt aag gct ctc gtt gcc gct ggt ccg cag ctc taa                      1620
   Gly Lys Ala Leu Val Ala Ala Gly Pro Gln Leu
       530                 535
<210> 65
   <211> 539
   <212> PRT
   <213> selenomonas ruminantium
<400> 65
   Met Ala Asn Ile Asp Leu Ser Gln Tyr Gly Ile Thr Gly Thr Thr Gly
   1               5                   10                  15
   Ile Leu His Asn Pro Ser Tyr Lys Thr Leu Phe Glu Glu Glu Thr Lys
               20                  25                  30
   Glu Gly Leu Thr Gly Tyr Glu Gln Gly Gln Val Ser Glu Leu Gly Ala
           35                  40                  45
   Val Asn Val Lys Thr Gly Ile Phe Thr Gly Arg Ser Pro Lys Asp Lys
       50                  55                  60
   Phe Ile Val Asp Asp Glu Thr Ser His Asp Thr Val Trp Trp Asp Ser
   65                  70                  75                  80
   Glu Asp Tyr His Asn Asp Asn His Arg Ala Thr Pro Glu Thr Trp Asn
                   85                  90                  95
   Ala Leu Lys Glu Ile Ala Lys Lys Glu Leu Ser Asn Lys Lys Leu Tyr
               100                 105                 110
   Val Val Asp Ala Phe Cys Gly Ala Asn Lys Asp Thr Arg Met Ala Val
           115                 120                 125
   Arg Phe Ile Val Glu Val Ala Trp Gln Ala His Phe Val Thr Asn Met
       130                 135                 140
   Phe Ile Gln Pro Thr Glu Glu Glu Leu Ala Asn Phe Lys Pro Asp Phe
   145                 150                 155                 160
   Val Val Tyr Asn Ala Ser Lys Ala Lys Val Glu Asn Tyr Lys Glu Leu
                   165                 170                 175
   Gly Leu His Ser Glu Thr Ala Val Val Phe Asn Leu Thr Ser Arg Glu
               180                 185                 190
   Gln Val Ile Ile Asn Thr Trp Tyr Gly Gly Glu Met Lys Lys Gly Met
           195                 200                 205
   Phe Ser Met Met Asn Tyr Phe Leu Pro Leu Lys Gly Ile Ala Ala Met
       210                 215                 220
   His Cys Ser Ala Asn Thr Asp Lys Gln Gly Gln Asn Thr Ala Ile Phe
   225                 230                 235                 240
   Phe Gly Leu Ser Gly Thr Gly Lys Thr Thr Leu Ser Thr Asp Pro Lys
                   245                 250                 255
   Arg Leu Leu Ile Gly Asp Asp Glu His Gly Trp Asp Asp Glu Gly Val
               260                 265                 270
   Phe Asn Phe Glu Gly Gly Cys Tyr Ala Lys Val Ile Asn Leu Asp Met
           275                 280                 285
   Glu Ser Glu Pro Asp Ile Tyr Gly Ala Ile Lys Arg Asn Ala Leu Leu
       290                 295                 300
   Glu Asn Val Thr Leu Asp Asp Lys Gly Asn Ile Asp Phe Ala Asp Lys
   305                 310                 315                 320
   Thr Ile Thr Glu Asn Thr Arg Val Ser Tyr Pro Ile Asp His Ile Lys
                   325                 330                 335
   Gly Thr Val Lys Gly Phe Val Asn Asp Lys Ser Ala Ala Pro Ala Ala
               340                 345                 350
   Lys Ser Val Ile Phe Leu Ser Ala Asp Ala Phe Gly Val Leu Pro Pro
           355                 360                 365
   Val Ser Ile Leu Thr Pro Glu Gln Thr Lys Tyr Tyr Phe Leu Ser Gly
       370                 375                 380
   Phe Thr Ala Lys Leu Ala Gly Thr Glu Arg Gly Ile Thr Glu Pro Thr
   385                 390                 395                 400
   Pro Thr Phe Ser Ala Cys Phe Gly Gln Ala Phe Leu Glu Leu His Pro
                   405                 410                 415
   Thr Lys Tyr Ala Glu Glu Leu Val Lys Lys Met Glu Ala Asn Gly Thr
               420                 425                 430
   Lys Ala Tyr Leu Val Asn Thr Gly Trp Asn Gly Ser Gly Lys Arg Ile
           435                 440                 445
   Ser Ile Lys Asp Thr Arg Gly Ile Ile Asp Ala Ile His Ser Gly Ala
       450                 455                 460
   Ile Lys Lys Ala Pro Thr Lys Lys Ile Pro Phe Phe Asn Leu Glu Val
   465                 470                 475                 480
   Pro Thr Glu Leu Glu Gly Val Asp Thr Asn Ile Leu Asp Pro Lys Asp
                   485                 490                 495
   Thr Tyr Ala Asn Pro Ala Asp Trp Glu Ala Lys Ala Lys Asp Leu Ala
               500                 505                 510
   Gln Arg Phe Ile Lys Asn Phe Asp Lys Tyr Thr Lys Asn Asn Glu Ala
           515                 520                 525
   Gly Lys Ala Leu Val Ala Ala Gly Pro Gln Leu
       530                 535
<210> 66
   <211> 37
   <212> DNA
   <213> Artificial sequence
<220>
   <223> primer
<400> 66
   catggcggcc gcttatttga tttcaatttt gtcccac 37
<210> 67
   <211> 51
   <212> DNA
   <213> Artificial sequence
<220>
   <223> primer
<400> 67
   gatcccgatc gaaggaggtt ataaaaaatg gaattgaatt cgtgtaattg c 51